# EUROPEAN PATENT SPECIFICATION

(11) **EP 2 288 722 B1**
(45) Date of publication and mention of the grant of the patent: **05.11.2014**
(21) Application number: 08742682.1
(22) Date of filing: 10.04.2008
(51) Int. Cl.: C12Q 1/68

(54) **PROSTATE CANCER-SPECIFIC ALTERATIONS IN ERG GENE EXPRESSION AND DETECTION AND TREATMENT METHODS BASED ON THOSE ALTERATIONS**
PROSTATAKARZINOM-SPEZIFISCHE ÄNDERUNGEN DER ERG-GENEXPRESSION UND AUF DIESEN ÄNDERUNGEN BERUHENDE NACHWEIS- UND BEHANDLUNGSVERFAHREN
ALTÉRATIONS DANS L'EXPRESSION DU GÈNE ERG SPÉCIFIQUES DU CANCER DE LA PROSTATE ET PROCÉDÉS DE DÉPISTAGE ET DE TRAITEMENT BASÉS SUR CES ALTÉRATIONS

(43) Date of publication of application: 02.03.2011
(73) Proprietor: The Henry M. Jackson Foundation for the Advancement of Military Medicine, Inc., Bethesda, MD 20817 (US)
(72) Inventor: SRIVASTAVA, Shiv, Potomac, MD 20854 (US); DOBI, Albert, Columbia, MD 21046 (US); SREENATH, Taduru, Germantown, MD 20874 (US); PETROVICS, Gyorgy, Bethesda, MD 20817 (US); SUN, Chen, Bethesda, MD 20814 (US)
(74) Representative: Dörries, Hans Ulrich
(86) International application number: PCT/US2008/004580
(87) International publication number: WO 2009/126122

(56) References cited:
- WO-A-02/10449
- WO-A-03/073826
- WO-A-2005/113816
- WO-A-2008/063769
- US-A1- 2005 272 080
- US-A1- 2007 212 702
- US-B2- 7 374 927
- PETROVICS GYORGY ET AL: "Frequent overexpression of ETS-related gene-1 (ERG1) in prostate cancer transcriptome" ONCOGENE, NATURE PUBLISHING GROUP, GB BASINGSTOKE, HANTS, vol. 24, no. 23, 26 May 2005 (2005-05-26), pages 3847-3852, XP002464141 ISSN: 0950-9232 cited in the application
- TOMLINS SCOTT A ET AL: "Recurrent fusion of TMPRSS2 and ETS transcription factor genes in prostate cancer" SCIENCE, AMERICAN ASSOCIATION FOR THE ADVANCEMENT OF SCIENCE, US, WASHINGTON, DC, vol. 310, no. 5748, 28 October 2005 (2005-10-28), pages 644-648, XP002464140 ISSN: 0036-8075
- OWCZAREK C M ET AL: "Detailed mapping of the ERG-ETS2 interval of human chromosome 21 and comparison with the region of conserved synteny on mouse chromosome 16" GENE, ELSEVIER, AMSTERDAM, NL, vol. 324, 7 January 2004 (2004-01-07), pages 65-77, XP004482475 ISSN: 0378-1119 cited in the application
- DATABASE GENESEQ [Online] 28 December 2007 'Viral regulatory miRNA SEQ ID NO 281972.' Retrieved from EBI, accession no. GSN:AJK29653 Database accession no. AJK29653
- DATABASE EMBL [Online] 10 May 2001 'Sequence 1711 from Patent WO0129262.' Retrieved from EBI, accession no. EMBL:AX116588 Database accession no. AX116588
- DATABASE EMBL [Online] 30 January 2004 'Sequence 18427 from Patent WO0210449.' Retrieved from EBI, accession no. EMBL:CQ548792 Database accession no. CQ548792
- 'SEQ ID NO 636076 of US20050272080A1', [Online] 08 December 2005, Retrieved from the Internet: <URL:http://seqdata.uspto.gov/> [retrieved on 2013-01-16]
- DATABASE EMBL [Online] 26 August 2008 'Sequence 636076 from patent US 7374927.' Retrieved from EBI, accession no. EMBL:GC436076 Database accession no. GC436076
- DATABASE EMBL [Online] 09 April 2005 'sma22d07.g1 Clint Pan troglodytes verus STS genomic, sequence tagged site.' Retrieved from EBI, accession no. EM_STS:BV548452 Database accession no. BV548452
- DATABASE EMBL [Online] 29 September 1998 '635H5T7 Human pBeloBAC11 (RSHubert) Homo sapiens STS genomic clone BAC 635H5 T7 promoter, sequence tagged site.' Retrieved from EBI, accession no. EM_STS:G29039 Database accession no. G29039
- SRIVASTAVA S: 'Structure and function of the splice variants of TMPRSS2-ERG, a prevalent genomic alteration in prostate cancer', [Online] September 2012, www.dtic.mil Retrieved from the Internet: <URL:http://www.dtic.mil/get-tr-doc/pdf?AD= ADA566991> [retrieved on 2013-12-16]

## Description

### TECHNICAL FIELD

The invention relates to polynucleotide and polypeptide sequences that are involved in, or associated with, prostate cancer. The invention further relates to therapeutic compositions and to methods of detecting, diagnosing, and treating prostate cancer.

### BACKGROUND

***E**TS* **R**elated **G**ene (*ERG*), a member of the *ETS* transcription family, was initially isolated and described in 1987 (Reddy et al., PROC. NATL. ACAD. SCI. USA 84:6131-35 (1987); Rao et al., SCIENCE 237:635-39 (1987)). Like other members of the *ETS* family, it plays a central role in mediating mitogenic signals transmitted by major cellular pathways, including the MAPK pathway. Proteins in the *ETS* family show a wide variety of expression patterns in human tissues. *ERG* is expressed in endothelial tissues, hematopoietic cells, kidney, and in the urogenital track. (Oikawa et al., GENE 303:11-34 (2003).) Expression of *ERG* has also been detected in endothelial cells (microvessels) of the stroma in a small proportion of prostate cancer. (Gavrilov et al., EuR J CANCER 37: 033-40 (2001).)

The ERG protein participates in the regulation of gene expression by binding both to DNA comprising a 5'- GGA(A/T) - 3' consensus sequence and to the Jun/Fos heterodimer. These interactions occur via the highly conserved *ETS* domain. (Verger et al., J BIOL CHEM 276: 17181-89 (2001).) Splice variants exist, and of the nine that have been reported, *ERG6* and *ERG9* have multiple stop codons that likely render them non-functional. (Owczarek et al., GENE 324: 65-77 (2004).) *ERG7* and *ERG8* can be distinguished from *ERG1-5* by the absence of exon 16. (*Id*.) In addition, the *ERG8* transcript is unique in its inclusion of a 3' sequence following exon 12, a portion of which forms part of the open reading frame. (*Id*.) *ERG8* had been previously described as a 1460 base pair linear mRNA, with the National Center for Biotechnology Information ("NCBI") Accession No. AY204742. (Owczarek *et al.* (2004).)

*ERG,* like other members of the *ETS* family, is a proto-oncogene with transforming activity. (Oikawa et al., GENE 303:11-34 (2003); Hsu et al., J CELL BIOCHEM 91:896-903 (2004); Reddy et al., PROC NATL ACAD SCI USA, 84:6131-35 (1987); Hart et al., ONCOGENE 10:1423-30 (1995); Sementchenko et al., ONCOGENE 17:2883-88 (1998).) Chromosomal translocations involving *ERG* have been linked to Ewing sarcoma, myeloid leukemia, and cervical carcinoma. (Oikawa et al., GENE 303:11-34 (2003).) It has recently been shown that *ERG1* is the most commonly overexpressed proto-oncogene in malignant prostatic tissue. (Petrovics et al., ONCOGENE 24:3847-52 (2005).) Independently, Tomlins et al., SCIENCE 310:644-48 (2005), described novel gene fusions involving ERG and *TMPRSS2,* an androgen-sensitive gene, that may provide at least one possible mechanism for *ERG1* overexpression. At least two additional studies have confirmed *ERG* rearrangements in prostate cancer. (Soller et al., GENES CHROMOSOMES CANCER 45:717-19 (2006); Yoshimoto et al., NEOPLASIA 8:465-69 (2006).)

Although prostate cancer is the most common non-skin cancer in North American men and the third leading cause of cancer mortality (Jemal et al., CANCER J CLIN 56:106-30 (2005)) remarkably little is known about critical events in prostatic carcinogenesis. While recent reports of high frequency genomic rearrangements involving the *ERG* locus and *ERG1* overexpression are intriguing, there remains a need in the art to identify and characterize the gene expression products of the *ERG* locus in prostate cancer. Cancer-derived transcripts, splice variant transcripts, and altered expression ratios between transcripts are highly specific tools that can be used for cancer diagnosis throughout the different stages of cancer development. In addition, targeted inhibition or activation of these products, and/or direct manipulation of cancer-specific promoters, can be used as highly selective therapeutic strategies to target the causative root of cancer. Thus, the identification of molecular alterations specific for prostate cancer would not only permit optimization of diagnosis and prognosis but also would permit establishment of individualized treatments tailored to the molecular profile of the tumor.

In addition, while prostate cancer is increasingly detected early, the prognosis of individual patients remains a challenge. Identification of molecular biomarkers representing functionally relevant pathways that can distinguish between aggressive and indolent forms of prostate cancer at early stages will have tremendous impact in improving prognostic and therapeutic decisions. Other than serum PSA, currently there are no rational (tumor biology based) prognostic or therapeutic molecular biomarkers available in the clinical practice of prostate cancer.

While 80% of prostate cancer patients respond well to surgery, radiation therapy or watchful waiting, about 20% will develop metastasis that is often fatal to patients. Initially, prostate cancer development is driven by the androgen receptor (AR) pathway. (Heinlein et al., ENDOCRINE REV 25:276-308 (2004); Linja et al., J STEROID BIOCHEM MOL BIOL 92: 255-64 (2004); Shaffer et al., LANCET ONCOL 4:407-14 (2003); Chen et al., NAT MED 10: 26-7 (2004).) However, frequent alterations of AR structure and/or function are well recognized during prostate cancer progression especially with metastatic disease. Other genetic pathways that are often altered in these late stage androgen-independent tumors include *p53* mutations, *BCL2* overexpression and mutations or reduced expression of *PTEN.* (Shaffer et al., LANCET ONCOL 4:407-14 (2003).) Importantly, both p53 and PTEN pathways may affect AR functions.

Defects in AR-mediated signaling are increasingly highlighted for potential causal roles in prostate cancer progression. (Heinlein et al., ENDOCRINE REV 25:276-308 (2004); Dehm et al., J CELL BIOCHEM 99: 333-344 (2006).) Prostate cancer associated alterations of AR functions by various mechanisms, including AR mutations, AR gene amplification, altered AR mRNA or AR protein levels, changes in AR interaction with co-activators/co-repressors and ligand independent AR activation by growth factorslcytokines, may all contribute to prostate cancer progression. (Gelmann, J CLIN ONCOL 20:3001-15 (2002); Grossman et al., J NATL CANCER INST 93: 1687-97 (2001).) Due to the lack of precise knowledge of AR dysfunctions in pathologic specimens, it is difficult to identify patients with functional defects of AR.

The choice of therapy for late stage prostate cancer is systemic androgen ablation, which eventually fails in most patients. Therefore, the knowledge of AR pathway dysfunctions that are predictive of androgen ablation therapy failure would significantly impact the patient stratification for new emerging therapeutic strategies.

Unlike in breast cancer where estrogen receptor protein status in primary tumor is effectively used in making therapeutic and prognostic decisions (Yamashita et al., BREAST CANCER 13(1):74-83 (2006); Martinez et al., AM J SURG 191(2):281-3 (2006); Giacinti et al., ONCOLOGIST 11(1):1-8 (2006); Regan et al., BREAST 14(6):582-93(2005); Singh et al., J CELL BIOCHEM 96(3):490-505 (2005)), AR protein expression status does not appear to be useful in prostate cancer, likely because many factors besides AR protein expression level may affect AR activity. Although AR expression can be detected throughout the progression of prostate cancer, it is heterogeneous and changes over time. Several studies have indicated that AR expression is reduced in poorly differentiated areas with a higher Gleason score. (Heinlein et al., ENDOCRINE REV 25:276-308 (2004); Linja et al., J STEROID BIOCHEM MOL BIOL 92: 255-64 (2004); Shaffer et al., LANCET ONCOL 4:407-14 (2003); Chen et al., NAT MED 10: 26-7 (2004); Gelmann, J CLIN ONCOL 20:3001-15 (2002); Grossman et al., J NATL CANCER INST 93:1687-97 (2001); Krishnan et al., CLIN CANCER RES 6:1922-30 (2000).)

In contrast, some recent reports found that higher AR expression is associated with higher clinical stage, higher Gleason score, and with decreased PSA recurrence-free survival. (Linja et al., CANCER RES 61:3550-55 (2001); Sweat et al., J UROL 161:1229-32 (1999); Li et al., Ann J SURG PATHOL 28:928-34 (2004).) Part of the reason for this controversy is the inherent heterogeneity of AR expression in the prostate and the semi-quantitative nature of immunohistochemical evaluations. (Krishnan et al., CLIN CANCER RES 6:1922-30 (2000).) In recent years, our laboratory has established novel insights into the androgen regulated transcriptome and identified AR targets which have promise in defining the role of AR dysfunctions in prostate cancer, as well as in providing novel biology based biomarkers and therapeutic targets during prostate cancer progression. (Xu et al., CANCER RES. 63(15):4299-304 (2003); Segawa et al., ONCOGENE 21(57):8749-58 (2002); Xu et al., INT J CANCER 92(3):322-8 (2001); Xu et al., GENOMICS 66(3): 257-263 (2000); Masuda et al., J MOL BIOL 353(4):763-71 (2005); Richter et al., PROSTATE CANCER PROSTATIC DIS 10(2):114-8 (2007). WO 2005/113816 A2 describes methods and kits for detecting cancer, in particular prostate cancer.

Nevertheless, a need still exists to streamline the functional evaluation of AR defects at early stages of prostate cancer, when the impact of this knowledge on disease management will be more profound. The present application meets this need by providing a read out for the measurement of the expression of carefully selected AR downstream targets. This read out provides information on the *in vivo* functional status of AR in prostate cancer cells, which helps to stratify patients based on AR signal amplitude and can be used to help prognose prostate cancer and provide new ways of managing and treating these patients.

In particular, a need exists to further characterize the ERG8 protooncogene and its role in prostate cancer. *ERG8* provides an untapped source of diagnostic, prognostic, and therapeutic agents applicable to prostate cancer

Citation of references herein shall not be construed as an admission that such references are prior art to the present invention.

### SUMMARY

Transcription of the *ERG* gene is altered in prostate cancer cells compared to benign cells. The present application describes for the first time the complete *ERG*8 nucleotide sequence and also describes the predominant expression of the *ERG*8 isoform in cancerous cells. It also provides the sequence and characterization of two unique, cancer-specific transcripts of the *ERG* locus, *ERG* Prostate Cancer-specific Isoform 1 (*EPC*1) and *EPC*2. The disclosed *ERG* isoforms can be used alone or in combination as biomarkers of prostate cancer, as targets for therapeutic intervention, or to develop therapeutic agents. In addition, the disclosure describes a novel, prostate cancer-specific *ERG* promoter. The *ERG* promoter can be used to selectively target expression of therapeutic proteins, such as cellular toxins, to prostate cancer cells. Polynucleotide transcripts produced from this novel promoter can also be detected as biomarkers for prostate cancer diagnosis, or to aid in prognosis of prostate cancer.

In one aspect, the disclosure provides the nucleic acid sequences and encoded protein sequences for cancer-specific gene transcripts of the *ERG* locus, including *ERG*8, *EPC*1, and *EPC*2. Antibodies to the encoded polypeptides, and to fragments of those polypeptides, are also described. In some embodiments, the antibody binds an epitope of the polypeptide or polypeptide fragment that is linear, whereas in other embodiments the epitope is conformational. In some embodiments, the epitope is contained within, or comprising, the unique carboxy-terminus of the EPC1 or EPC2 polypeptide. Some of the antibodies that bind an epitope in the carboxy terminus of EPC1 or

EPC2 also bind the respective EPC1 or EPC2 polypeptide.

The disclosure further provides kits for detecting prostate cancer. These kits can be used to detect (either qualitatively or quantitatively) nucleic acids or proteins that serve as prostate cancer markers. For example, the expression of prostate cancer-specific isoforms of the *ERG* gene, such as *ERG*8, *EPC*1*, EPC*2, or the transcripts produced by the prostate cancer-specific promoter, when detected in a biological sample from a subject, either alone or in combination with other cancer markers, can be used to indicate the presence of prostate cancer in the subject or a higher predisposition of the subject to develop prostate cancer, or they can be used to predict the severity or stage of prostate cancer, such as whether the cancer is high risk or a moderate risk cancer.

In some embodiments, the kits comprise a nucleic acid probe, such as the probes described elsewhere in the disclosure, that hybridizes under defined conditions to an *ERG* sequence. The nucleic acid probe can hybridize to SEQ ID NO: 1 (*ERG*8), SEQ ID NO: 3 (*EPC*1), SEQ ID NO: 5 (*EPC*2), SEQ ID NO: 30 (*ERG*8), or SEQ ID NO: 46 (*ERG*8) (or sequences complimentary to SEQ ID NO: 1, SEQ ID NO: 3, SEQ ID NO: 5, SEQ ID NO: 30, or SEQ ID NO: 46), or a combination of probes can be used to hybridize to *ERG*8 and *EPC*1, ERG8 and *EPC*2, *EPC*1 and *EPC*2, or even *ERG*8, *EPC*1, and *EPC*2. In other embodiments, the kits comprise first and second oligonucleotide primers that hybridize to non-overlapping sequences in *ERG8* (SEQ ID NOS: 1, 30, and 46), *EPC*1 (SEQ ID NO: 3), or EPC2 (SEQ ID NO: 5). In some embodiments, primer pairs that hybridize to *ERG*8 and *EPC*1; *ERG*8 and *EPC*2; *EPC*1 and *EPC*2; or *ERG8, EPC*1, and *EPC*2, are used in combination. In such cases, one or more of the *ERG*8, *EPC*1, or *EPC*2 primers may be the same.

The disclosure additionally describes diagnostic kits comprising an anti-ERG isoform-specific antibody, for example, an anti-ERG8 antibody, an anti-EPC1 antibody, or anti-EPC2 antibody. In one embodiment, the disclosure provides an anti-EPC1 antibody that binds an epitope comprising acids amino acids 217 to 220 of SEQ ID NO: 4. In another embodiment, the antibody is an anti-EPC2 antibody that binds an epitope within or comprising amino acids 28 to 97 of SEQ ID NO: 6. In each case, the epitope can be a linear epitope or a conformational epitope. In some embodiments, combinations of antibodies can be included in the kit. For example, a kit can comprise anti-ERG8 and anti-EPC1 antibodies, anti-ERG8 and anti-EPC2 antibodies, anti-EPC1 and anti-EPC2 antibodies, or anti-ERGB, anti-EPC1, and anti-EPC2 antibodies. The antibodies can be, optionally, detectably labeled.

*ERG* isoform expression can be used to diagnose or prognose prostate cancer. The disclosure therefore also provides methods for detecting the expression of one or more of *ERG*8, *EPC*1, or *EPC*2 in a biological sample, such as prostate tissue, blood, serum, plasma, urine, saliva, or prostatic fluid. For example, in some embodiments, the methods comprise detecting amplification products of *ERG*8, *EPC*1, or *EPC*2 using hybridization-based techniques. In other embodiments, amplification products are size separated and visualized as part of the detection methods. The methods of diagnosing or prognosing prostate cancer can further comprise measuring the expression level (*e.g*. mRNA or polypeptide) of *ERG*8, *EPC*1, or *EPC*2, and correlating the expression level of the *ERG* isoform with the presence of prostate cancer or a higher predisposition to develop prostate cancer in the subject, or with the severity or stage of prostate cancer, such as high risk or moderate risk prostate cancer.

In some embodiments, the methods comprise detecting the expression of the *ERG*8 isoform. In other embodiments, it is the expression of the *EPC*1 isoform that is detected. In yet other embodiments, the *EPC*2 isoform is detected. In still other embodiments, the methods comprise detecting the *ERG*8 and *EPC*1 isoforms in combination, the *ERG*8 and *EPC*2 isoforms in combination, the *EPC*1 and *EPC*2 isoforms in combination, or the combination of the *ERG*8, *EPC*1, and *EPC*2 isoforms. In each case, each *ERG* isoform can be detected and/or measured by detecting and/or measuring the transcript, or by detecting and/or measuring the corresponding polypeptide.

Therapeutic uses of treating prostate cancer and treating disorders of prostate hyperproliferation are also disclosed. For example, the disclosure provides uses of treating prostate cancer comprising destabilizing a prostate cancer-specific *ERG* gene transcript in prostate cancer cells. In some embodiments, the uses comprise destabilizing one, all, or any combination of *ERG*8, *EPC*1, *EPC*2, *ERG*1, *ERG*2, and/or *ERG*3 transcripts, resulting in degradation of those transcripts and inhibition of expression of the encoded polypeptide(s). In one embodiment, the destabilization employs siRNA. In another embodiment, the uses employ small hairpin RNAs (shRNA). In yet another embodiment, an antisense molecule is used to destabilize the transcript(s). In still another embodiment, a ribozyme is used to cause destabilization. Small molecule inhibitors can also be used to inhibit expression of one or more ERG isoforms. The disclosure also provides methods of using an antibody to one or more ERG isoforms to treat prostate cancer or disorders of prostate hyperproliferation. Thus, in varying embodiments the disclosure provides methods of treating prostate cancer or disorders of prostate hyperproliferation comprising administering an anti-ERG8, an anti-EPC1, an anti-EPC2, an anti-ERG1, and anti-ERG2, an anti-ERG3 antibody, or a combination of those antibodies. In some embodiments, a single antibody may be specific for one or more proteins encoded by the disclosed ERG isoforms.

In another embodiment, the present application provides a panel of biomarkers for prostate cancer, methods and systems for using those biomarkers to diagnose and prognose prostate cancer, and diagnostic and prognostic kits comprising reagents used to detect the biomarkers. In one embodiment the panel comprises a combination of two or more of a set of six androgen inducible/co-regulated genes (*PSA*/*KLK*3, *PMEPA*1, *NKX*3.1, *ODC*1, *AMD*1, and *ERG*). In some embodiments, the ERG gene is *EPC*1, *EPC*2, *ERG*1, *ERG*2, *ERG*3, *ERG*8, or combinations thereof. \

The present application also provides prognostic kits that detect or measure the levels of two or more androgen inducible/co-regulated genes. The prognostic kits are used in methods of predicting the functional status of *in vivo* androgen receptor signaling or in methods of predicting prostate cancer progression or severity, such as predicting whether the prostate cancer is a moderate risk prostate cancer or a high risk prostate cancer, predicting the prostate cancer stage (*e.g*., using the T staging system (pTX, pT0, PT1, pT2, pT3, pT4) or the Whitmore-Jewett system (A, B, C, D)), or predicting whether the prostate cancer is progressing, regressing, or in remission. The prognostic kits can also be used to predict disease-free survival following prostatectomy, which can be defined, for example, by serum PSA level equal or higher than 0.2 ng/ml after prostatectomy. In some embodiments, the prognostic panel comprises two or more of the following genes: *PSA*/*KLK*3, *PMEPA*1, *NKX*3.1, *ODC*1, *AMD*1, and *ERG.* In certain embodiments, the ERG gene is *EPC*1, *EPC*2, *ERG*1, *ERG*2, *ERG*3, *ERG*8, or combinations thereof. Accordingly, assays using the prognostic kits can detect or measure the levels of two or more of these genes. For example, a prognostic kit can be used to measure the levels of two, three, four, five, six, or even more androgen induciblelco-regulated genes.

In certain embodiments, the prognostic assay further comprises detecting or measuring PSA, % PSA, PSA doubling time, PSA velocity, prostate volume or a combination of these indicators.

In prognostic embodiments, the method of prognosing prostate cancer can comprise detecting or measuring in a biological sample from an individual the expression of two or more of genes chosen from *PSA*/*KLK3, PMEPA1, NKX3.1, ODC1, AMD1,* and *ERG*; and comparing, for the expression of each gene detected or measured, the results obtained in (a) with the expression of the same gene in a control sample.

In a prognostic method, the altered expression of the two or more genes in the patient sample relative to the control sample is predictive of disease severity, for example a moderate risk prostate cancer or a high risk prostate cancer. The altered expression may also be predictive of whether the prostate cancer is progressing, regressing, or in remission. Alternatively, a threshold value of gene expression can be selected and used as the control sample. In this case, if the gene expression level is less than the threshold value, it is considered reduced. The threshold value can be determined using known techniques. For example, the value can be determined from the mRNA copy number or the cycle threshold value.

Although increases and decreases of at least 10% relative to a control or threshold value can be used in the prognostic methods, other values may also be used. For example, the increase or decrease may be at least 20, 30, 40, 50, 60, 70, 80, 90, 100, 200, 300, 400, or even 500%. The increase or decrease may also be expressed in terms of statistical significance, where a statistically significant increase or decrease in expression, such as p< 0.05, p< 0.01, p< 0.005, or p< 0.001, indicates the presence of prostate cancer or a higher predisposition to develop prostate cancer, prostate cancer progression, or disease severity.

In some prognostic embodiments, a decrease in expression levels of the androgen inducible/co-regulated gene(s) is used to predict compromised androgen receptor signaling, which in turn is predictive of the presence or predisposition to develop high risk or advanced stage prostate cancer or a reduced disease-free survival time following prostatectomy.

The disclosure also provides methods of detecting the expression of two or more of *PSA*/*KLK*3, *PMEPA*1, *NKX*3.1, *ODC*1*, AMD*1, and *ERG* (including *EPC*1, *EPC*2, *ERG*1, *ERG*2, *ERG*3 or *ERG*8) in a biological sample, such as prostate tissue or a biofluid, such as, blood, serum, plasma, urine, saliva, or prostatic fluid. For example, in some embodiments, the methods comprise detecting amplification products of *PSA*/*KLK*3, *PMEPA*1, *NKX*3.1, *ODC*1, *AMD*1, and *ERG* using hybridization-based techniques. In other embodiments, amplification products are size separated and visualized as part of the detection methods. The methods of prognosing prostate cancer can also comprise measuring the expression level of the proteins encoded by *PSA*/*KLK*3, *PMEPA*1, *NKX*3.1, *ODC*1, *AMD*1, and *ERG,* for example by using an antibody.

Additional objects will be set forth in part in the description that follows, and in part will be understood from the description, or may be learned by practice of the invention. It is to be understood that both the foregoing general description and the following detailed description are exemplary and explanatory only and are not restrictive of the invention as claimed.

### BRIEF DESCRIPTION OF THE DRAWINGS

Figure 1 shows the nucleotide sequence of the complete *ERG*8 gene (SEQ ID NO: 30), aligned with the partial gene sequence of NCBI AY204742 (SEQ ID NO: 48).
Figure 2 presents PCR amplification gels of *ERG*1, *ERG*2, *ERG*3, and *ERG*8 transcripts in normal prostate tissue (NP) and in the prostate cancer cell line VCaP.
Figure 3 shows the PCR amplification results for *ERG*8 transcript expression in tumor cells (T) and benign epithelial cells (N) from eight patients.
Figure 4 shows the PCR amplification results for *EPC*1 transcript expression in tumor cells (T) and benign epithelial cells (N) from five patients.
Figure 5 shows the number of copies of the ERG isoforms. Figure 5A presents a schematic diagram of the primer positions for *EPC*1, *ERG*8, and *ERG*12 specific primers. Figure 5B presents the copy numbers of *ERG*1&2*, ERG*8, and *EPC*1 in VCaP cells. Figure 5C presents the copy numbers of *ERG*8 and *ERG*1&2 using microdissected tumor cells from ten prostate cancer patients.
Figure 6 shows a map of alternative transcription start sites in *ERG* exon 9 (nucleotides 486 to 532 of SEQ ID NO: 7).
Figure 7 plots the ability of three segments of the prostate cancer-specific *ERG* promoter to support expression of a luciferase report construct in the VCaP cell line in comparison to the LNCaP cell line.
Figure 8 provides the results of a Pearson correlation analysis of *TMPRSS2-ERG* fusion A transcript expression with *ERG*1, *AR, PSA, PMEPA*1 *and LTF* expression in tumor tissue.
Figure 9A shows that downregulating *ERG* increases the expression of androgen receptor responsive genes. The top panel shows a gel demonstrating that inhibition of ERG with two different siRNAs results in increased expression of androgen-inducible *PSA* and *NKX*3.1 transcripts. The bottom panel shows that PSA levels also increase in the culture supernatant of VCaP cells when ERG is inhibited with siRNAs.

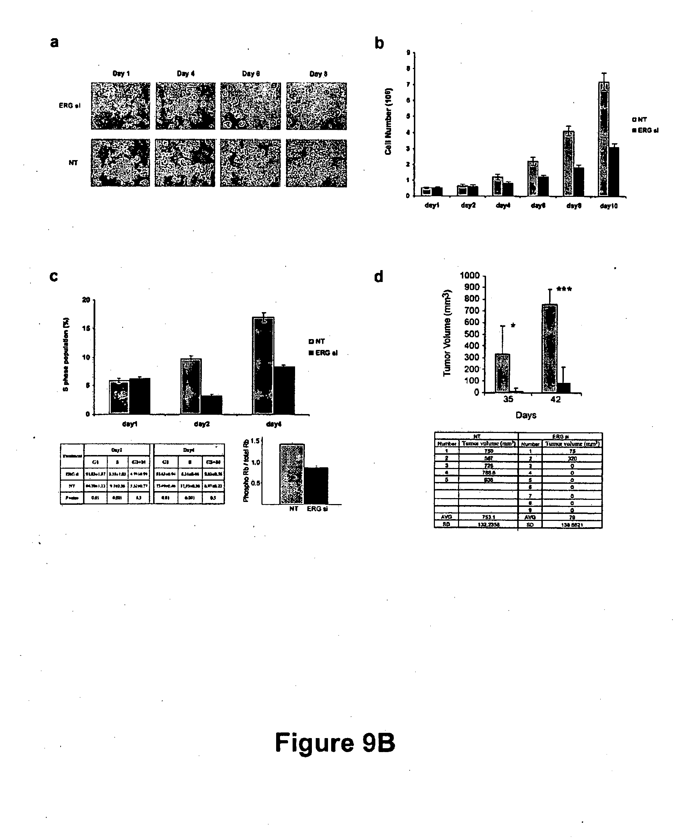

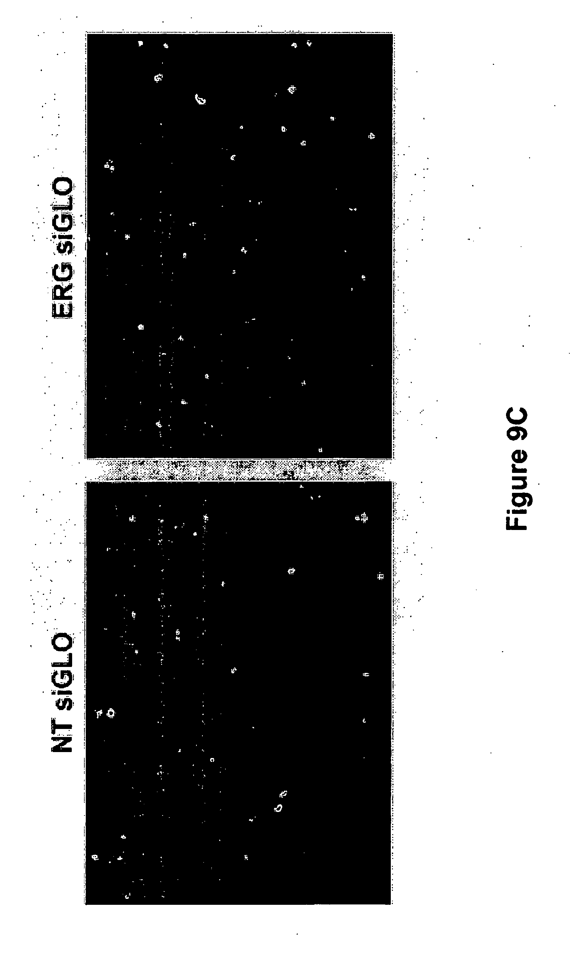

Figure 9B shows that *ERG* knockdown inhibits prostate tumor cell growth both *in vitro* and in an *in vivo* SCID mouse tumorigenicity assay. The top left panel shows the morphology of VCaP cells transfected with 50 nM *ERG* siRNA or control ("NT") RNA. The top right panel shows the inhibitory effect of *ERG* siRNA (dark grey bars) on VCaP cell proliferation, compared to control RNA (light grey bars). The bottom left panel shows a cell cycle analysis demonstrating the inhibitory effect of *ERG* siRNA (dark grey bars) on the number of cells in S phase, compared to control RNA (light grey bars). The table shows a redistribution of the number of cells in G1, S and G2+M phases as a result of *ERG* siRNA treatment, measured by FACS analysis. The bottom right panel shows the inhibitory effect of *ERG* siRNA (dark grey bars) on *in vivo* tumor volume, compared to control RNA (light grey bars).
Figure 9C demonstrates that the transfection efficiency of VCaP cells transfected with 50 nM of *ERG* siGLO or NT siGLO (both from Dharmacon Research, Lafayette, CO) and cultured for two days was nearly 100%.
Figure 10 is a diagram showing that ERG expression can result in inhibition of the androgen receptor responsive genes *PSA* and *NKX*3.1, thereby inhibiting cellular differentiation.
Figure 11 shows the results of siRNA inhibition of *ERG* expression in VCaP prostate cancer cells. Figure 11A shows a microscope field of control VCaP cells and Figure 11B shows a microscope field of cells treated with siRNA-1 (SEQ ID NO: 28).
Figure 12 compares the intensity of gene expression of the androgen regulated genes *PSA*/*KLK*3*, NKX*3.1, *PMEPA*1, *ODC*1, *AMD*1, and *ERG* in tumor and matched benign cells from 40 prostate cancer patients. Z-score normalized GeneChip derived expression intensities are depicted by heat maps on a high-to-low scale after hierarchical clustering. Patient numbers (N=40) are listed above the heat map. Matched tumor and benign specimens are listed in the same order.
Figure 13 shows a heat map display comparing the intensity of gene expression of the prostate cancer related genes *ERG, AMACR, DD*3, *PSGR,* and *PCGEM1* in cells microdissected from prostate tissue sections.
Figure 14 shows the correlation of androgen regulated *PSA*/*KLK3* and *PMEPA*1 genes with *ERG* expression in tumor cells of prostate cancer patients harboring *TMPRSS2-ERG* fusion using QRT-PCR.
Figure 15 demonstrates that *ERG* expression mirrors androgen signaling in prostate cancer tissue. *TMPRSS2-ERG* fusion (left panel) and *PSA*/*KLK*3 (right panel) transcript levels were compared in prostate cancer cells of pT3 and pT2 stage tumors by quantitative PCR. Y-axis scales represent fold changes of tissue expression levels relative to the expression of the *GAPDH* housekeeping gene.
Figure 16 shows the distribution of biochemical recurrence and tissue *PSA*/*KLK*3 mRNA expression in tumor cells of prostate cancer (CaP) patients. Relative expression of *PSA*/*KLK*3 mRNA in tumor cells, represented by vertical bars, is shown on a log2 scale. Darkened bars indicate patients with biochemical recurrence.
Figure 17 shows a Kaplan-Meier survival estimation curve for time to PSA recurrence-free survival by tumor tissue *PSA*/*KLK*3 mRNA quintiles among patients with serum PSA 2-10 ng/ml. Quintiles are presented in decreasing order with quintile 1 referring to the highest and quintile 5 to the lowest *PSA*/*KLK*3 expression (N=79). Lower tissue *PSA*/*KLK3* mRNA expression in prostate tumor cells correlates with an increased risk of biochemical recurrence.
Figure 18 shows the activation of the oncogene *C-MYC* by *ERG.* The left panel shows the result of RT-PCR analysis of VCaP cells treated with *ERG* siRNA. The top right panel shows the morphology of the VCaP cells after eight days of treatment with control ("NT"), *ERG* siRNA, *MYC* siRNA, and both *ERG* siRNA and *MYC* siRNA. The bottom right panel shows a Western blot analysis of the effect of *ERG* siRNA on *C-MYC* expression in VCaP cells. It also shows the correlation between *ERG* expression and *MYC* expression in microdissected human prostate tumors.
Figure 19 shows the effect of *ERG* siRNA on the density and morphology of VCaP cells, compared to NT control cells.
Figure 20 shows a gene network in *ERG*-expressing human prostate tumors. Seven well-differentiated prostate tumors overexpressing *ERG* were analyzed with Bibliosphere software. Red (medium grey) and yellow (light grey) boxes indicate upregulation, shades of blue (dark grey) indicate downregulation.
Figure 21 shows a gene network affected in response to *ERG* knockdown in VCaP cells.
Figure 22 shows Western blots demonstrating diminishing PSA protein levels and decreased recruitment of AR to the PSA AREIII enhancer in response to transient ERG expression.
Figures 22A and 22B show VCaP and LNCaP cells, respectively, infected with adenoviral ERG ("Ad-ERG") or adenoviral control ("Control") vectors. Cell lysates prepared at 24, 48, and 72 hours post-infection were analyzed by immunoblot using anti-ERG, anti-PSA, and anti-tubulin antibodies.
Figures 22C and 22D show ChIP assessment of AR recruitment to the *KLK3*/PSA gene AREIII enhancer in VCaP and LNCaP cells in response to the transient expression of ERG by adenoviral Ad-ERG or Control vectors. "Input" indicates control genomic DNA amplicons.

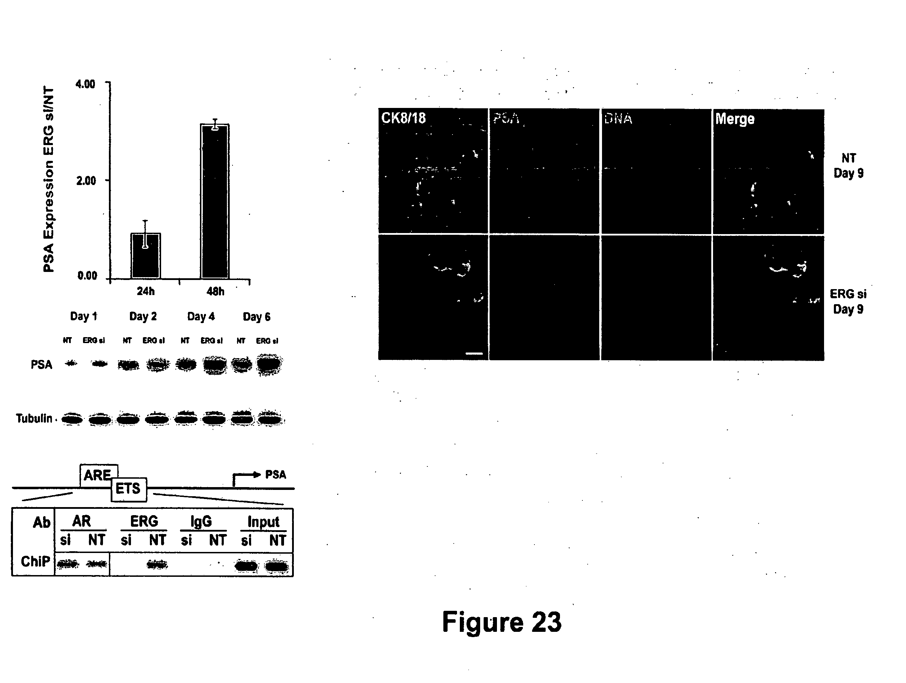

Figure 23 shows the repression of prostate differentiation genes by *ERG.* The top left panel shows the increase in PSA mRNA expression resulting from *ERG* siRNA transfection of VCaP cells, measured by QRT-PCR. The top middle panel shows a corresponding increase in PSA protein expression, measured by Western blot. The bottom left panel shows increased AR binding to the PSA enhancer ("ARE") and decreased *ERG* recruitment to the overlapping *ETS* cognate element, measured by ChIP assay. The right panel shows an immunofluorescence micrograph of VCaP cells nine days after treatment with *ERG* siRNA. The cells were stained with antibodies to cytokeratin ("CK8/18") or PSA, or stained for DNA; the right panel compares merged images. The scale bar represents 25 microns.

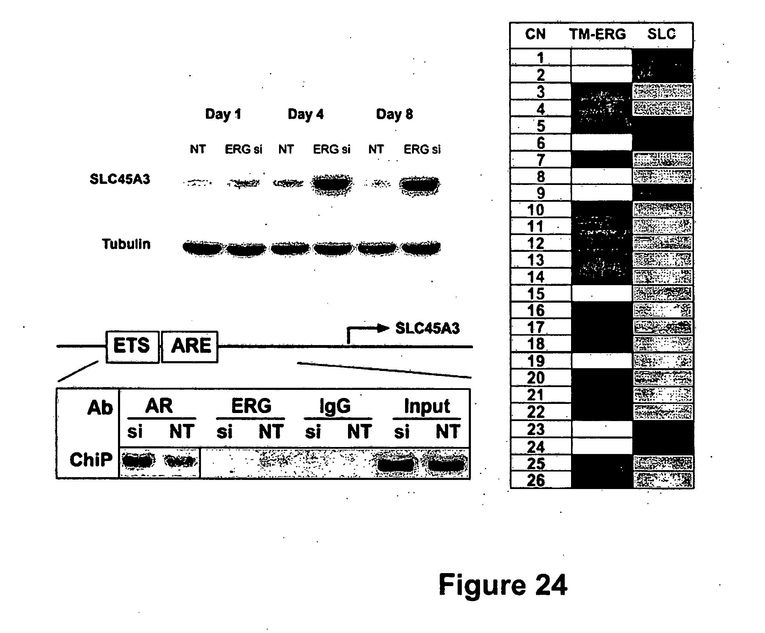

Figure 24 shows the increase in prostein ("SLC45A3") expression in VCaP cells resulting from transfection with *ERG* siRNA. The top left panel shows a Western blot with antibodies to SLC45A3 and tubulin (control). The bottom left panel assesses recruitment of AR and *ERG* to the SLC45A3 promoter upstream ARE and ETS elements by ChIP assay. The right panel shows a matrix of immunostaining results from 26 prostate tumors examined with an antibody to SLC45A3. "CN" indicates the case number of the tumor tissue. "TM-ERG" indicates the presence (black bar) or absence (white bar) of the *TMPRSS2-ERG* gene fusion in the tumor. "SLC" indicates strong (dark grey bars) or weak (light grey bars) immunohistochemical staining with an antibody to SLC45A3.

### DETAILED DESCRIPTION

### Definitions

The term "ERG" refers to the *ERG* gene, as well as to the various *ERG* cDNAs and mRNAs described in the disclosure. Unless a specific isoform or subset of isoforms is indicated, the term *ERG* includes *ERG1, ERG2, ERG3, ERG4, ERG5, ERG6, ERG7, ERG8, ERG9, EPC1, EPC2,* and the truncated *ERG* transcripts that result from activation of the prostate cancer-specific promoter described herein. The phrasing "*ERG*, but not" one or more specifically mentioned *ERG* isoforms may be used in embodiments in which several different, but not all, of the ERG isoforms are contemplated. The cDNA sequence of the *ERG1* gene is published in GenBank under the accession number M21535. The cDNA sequence of the *ERG2* gene is published in GenBank under the accession number M17254. The term "ERG8" refers to the isoform described, *e.g.*, by SEQ ID NO: 1, SEQ ID NO: 30, and SEQ ID NO: 46. The exon usage of *ERG* isoforms 1-9 is presented in Owczarek et al., GENE 324:65-77 (2004). When the context does not clearly exclude it, *ERG* also refers to the various ERG polypeptides encoded by the different isoforms. Further, although italics are generally used to refer to nucleic acids, the use of italics is not to be construed as excluding the encoded polypeptide.

To "destabilize" one or more transcripts means to cause degradation of that/those transcript(s) such that expression of the encoded polypeptide(s) is inhibited or knocked-down. Silent interfering RNA (siRNA), small hairpin RNA (shRNA) (for example, as described by Paddison et al., GENES DEV 16(8):948-58 (2002), antisense molecules, ribozymes, and combinations of these approaches can be used for destabilizing a transcript(s).

A "moderate risk" prostate cancer is cancer in which the patient has, for example, no PSA recurrence, a Gleason score of 6-7, T2a-T3b stage, no seminal vesicle invasion, and well- or moderately-differentiated tumor.

A "high risk" prostate cancer is cancer in which the patient has, for example, PSA recurrence, a Gleason score of 8-9, T3c stage, seminal vesicle invasion, and poor tumor differentiation.

The term "altered expression" refers both to qualitative differences (*i.e.,* that gene or protein expression is detectable versus undetectable) and to quantitative differences (*i.e*., differences in measured levels of gene or protein expression).

The term "isolated" refers to a molecule that is substantially free of its natural environment. Any amount of that molecule elevated over the naturally occurring levels due to any manipulation, *e.g*., over expression, partial purification, etc., is encompassed with the definition. With regard to partially purified compositions only, the term refers to an isolated compound that is at least 50-70%, 70-90%, 90-95% (w/w), or more pure.

The phrase "substantially identical," or "substantially as set out," means that a relevant sequence is at least 70%, 75%, 80%, 85%, 90%, 95%, 97, 98, or 99% identical to a given sequence. By way of example, such sequences may be allelic variants, sequences derived from various species, or they may be derived from the given sequence by truncation, deletion, amino acid substitution, or addition. For polypeptides, the length of comparison sequences will generally be at least 20, 30, 50, 100 or more amino acids. For nucleic acids, the length of comparison sequences will generally be at least 50, 100, 150, 300, or more nucleotides. Percent identity between two sequences is determined by standard alignment algorithms such as, for example, Basic Local Alignment Tool (BLAST) described in Altschul et al., J MOL BIOL 215:403-410 (1990), the algorithm of Needleman et al., J MOL BIOL 48:444-453 (1970), or the algorithm of Meyers et al., COMPUTAPPL BIOSCI 4:11-17 (1988).

"Protein" is used interchangeably with the terms "peptide" and "polypeptide" and refers to any chain of amino acids, regardless of length or posttranslational modification (*e.g*., glycosylation or phosphorylation), or source (*e.g.,* species).

The terms "polynucleotide," "oligonucleotide," "nucleic acid," and "DNA" are used interchangeably herein and refer to deoxyribonucleic acid (DNA), and, where appropriate, ribonucleic acid (RNA). The term should also be understood to include nucleotide analogs, and single or double stranded polynucleotides. Examples of polynucleotides include, but are not limited to, plasmid DNA or fragments thereof, viral DNA or RNA, anti-sense RNA, etc. The term "plasmid DNA" refers to double stranded DNA that is circular.

As used herein the term "hybridization under defined conditions," or "hybridizing under defined conditions," is intended to describe conditions for hybridization and washes under which nucleotide sequences that are significantly identical or homologous to each other remain bound to each other. The conditions are such that sequences, which are at least about six and more preferably at least about 20, 30, 40, 50, 100, 150, 300, or more nucleotides long and at least about 70%, more preferably at least about 80%, even more preferably at least about 85-90% identical, remain bound to each other. The percent identity can be determined as described in Altschul et al., NUCLEIC ACIDS RES 25:3389-3402 (1997). Appropriate hybridization conditions can be selected by those skilled in the art with minimal experimentation as exemplified in Ausubel et al., CURRENT PROTOCOLS IN MOLEC BIOL, John Wiley & Sons (2004). Additionally, stringent conditions are described in Sambrook et al. MOLEC CLONING: A LABORATORY MANUAL, 3rd ed., Cold Spring Harbor Laboratory Press (2001).

A nonlimiting example of defined conditions of low stringency is as follows: Filters containing DNA are pretreated for six hours at 40°C in a solution containing 35% formamide, 5x SSC, 50 mM Tris-HCl (pH 7.5), 5 mM EDTA, 0.1% PVP, 0.1% Ficoll, 1% BSA, and 500 µg/ml denatured salmon sperm DNA. Hybridizations are carried out in the same solution with the following modifications: 0.02% PVP, 0.02% Ficoll, 0.2% BSA, 100 µg/ml salmon sperm DNA, 10% (wt/vol) dextran sulfate, and 5-20 x 10⁶ cpm ³²P-labeled probe is used. Filters are incubated in the hybridization mixture for 18-20 hours at 40°C, and then washed for 1.5 hours at 55°C in a solution containing 2x SSC, 25 mM Tris-HCl (pH 7.4), 5 mM EDTA, and 0.1 % SDS. The wash solution is replaced with fresh solution and incubated an additional 1.5 hours at 60°C. Filters are blotted dry and exposed for autoradiography. Other conditions of low stringency well known in the art may be used (*e.g.,* as employed for cross-species hybridizations).

A non-limiting example of defined conditions of high stringency is as follows: Prehybridization of filters containing DNA is carried out for 8 hours to overnight at 65°C in buffer composed of 6x SSC, 50 mM Tris-HCl (pH 7.5), 1 mM EDTA, 0.02% PVP, 0.02% Ficoll, 0.02% BSA, and 500 µg/ml denatured salmon sperm DNA. Filters are hybridized for 48 hours at 65°C in the prehybridization mixture containing 100 µg /ml denatured salmon sperm DNA and 5-20 x 10⁶ cpm of ³²P-labeled probe. Filters are washed for 1 hour at 37°C in a solution containing 2x SSC, 0.01% PVP, 0.01% Ficoll, and 0.01% BSA. This is followed by a wash in 0.1x SSC at 50°C for 45 minutes. Another non-limiting example of defined conditions of high stringency is as follows: Prehybridization of filters containing DNA is carried out for eight hours to overnight at 65°C in buffer composed of 6x SSC, 50 mM Tris-HCl (pH 7.5), 1 mM EDTA, 0.02% PVP, 0.02% Ficoll, 0.02% BSA, and 500 µg/ml denatured salmon sperm DNA. Filters are hybridized for 12 hours at 65°C in the prehybridization mixture containing 100 µg /ml denatured salmon sperm DNA and 5-20 x 10⁶ cpm of ³²P-labeled probe. Filters are washed for 1 hour at 37°C in a solution containing 2x SSC, 0.01% PVP, 0.01% Ficoll, and 0.01% BSA. This is followed by a wash in 0.1x SSC at 50°C for 45 minutes. Other conditions of high stringency well known in the art may be used. An oligonucleotide hybridizes specifically to a target sequence under high stringency conditions.

The term "primer" or "oligonucleotide primer" means an oligonucleotide capable of binding to a region of a target nucleic acid, or its complement, and promoting nucleic acid amplification of the target nucleic acid. Generally, a primer will have a free 3' end that can be extended by a nucleic acid polymerase. Primers also generally include a base sequence capable of hybridizing via complementary base interactions either directly with at least one strand of the target nucleic acid or with a strand that is complementary to the target sequence. A primer may comprise target-specific sequences and optionally other sequences that are non-complementary to the target sequence. These non-complementary sequences may comprise, for example, a promoter sequence or a restriction endonuclease recognition site.

The term "solid support" means a material that is essentially insoluble under the solvent and temperature conditions of the assay method, comprising free chemical groups available for joining an oligonucleotide or nucleic acid. Preferably, the solid support is covalently coupled to an oligonucleotide designed to directly or indirectly bind a target nucleic acid. When the target nucleic acid is an mRNA, the oligonucleotide attached to the solid support is preferably a poly-T sequence. A preferred solid support is a particle, such as a micron- or submicron-sized bead or sphere. A variety of solid support materials are contemplated, such as, for example, silica, polyacrylate, polyacrylamide, a metal, polystyrene, latex, nitrocellulose, polypropylene, nylon or combinations thereof. In some embodiments, the solid support is capable of being attracted to a location by means of a magnetic field, such as a solid support having a magnetite core.

The term "detecting" or "detection" means any of a variety of methods known in the art for determining the presence of a nucleic acid or a protein. For example, hybridizing a labeled probe to a portion of a nucleic acid is one way to detect that nucleic acid. Binding an antibody that is either directly or indirectly labeled to a protein of interest is an example of a method of detecting that protein. Methods for labeling nucleic acids and antibodies (as well as other proteins) are well known in the art. Labels can be either detectable or functional labels, and include radiolabels (*e.g*., ¹³¹I, ¹²⁵I, ³⁵S, and ⁹⁹Tc), enzymatic labels (*e.g*., horseradish peroxidase or alkaline phosphatase), chemiluminescent labels, and other chemical moieties (*e.g.,* biotin). A labeled probe is an oligonucleotide that specifically binds to another sequence and contains a detectable group which may be, for example, a fluorescent moiety, a chemiluminescent moiety (such as an acridinium ester (AE) moiety that can be detected chemiluminescently under appropriate conditions (as described in U.S. Pat. No. 5,283,174)), a radioisotope, biotin, avidin, enzyme, enzyme substrate, or other reactive group. Other well known detection techniques include, for example, gel filtration, gel electrophoresis and visualization of the amplicons by, for example, staining with ethidium bromide, and High Performance Liquid Chromatography (HPLC). Antibody-based detection methods include ELISA, western blotting, radioimmunoassay (RIA), immunohistochemistry, and other techniques that are well known in the art. As used throughout the specification, the term "detecting" or "detection" includes either qualitative or quantitative detection.

The term "treatment" is used interchangeably herein with the term "therapeutic use" and refers to both therapeutic treatment and prophylactic/preventative measures. Those in need of treatment may include individuals already having a particular medical disorder as well as those who may ultimately acquire the disorder.

The term "effective dose," or "effective amount," refers to that amount of the compound that results in amelioration of symptoms in a patient or a desired biological outcome, *e.g.*, inhibition of cell proliferation. The effective amount can be determined as described in the subsequent sections.

The term "modulatory compound" is used interchangeably with the term "therapeutic" and as used herein means any compound capable of "modulating" either prostate cancer-specific gene expression at the transcriptional, translational, or post-translational levels or modulating the biological activity of a prostate cancer-specific polypeptide. The term "modulate" and its cognates refer to the capability of a compound acting as either an agonist or an antagonist of a certain reaction or activity. The term modulate, therefore, encompasses the terms "activate" and "inhibit." The term "activate," for example, refers to an increase in the expression of the prostate cancer-specific gene or activity of a prostate cancer-specific polypeptide in the presence of a modulatory compound, relative to the activity of the gene or the polypeptide in the absence of the same compound. The increase in the expression level or the activity is preferably at least about 10%, 20%, 30%, 40%, 50%, 60%, 70%, 80%, 90%, or higher. Analogously, the term "inhibit" refers to a decrease in the expression of the prostate cancer-specific gene or activity of a prostate cancer-specific polypeptide in the presence of a modulatory compound, relative to the activity of the gene or the polypeptide in the absence of the same compound. The decrease in the expression level or the activity is preferably at least about 10%, 20%, 30%, 40%, 50%, 60%. 70%, 80%, 90%, or higher. The expression level of the prostate cancer-specific gene or activity of a prostate cancer-specific polypeptide can be measured as described herein or by techniques generally known in the art.

"Antibody" refers to an immunoglobulin or fragment thereof, and encompasses any polypeptide comprising an antigen-binding fragment or an antigen-binding domain. The term includes but is not limited to polyclonal, monoclonal, monospecific, polyspecific, humanized, human, single-chain, chimeric, synthetic, recombinant, hybrid, mutated, grafted, and *in vitro* generated antibodies. Unless preceded by the word "intact", the term "antibody" includes antibody fragments such as Fab, F(ab')₂, Fv, scFv, Fd, dAb, and other antibody fragments that retain antigen-binding function. Unless otherwise specified, an antibody is not necessarily from any particular source, nor is it produced by any particular method.

The terms "specific interaction," "specific binding," or the like, mean that two molecules form a complex that is relatively stable under physiologic conditions. The term is also applicable where, *e.g.,* an antigen-binding domain is specific for a particular epitope, which is carried by a number of antigens, in which case the specific binding member carrying the antigen-binding domain will be able to bind to the various antigens carrying the epitope. Specific binding is characterized by a high affinity and a low to moderate capacity. Nonspecific binding usually has a low affinity with a moderate to high capacity. Typically, the binding is considered specific when the affinity constant Kₐ is higher than 10⁶ M⁻¹, more preferably higher than 10⁷ M⁻¹, and most preferably 10⁸ M⁻¹. If necessary, non-specific binding can be reduced without substantially affecting specific binding by varying the binding conditions. Such conditions are known in the art, and a skilled artisan using routine techniques can select appropriate conditions. The conditions are usually defined in terms of concentration of antibodies, ionic strength of the solution, temperature, time allowed for binding, concentration of non-related molecules (*e.g.,* serum albumin, milk casein), etc.

### Prostate Cancer-Specific ERG Nucleic Acids

The disclosure describes prostate cancer-specific *ERG* isoform nucleic acids, in particular, *ERG8, EPC1, EPC2,* and a prostate cancer-specific promoter located within exon 9 of the *ERG* gene. In the case of *ERG8,* a partial-length splice variant of the *ERG* gene has been described (Owczarek *et al.* (2004)), but the complete *ERG*8 nucleotide sequence and *ERG*8 overexpression in the context of prostate cancer was not previously known. *ERG*8 is herein reported to comprise 2441 nucleotides. It is overexpressed in 60-75% of prostate tumors, thus provides a target for the detection, prognosis, and treatment of prostate cancer.

The protein encoded by *ERG8* lacks the DNA binding domain found in ERG1 and ERG2 but retains the entire protein-protein interaction domain. The expression of ERG8, therefore, likely results in the functional nullification of protein interaction partners of ERG1 and ERG2, resulting in a dominant negative effect.

The disclosure also shows that fusions occur between *ERG8* and *TMPRSS2.* An example of a TMPRSS2-ERG8 fusion transcript is: 
TAGGCGCGAG CTAAGCAGGA GGCGGAGGCG GAGGCGGAGG GCGAGGGGCG 50
GGGAGCGCCG CCTGGAGCGC GGCAGGAAGC CTTATCAGTT GTGAGTGAGG 100
ACCAGTCGTT GTTTGAGTGT GCCTACGGAA CGCCACACCT GGCTAAGACA 150
GAGATGACCG CGTCCTCCTC CAGCGACTAT GGACAGACTT CCAAGATGAG 200
CCCACGCGTC CCTCAGCAGG ATTGGCTGTC TCAACCCCCA GCCAGGGTCA 250
CCATCAAAAT GGAATGTAAC CCTAGCCAGG TGAATGGCTC AAGGAACTCT 300
CCTGATGAAT GCAGTGTGGC CAAAGGCGGG AAGATGGTGG GCAGCCCAGA 350
CACCGTTGGG ATGAACTACG GCAGCTACAT GGAGGAGAAG CACATGCCAC 400
CCCCAAACAT GACCACGAAC GAGCGCAGAG TTATCGTGCC AGCAGATCCT 450
ACGCTATGGA GTACAGACCA TGTGCGGCAG TGGCTGGAGT GGGCGGTGAA 500
AGAATATGGC CTTCCAGACG TCAACATCTT GTTATTCCAG AACATCGATG 550
GGAAGGAACT GTGCAAGATG ACCAAGGACG ACTTCCAGAG GCTCACCCCC 600
AGCTACAACG CCGACATCCT TCTCTCACAT CTCCACTACC TCAGAGAGAC 650
TCCTCTTCCA CATTTGACTT CAGATGATGT TGATAAAGCC TTACAAAACT 700
CTCCACGGTT AATGCATGCT AGAAACACAG GGGGTGCAGC TTTTATTTTC 750
CCAAATACTT CAGTATATCC TGAAGCTACG CAAAGAATTA CAACTAGGCC 800
AGGTACGAAA ACACCCCTGT GTGATCTCTT CATTGAGAGA CATCCCAGAT 850
GTCCTGCTGA GATCCGTGCC CTAAGTCACG TGATACAAAG AGAGCTGATC 900
CCGGAGCTGA AGCCAGTCCC AGACAGTCTT ATTCTGCCTC TGTTGATTTG 950
GAGACTAAAT CCACTCAAAC CATTTCATTC AAAGACCACA CTAAAGGAAT 1000
TAAGAGCAGA TTAGCCCTTT AACTAGCTTT TCAGAAAGAC AGATGGGCAA 1050
AGAAGGCATC CTGGATGCCT GGCAGTTAGG AATAGGCCGA CTTTTGAACT 1100
AACAGAAGGA TCTGTCCCTC CTCGGGGGAA GAGCACAAAA CAAGGACACT 1150
CCCCAGATTC ACAGTGAC
 (SEQ ID NO: 1). The TMPRSS2-derived sequence at nucleotides 1-75 is shown in bold font. Exon junctions are shown in grey boxes. The initiation codon and stop codon are shown in bold italics. The unique 3' sequence at nucleotides 803-1168 is also shown in bold font. The amino acid sequence of ERG8 is: 
MTASSSSDYG QTSKMSPRVP QQDWLSQPPA RVTIKMECNP SQVNGSRNSP 50
DECSVAKGGK MVGSPDTVGM NYGSYMEEKH MPPPNMTTNE RRVIVPADPT 100
LWSTDHVRQW LEWAVKEYGL PDVNILLFQN IDGKELCKMT KDDFQRLTPS 150
YNADILLSHL HYLRETPLPH LTSDDVDKAL QNSPRLMHAR NTGGAAFIFP 200
NTSVYPEATQ RITTRPGTKT PLCDLFIERH PRCPAEIRAL SHVIQRELIP 250
ELKPVPDSLI LPLLIWRLNP LKPFHSKTTL KELRAD
 (SEQ ID NO: 2). The unique carboxy terminus of ERG8 is shown in bold font.

The invention provides an isolated double-stranded nucleic acid molecule comprising a nucleic acid molecule with the polynucleotide sequence SEQ ID NO: 46, or the complement thereof. Also described is an isolated double-stranded nucleic acid molecule comprising a nucleic acid molecule with the polynucleotide sequence SEQ ID NO: 1 or SEQ ID NO: 30, or the complement of any of these. The entire nucleotide sequence of the ERG8/TMPRSS fusion molecule is: 
TAGGCGCGAG CTAAGCAGGA GGCGGAGGCG GAGGCGGAGG GCGAGGGGCG 50
GGGAGCGCCG CCTGGAGCGC GGCAGGAAGC CTTATCAGTT GTGAGTGAGG 100
ACCAGTCGTT GTTTGAGTGT GCCTACGGAA CGCCACACCT GGCTAAGACA 150
GAGATGACCG CGTCCTCCTC CAGCGACTAT GGACAGACTT CCAAGATGAG 200
CCCACGCGTC CCTCAGCAGG ATTGGCTGTC TCAACCCCCA GCCAGGGTCA 250
CCATCAAAAT GGAATGTAAC CCTAGCCAGG TGAATGGCTC AAGGAACTCT 300
CCTGATGAAT GCAGTGTGGC CAAAGGCGGG AAGATGGTGG GCAGCCCAGA 350
CACCGTTGGG ATGAACTACG GCAGCTACAT GGAGGAGAAG CACATGCCAC 400
CCCCAAACAT GACCACGAAC GAGCGCAGAG TTATCGTGCC AGCAGATCCT 450
ACGCTATGGA GTACAGACCA TGTGCGGCAG TGGCTGGAGT GGGCGGTGAA 500
AGAATATGGC CTTCCAGACG TCAACATCTT GTTATTCCAG AACATCGATG 550
GGAAGGAACT GTGCAAGATG ACCAAGGACG ACTTCCAGAG GCTCACCCCC 600
AGCTACAACG CCGACATCCT TCTCTCACAT CTCCACTACC TCAGAGAGAC 650
TCCTCTTCCA CATTTGACTT CAGATGATGT TGATAAAGCC TTACAAAACT 700
CTCCACGGTT AATGCATGCT AGAAACACAG GGGGTGCAGC TTTTATTTTC 750
CCAAATACTT CAGTATATCC TGAAGCTACG CAAAGAATTA CAACTAGGCC 800
AGGTACGAAA ACACCCCTGT GTGATCTCTT CATTGAGAGA CATCCCAGAT 850
GTCCTGCTGA GATCCGTGCC CTAAGTCACG TGATACAAAG AGAGCTGATC 900
CCGGAGCTGA AGCCAGTCCC AGACAGTCTT ATTCTGCCTC TGTTGATTTG 950
GAGACTAAAT CCACTCAAAC CATTTCATTC AAAGACCACA CTAAAGGAAT 1000
TAAGAGCAGA TTAGCCCTTT AACTAGCTTT TCAGAAAGAC AGATGGGCAA 1050
AGAAGGCATC CTGGATGCCT GGCAGTTAGG AATAGGCCGA CTTTTGAACT 1100
AACAGAAGGA TCTGTCCCTC CTCGGGGGAA GAGCACAAAA CAAGGACACT 1150
CCCCAGATTC ACAGTGACCG ATTATCAGTA TGTCACAAGA AGCCAGTCTT 1200
GCAGAGCAGA AGCATGCAAC CAGTAGTATT TACATCTGAA TCTTACTGCC 1250
TGTCCTCCAA ATGATTTAAT TAGGTAATAA ATTTACATGC CATTCATGCA 1300
AAAATAAACA TCTATCAAGT GCCCATTAGT GCCAAGCGTG GTGTTAGACT 1350
CTGGGAATAT ATAGATGAAC CAGGCTTCAG TAAGCTTCCT GTCTTCAGAA 1400
AGTTTACTTC TTCATTCAGC TTGGTTTGTT CATTTGCTGA GTGCCTCCTC 1450
TGTGCCAGCC ACGGATGGTA TGATGGTGAA CAAACCGAAA TGTTTTGCCT 1500
CCAGTTCTAG ATGTTTCAGT AGAGTGACCT AGAGCCAGAG AGACACATAT 1550
GTACACATAA ATGTTTTCCC TAATGTGATA GATTTTATGG TAGAGGAACC 1600
ACTTCTAGCA ATACAGGGCG TAGGAGCAGG GGTGGGGAGG AACTCAATCC 1650
CCCATGAAAG GCATAAAGTT GCTTTCCAGA GGAATGGCCA CATGGCAAAG 1700
GGGAATTAGA TGTTTGCCAG ACGAATAATG AGCAGGGAGA GAGGGCATTT 1750
CCCAGAAGGG TATAGCTTGC CTTTAGCATT TGTCCTCTCC CTGGGACTTA 1800
CATCAGCCCG ATAAGCTAGG TATCATTGTA CCAGCCTCAC AGCTGATGAC 1850
 
ATTGTGTTCA GGGTGGTGGG ATGGTTTCTC CATATTCATA CATGCTTCCA 1900
GAATTCATGT TAAACTCTAT CACATATCCG GAATACACAA GTCTCAGTTC 1950
GAACTGGTTC AAGATCTAGG CTTGGCAACT ACTCTTTCTT TCTAATGAGA 2000
AAGACTGGGG GCCCAGGGAG CTAAAGAGAA TGAATGAGGA AGCTTCTCAG 2050
GCTGTTCAAA TACTGACACT GCCCTGGTTA CTGCCTAGTG ACTTCAGGCT 2100
GGCAATTTTC TCTTCTCTAA CGTCAGAGAA AAAGTTTACT GTCTTGCTCC 2150
TGGGAAGCAT GATGGAAAGG CTTAGCAGCT AAGGGGTACT AAGAGGTAGT 2200
AAGTCATCTC TGTCATGTAA AAGATTTCAC AGGCCATTGA AACATGGGCA 2250
AGACCCAGTG CCTAGAGTCT GCAAGATTGGT CCTAAAGAC ATCCACCACG 2300
TGTATTGCGA GTGGAAAATA GAAATTCATG TTTGACTCAA GCTTTAGAGA 2350
TTTTGTAATT CTGTGAGCAT TTAAAAAATA TTTCCATATA AACTAAAAAA 2400
ATAAAAACTA TTTCCAAAAA AAAAAAAAAA AAAAACTCGA G 2441
 (SEQ ID NO: 30). SEQ ID NO: 1 is identical to nucleotides 1-1168 of SEQ ID NO: 30. The *TMPRSS2*-derived sequence is shown in bold font and the initiation codon and stop codon are shown in bold italics, also as shown in SEQ ID NO: 1. The shaded region, SEQ ID NO: 48, identifies an adenosine-rich area corresponding to nucleotides 1300-1310 of SEQ ID NO: 30. It aligns with the 3' end of AY204742, as shown below.
AAAAATAAACA (SEQ ID NO: 48)
AAAAAAAAAAA (SEQ ID NO: 49)
The two bolded nucleotides, T1305 and C1309 of SEQ ID NO: 30, both correspond to adenosines in the AY204742 sequence, suggesting that AY204742 may previously have been erroneously considered to possess a polyA tail at this site.

Nucleotides 802-2415 comprise a region specific to *ERG*8. This region encodes the unique carboxy terminus and also comprises a non-coding 3' region, as shown below: 
GTACGAAAAC ACCCCTGTGT GATCTCTTCA TTGAGAGACA TCCCAGATGT 50
CCTGCTGAGA TCCGTGCCCT AAGTCACGTG ATACAAAGAG AGCTGATCCC 100
GGAGCTGAAG CCAGTCCCAG ACAGTCTTAT TCTGCCTCTG TTGATTTGGA 150
GACTAAATCC ACTCAAACCA TTTCATTCAA AGACCACACT AAAGGAATTA 200
AGAGCAGATT AGCCCTTTAA CTAGCTTTTC AGAAAGACAG ATGGGCAAAG 250
AAGGCATCCT GGATGCCTGG CAGTTAGGAA TAGGCCGACT TTTGAACTAA 300
CAGAAGGATC TGTCCCTCCT CGGGGGAAGA GCACAAAACA AGGACACTCC 350
CCAGATTCAC AGTGACCGAT TATCAGTATG TCACAAGAAG CCAGTCTTGC 400
AGAGCAGAAG CATGCAACCA GTAGTATTTA CATCTGAATC TTACTGCCTG 450
TCCTCCAAAT GATTTAATTA GGTAATAAAT TTACATGCCA TTCATGCAAA 500
AATAAACATC TATCAAGTGC CCATTAGTGC CAAGCGTGGT GTTAGACTCT 550
 
GGGAATATAT AGATGAACCA GGCTTCAGTA AGCTTCCTGT CTTCAGAAAG 600
TTTACTTCTT CATTCAGCTT GGTTTGTTCA TTTGCTGAGT GCCTCCTCTG 650
TGCCAGCCAC GGATGGTATG ATGGTGAACA AACCGAAATG TTTTGCCTCC 700
AGTTCTAGAT GTTTCAGTAG AGTGACCTAG AGCCAGAGAG ACACATATGT 750
ACACATAAAT GTTTTCCCTA ATGTGATAGA TTTTATGGTA GAGGAACCAC 800
TTCTAGCAAT ACAGGGCGTA GGAGCAGGGG TGGGGAGGAA CTCAATCCCC 850
CATGAAAGGC ATAAAGTTGC TTTCCAGAGG AATGGCCACA TGGCAAAGGG 900
GAATTAGATG TTTGCCAGAC GAATAATGAG CAGGGAGAGA GGGCATTTCC 950
CAGAAGGGTA TAGCTTGCCT TTAGCATTTG TCCTCTCCCT GGGACTTACA 1000
TCAGCCCGAT AAGCTAGGTA TCATTGTACC AGCCTCACAG CTGATGACAT 1050
TGTGTTCAGG GTGGTGGGAT GGTTTCTCCA TATTCATACA TGCTTCCAGA 1100
ATTCATGTTA AACTCTATCA CATATCCGGA ATACACAAGT CTCAGTTCGA 1150
ACTGGTTCAA GATCTAGGCT TGGCAACTAC TCTTTCTTTC TAATGAGAAA 1200
GACTGGGGGC CCAGGGAGCT AAAGAGAATG AATGAGGAAG CTTCTCAGGC 1250
TGTTCAAATA CTGACACTGC CCTGGTTACT GCCTAGTGAC TTCAGGCTGG 1300
CAATTTTCTC TTCTCTAACG TCAGAGAAAA AGTTTACTGT CTTGCTCCTG 1350
GGAAGCATGA TGGAAAGGCT TAGCAGCTAA GGGGTACTAA GAGGTAGTAA 1400
GTCATCTCTG TCATGTAAAA GATTTCACAG GCCATTGAAA CATGGGCAAG 1450
ACCCAGTGCC TAGAGTCTGC AAGATTGGTC CTAAAGACAT CCACCACGTG 1500
TATTGCGAGT GGAAAATAGA AATTCATGTT TGACTCAAGC TTTAGAGATT 1550
TTGTAATTCT GTGAGCATTT AAAAAATATT TCCATATAAA CTAAAAAAAT 1600
AAAAACTATT TCC 1613
 (SEQ ID NO: 46). The ERG8 specific carboxyl terminus of the encoded polypeptide is: 
GTKTPLCDLF IERHPRCPAE IRALSHVIQR ELIPELKPVP DSLILPLLIW
RLNPLKPFHS KTTLKELRAD
 (SEQ ID NO: 47).

*EPC1* is an *ERG* isoform that is selectively expressed in cancerous prostate cells. The nucleic acid sequence of *EPC1* is: 
GCAGGAGGCG GAGGCGGAGG CGGAGGGCGA GGGGCGGGGA GCGCCGCCTG 50
GAGCGCGGCA GGAAGCCTTA TCAGTTGTGA GTGAGGACCA GTCGTTGTTT 100
GAGTGTGCCT ACGGAACGCC ACACCTGGCT AAGACAGAGA TGACCGCGTC 150
CTCCTCCAGC GACTATGGAC AGACTTCCAA GATGAGCCCA CGCGTCCCTC 200
AGCAGGATTG GCTGTCTCAA CCCCCAGCCA GGGTCACCAT CAAAATGGAA 250
TGTAACCCTA GCCAGGTGAA TGGCTCAAGG AACTCTCCTG ATGAATGCAG 300
TGTGGCCAAA GGCGGGAAGA TGGTGGGCAG CCCAGACACC GTTGGGATGA 350
ACTACGGCAG CTACATGGAG GAGAAGCACA TGCCACCCCC AAACATGACC 400
ACGAACGAGC GCAGAGTTAT CGTGCCAGCA GATCCTACGC TATGGAGTAC 450
AGACCATGTG CGGCAGTGGC TGGAGTGGGC GGTGAAAGAA TATGGCCTTC 500
 
CAGACGTCAA CATCTTGTTA TTCCAGAACA TCGATGGGAA GGAACTGTGC 550
AAGATGACCA AGGACGACTT CCAGAGGCTC ACCCCCAGCT ACAACGCCGA 600
CATCCTTCTC TCACATCTCC ACTACCTCAG AGAGACTCCT CTTCCACATT 650
TGACTTCAGA TGATGTTGAT AAAGCCTTAC AAAACTCTCC ACGGTTAATG 700
CATGCTAGAA ACACAGGGGG TGCAGCTTTT ATTTTCCCAA ATACTTCAGT 750
ATATCCTGAA GCTACGCAAA GAATTACAAC TAGGCCAGTC TCTTACAGAT 800
AAAACAACAG AACCAGTGCC AGAAAGCAGC CTTCCCTTAC ATGGGCACTT 850
CTGCCAAGCA TATGAGTTCA TTGCCTTGAA GATCAAAGTC AAAGAGAAAT 900
GGAGAGGGTG TTGAAATGAT CAGCGAAAAT TAAATGTAAA ATATATTCTT 950
ATTGGAAGTC TGATGCTCTA TTATCAATAA AGGACACATA GCAAAGATAA 1000
AAAAAAAAAA AAAAAAAAA
 (SEQ ID NO: 3). In the sequence, the *TMPRSS2*-derived sequence is shown in bold font. Exon junctions are shown in grey boxes. The initiation codon and stop codon are shown in bold italics. The 3' end of the *EPC1* transcript is distinct from all known *ERG* isoforms. This unique sequence is shown in bold font. The amino acid sequence of EPC1 is: 
MTASSSSDYG QTSKMSPRVP QQDWLSQPPA RVTIKMECNP SQVNGSRNSP 50
DECSVAKGGK MVGSPDTVGM NYGSYMEEKH MPPPNMTTNE RRVIVPADPT 100
LWSTDHVRQW LEWAVKEYGL PDVNILLFQN IDGKELCKMT KDDFQRLTPS 150
YNADILLSHL HYLRETPLPH LTSDDVDKAL QNSPRLMHAR NTGGAAFIFP 200
NTSVYPEATQ RITTRPVSYR
 (SEQ ID NO: 4). *EPC1* comprises additional nucleotides at its 3' end that encode four unique amino acids at the carboxy terminus of the EPC1 protein. These four unique amino acids are underlined in SEQ ID NO: 4. Because *EPC1,* like *ERG8*, lacks the coding sequences for the DNA-binding domain, it may also have a dominant negative effect.

*EPC2* is also selectively expressed in cancerous prostate cells. The nucleic acid sequence of *EPC2* is: 
ACATCTTGTT ATTCCAGAAC ATCGATGGGA AGGAACTGTG CAAGATGACC 50
AAGGACGACT TCCAGAGGCT CACCCCCAGC TACAACGCCG ACATCCTTCT 100
CTCACATCTC CACTACCTCA GAGAGAGTAA GCTCCCCCTT CCTCCAAGGA 150
TAGATGGCTG TGGCTATGGT TCTTATGACC CGAGCTTCAG AGGGTTCAAC 200
CAGGTGTGTC GACAGCATCC TCCTGCCCTC GCCCAGTTCC CACTGGGGAT 250
CCGAGGGAGC CACATGCTTG GGTCCTGCGA CCAAGAAGAT GGAATGTCAA 300
AGGGGAAAGG AAGCGTTAAC TGGTCACACA TTAGTTAAGT CTCCATGATA 350
CCCCGAATCA AAATAGAATC ATTAAGGCTT CTCTTTCGTA GGAATTAGGG 400
GGATTATTCT CCCTAAAGCT ACATGAAGCC CCACTTTATA TTCTAACCTG 450
AGCACAGAAC AAGGGAAGTT TTCACTTTGT ATCATGTGAT TCGGCTTAAC 500
CTGACAGAAA GGGATGGCAT GTTGGCATGA ATCCAGAATG TTTGCTGCAT 550
 
GCTTTAATTT CTACAACGTC CAGCATGGTG AGAAGGAAGT AGTGTGACAG 600
ACAGTGAGGT GGATAAATTC TCCTCCATTG CTTTGCCTGG CATCCCAACC 650
ACTTCTTCCC TGAATTAAAG ACGGGCCCCC ATGTAGGTTT TAACATGCTA 700
ACAAGTAGCA GGTTGCTGGA AATAGTTATA AGCTTCCCAT GATGTTAGTG 750
TGGGAGTGGG GGAACGGTTT CTTTCTTTCT TTTTCTTTCT TTTTTTTTTT 800
TTTTTTT
 (SEQ ID NO: 5). The initiation codon and stop codon are shown in bold italics. An exon junction is shown in the grey box. The unique 3' sequence is shown in bold font. The amino acid sequence of EPC2 is:
MTKDDFQRLT PSYNADILLS HLHYLRESKL PLPPRIDGCG YGSYDPSFRG 50
FNQVCRQHPP ALAQFPLGIR GSHMLGSCDQ EDGMSKGKGS VNWSHIS
(SEQ ID NO: 6). The unique carboxy terminus of EPC2 is shown in bold font in SEQ ID NO: 6.

The disclosure also describes the activation of a promoter in prostate cancer cells. Activation of this promoter produces transcripts coding for *ERG* isoforms lacking the N-terminal protein-protein interaction domain of wild type ERG. Therefore, expression products of this promoter sequence in prostate cancer cells appear to act as dominant negative or gain-of-function molecules. The promoter is located within the following sequence from exon 9 of the *ERG* gene: 
TCTGTCGCCA GTCTGGAGTG CAGTGGCATG ATCTCAGCTC ACTGCAACCT 50
CCACCTCCCG GATTCAAGCA ATTTTCCTGC CTCAGCCTCC TGAGTAGCTG 100
GGACTACAGG CATGCCCAGC TAATTTTTGT ATTTTTAGTA GAGACGGGGT 150
TTCACCATGT TGGCCAGGAT GGTCTGGATC TCTTGACCTC ATGATCCGCC 200
CACCTCGGCA TCCCAAAGTG TTGGGACTAC AGGCATGAGC CACGGCACCC 250
CGCCTGTATT TGGCTTTTCA CACTTGTCCT TTCTCCCCCA GTCTCTTCCG 300
CCTTGCCCTT CTTTGGTTCT CTCTGTGTAT TGTGAGAAGT CGATGGAGAC 350
ATGCTCTTTG ATTGCTGTTA TAATGGAAGA ATATTTCTTC TCCTCCAGGA 400
ACTCTCCTGA TGAATGCAGT GTGGCCAAAG GCGGGAAGAT GGTGGGCAGC 450
CCAGACACCG TTGGGATGAA CTACGGCAGC TACATGGAGG AGAAGCACAT 500
GCCACCCCCA AACATGACCA CGAACGAGCG CAGAGTTATC GTGCCAGCAG 550
GTCAGGTGCC CACAGCTTCA CTGCCCTCGG CAGATCGCAA CTTCCCCAAG 600
GCTAGGCTGA GCCTCAGGGA GCTCTTCTCC CCCACCTGTG GCATTGATCA 650
 (SEQ ID NO: 7). In the sequence, the most 3' transcription start site is bolded and shown in a grey box. A sequence comprising at least nucleotides 521 to 650 of SEQ ID NO: 7 retain promoter activity.

### Diagnostic Compositions and Methods

The *ERG* isoform nucleic acids, the polypeptides they encode, and antibodies to those polypeptides can be employed in various diagnostic and prognostic applications for prostate cancer because *ERG8, EPC1, EPC2,* and the transcripts from the prostate cancer-specific promoter are each associated with prostate cancer.

Accordingly, the disclosure provides methods for detecting prostate cancer in a biological sample, comprising combining the biological sample with at least a first and a second oligonucleotide primer under hybridizing conditions, wherein the first oligonucleotide primer contains a sequence that hybridizes to a first sequence in a target sequence from *ERG8, EPC1, EPC2,* or the transcripts from the prostate cancer-specific promoter and the second oligonucleotide primer contains a sequence that hybridizes to a second sequence in a nucleic acid strand complementary to the target sequence, wherein the first sequence does not overlap with the second sequence; amplifying a plurality of amplification products when the target sequence is present in the biological sample by adding at least one polymerase activity to the biological sample containing the first and second oligonucleotide primers; immobilizing the plurality of amplification products on a solid support; combining an oligonucleotide probe with the immobilized plurality of amplification products to thereby permit the probe to hybridize to at least one immobilized amplification product; and detecting whether a signal results from hybridization between the oligonucleotide probe and at least one amplification product, wherein detection of the signal indicates the expression of *ERG8, EPC1, EPC2,* or the transcripts from the prostate cancer-specific promoter and the presence of prostate cancer in the biological sample. Detecting a signal resulting from hybridization between the oligonucleotide probe and the at least one amplification product can be used to diagnose or prognose prostate cancer.

In some embodiments in which the *ERG* isoform is fused to *TMPRSS2,* the first oligonucleotide primer contains a sequence that hybridizes to a first sequence in a target sequence from *TMPRSS2* and the second oligonucleotide primer contains a sequence that hybridizes to a second sequence in a nucleic acid strand complementary to a target sequence from *ERG8, EPC1, EPC2,* or the transcripts from the prostate cancer-specific promoter.

Accordingly, the disclosure provides methods for detecting prostate cancer in a biological sample, wherein the target sequence comprises all or part of SEQ ID NO: 1, SEQ ID NO: 3, SEQ ID NO: 5, SEQ ID NO: 7, SEQ ID NO: 30, or SEQ ID NO: 46. In other embodiments, the target sequence comprises nucleotides 75 to 1168 of SEQ ID NO: 1, nucleotides 803 to 1168 of SEQ ID NO: 1, nucleotides 61 to 1019 of SEQ ID NO: 3, nucleotides 788 to 1019 of SEQ ID NO: 3, a nucleic acid molecule comprising SEQ ID NO: 5, nucleotides 127 to 807 of SEQ ID NO: 5, nucleotides 942-1046 of SEQ ID NO: 46, nucleotides 1285-1374 of SEQ ID NO: 46, or nucleotides 1451-1541 of SEQ ID NO: 46.

In some embodiments, the oligonucleotide probe(s), rather than the amplification products, may be optionally fixed to a solid support.

In yet other embodiments, the immobilization and subsequent steps are omitted and the plurality of amplification products are detected by size separation followed by staining with a reagent, such as ethidium bromide, that detects DNA. This embodiment may optionally further comprise photographing the stained DNA to preserve the results. In these embodiments, detection of the amplification products can be used to diagnose or prognose prostate cancer as well.

When detecting *ERG* isoform expression in a biological sample, the oligonucleotide probe, first oligonucleotide primer, and second oligonucleotide primer, each comprise a nucleic acid sequence that is capable of hybridizing under defined conditions (for example under high stringency hybridization conditions; such as hybridization for 12 hours at 65°C in 6x SSC followed by a wash in 0.1x SSC at 50°C for 45 minutes) to a nucleic acid sequence of an *ERG* isoform. Thus, the oligonucleotide probe, first oligonucleotide primer, and second oligonucleotide primer comprises, for example, a nucleic acid sequence of an *ERG* isoform, such as SEQ ID NO: 1 (*ERG8*), SEQ ID NO: 3 (*EPC1*), SEQ ID NO: 5 (*EPC*2), SEQ ID NO: 30 (*ERG*8), SEQ ID NO: 46 (*ERG8*), a transcript from the prostate cancer-specific promoter (SEQ ID NO: 7), or a nucleic acid molecule comprising a fragment thereof, or a sequence complementary thereto. The oligonucleotide probe, first oligonucleotide primer, or second oligonucleotide primer can be a fragment comprising at least about 15, at least about 20, at least about 30, at least about 40, or at least about 50 contiguous nucleotides of a nucleic acid sequence of *ERG8, EPC1, EPC2,* or a transcript from the prostate cancer-specific promoter, or a sequence complementary thereto.

In some embodiments, the methods comprise detecting the expression of the *ERG8* isoform. In other embodiments, expression of the *EPC1* isoform is detected. In yet other embodiments, expression of the *EPC2* isoform is detected. While in some embodiments, transcripts from the prostate cancer-specific promoter are detected. In still other embodiments, the methods comprise detecting the *ERG8* and *EPC1* isoforms in combination, the *ERG8* and *EPC2* isoforms in combination, the *EPC1* and *EPC2* isoforms in combination, or the combination of the *ERG8, EPC1,* and *EPC2* isoforms. In other embodiments, the method comprises detecting one or more transcripts from the prostate cancer-specific promoter either alone or in combination with one or more of ERG8, EPC1, or EPC2. In some embodiments, the methods further comprise detecting other prostate cancer-specific markers, such as ERG1, ERG2, PSA, DD3, AMAR, LTF, NPY, SPOCK, CRISP3, PLA2G7, TMEFF2, F5, SMOC, ACPP, TGM4, MSMB, WIF1, OLFM4, PI15, PDGFD, CHGA, CAV1, RLN1, IGFBP7, BGN, FMOD, AGR2, SERPINA3, AZGP1, FAM3B, CD164, or the presence of a *TMPRSS-ERG* fusion.

Polypeptides encoded by *ERG8, EPC1,* or *EPC2* can also be detected and/or measured in a biological sample. For example, antibodies, optionally labeled, can be used to detect each polypeptide using well known techniques, such as ELISA. The biological sample can be, *e.g.,* prostate tissue, blood, serum, plasma, urine, saliva, or prostatic fluid.

In another aspect, the disclosure provides a method of diagnosing or prognosing prostate cancer, comprising measuring the expression level (*e.g.* mRNA or polypeptide) of *ERG8, EPC1, EPC2* or a transcript from the prostate cancer-specific promoter; and correlating the expression level of an ERG isoform with the presence of prostate cancer or a higher predisposition to develop prostate cancer in the subject.

The skilled artisan will understand how to correlate expression levels or patterns of *ERG8, EPC1, EPC2,* or a transcript from the prostate cancer-specific promoter with the presence of prostate cancer or a higher predisposition to develop prostate cancer. For example, the expression levels can be quantified such that increased or decreased expression levels relative to a control sample or other standardized value or numerical range indicate the presence of prostate cancer or a higher predisposition to develop prostate cancer.

The increased or decreased expression levels may be measured relative to the expression level of *ERG8, EPC1, EPC2,* or a transcript from the prostate cancer-specific promoter, or the corresponding polypeptide, in normal, matched tissue, such as benign prostate epithelial cells from the same subject. Alternatively, the expression level of *ERG8, EPC1, EPC2,* or a transcript from the prostate cancer-specific promoter, or the corresponding polypeptide, may be measured relative to the expression of the gene or polypeptide in other noncancerous samples from the subject or in samples obtained from an individual who does not have cancer. Expression of a gene or the corresponding polypeptide may also be normalized by comparing it to the expression of other cancer-specific markers. For example, a prostate specific marker, such as PSA or *TMPRSS2-ERG,* can be used as a control to compare and/or normalize expression levels of *ERG8, EPC1, EPC2,* or a transcript from the prostate cancer-specific promoter, or the corresponding polypeptide.

By way of example, the method of diagnosing or prognosing prostate cancer can comprise measuring the expression levels of the *ERG8, EPC1, EPC2,* or a transcript from the prostate cancer-specific promoter, isoforms, or any combination thereof, and diagnosing or prognosing prostate cancer, where an increased expression level of *ERG8, EPC1,* or *EPC2* of at least 10%, 20%, 30%, 40%, 50%, 60%, 70%, 80%, 90%, 100%, 200%, or more, as compared to the control sample indicates the presence of prostate cancer or a higher predisposition in the subject to develop prostate cancer, or indicates the severity or stage of prostate cancer, such as whether the cancer is a high risk or a moderate risk prostate cancer.

The expression levels of *ERG8, EPC1, EPC2,* or a transcript from the prostate cancer-specific promoter (*e.g.,* mRNA or polypeptide expression) can be detected according to the methods described herein or using any other known detection methods, including, without limitation, immunohistochemistry, Southern blotting, northern blotting, western blotting, ELISA, and nucleic acid amplification procedures that include but not limited to PCR, transcription-mediated amplification (TMA), nucleic acid sequence-based amplification (NASBA), self-sustained sequence replication (3SR), ligase chain reaction (LCR), strand displacement amplification (SDA), and loop-mediated isothermal amplification (LAMP).

Nucleic acids are also provided for detecting prostate cancer, and one or more of these nucleic acids may optionally be provided as part of a kit. In some embodiments, the nucleic acid is a nucleic acid probe, such as the probes described elsewhere in the disclosure, that hybridizes to a prostate cancer-specific transcript. For example, in one embodiment, the probe is capable of hybridizing to the desired sequence under high stringency hybridization conditions, such as hybridization for 12 hours at 65°C in 6x SSC followed by a wash in 0.1x SSC at 50°C for 45 minutes. The probe can include SEQ ID NO: 1, SEQ ID NO: 30, or SEQ ID NO: 46 itself, or a fragment of SEQ ID NO: 1, SEQ ID NO: 30, or SEQ ID NO: 46 comprising at least about 15, 20, 30, 40, 50, 60, 70, 80, 90, 100, 150, or 200 contiguous nucleotides of SEQ ID NO: 1, SEQ ID NO: 30, or SEQ ID NO: 46, or a sequence complementary thereto. In one embodiment, the fragment comprises all or part of nucleotides 75 to 1168 of SEQ ID NO: 1. For example, the fragment may comprise nucleotides 801 to 1168 of SEQ ID NO: 1, or a nucleic acid molecule comprising at least about 15, 20, 30, 40, 50, 60, 70, 80, 90, 100, 150, or 200 contiguous nucleotides of nucleotides 801 to 1168 of SEQ ID NO: 1. Also by way of example, the fragment may comprise nucleotides 942-1046 of SEQ ID NO: 46, nucleotides 1285-1374 of SEQ ID NO: 46, or nucleotides 1451-1541 of SEQ ID NO: 46.

In some embodiments, the probe selectively hybridizes to the *ERG8* isoform but does not hybridize to *ERG1, ERG2, ERG3, ERG4, ERG5, ERG16, ERG7, ERG9, EPC1, EPC2,* a transcript from the prostate cancer-specific promoter, or *TMPRSS2* under defined conditions, including, for example, high stringency hybridization conditions. The length of the probe may vary depending, for example, on the hybridization conditions and the percent identify between the target sequence and the probe, and, therefore can be up to about 6, 10, 20, 30, 40, 50, 100, 150, 200, 300, 400, or 500 nucleotides long.

In some embodiments, therefore, the disclosure provides an isolated nucleic acid comprising at least about 15 contiguous nucleotides of nucleotides 801 to 1168 of SEQ ID NO: 1, wherein the nucleic acid is capable of hybridizing to SEQ ID NO: 1, or the complement thereof, under conditions of high stringency but not to *ERG1, ERG2, ERG3, ERG4, ERG5, ERG6, ERG7, ERG9, EPC1, EPC2,* a transcript from the prostate cancer-specific promoter, or *TMPRSS2.* In some embodiments, the nucleic acid is up to about 50 nucleotides long. In other embodiments the probe is capable of hybridizing to the desired sequence under conditions of high stringency comprising hybridization for 12 hours at 65°C in 6x SSC followed by a wash in 0.1x SSC at 50°C for 45 minutes.

In another embodiment, the probe hybridizes to SEQ ID NO: 3, or to a sequence within nucleotides 61 to 1019 or 788 to 1068 of SEQ ID NO: 3 (*EPC1*), or to the complement thereof, under defined hybridization conditions. For example, in one embodiment, the probe is capable of hybridizing to the desired sequence under high stringency hybridization conditions, such as, hybridization for 12 hours at 65°C in 6x SSC followed by a wash in 0.1x SSC at 50°C for 45 minutes. The probe can include SEQ ID NO: 3 itself, or a fragment of SEQ ID NO: 3 comprising at least about 15, 20, 30, 40, 50, 60, 70, 80, 90, 100, 150, or 200 contiguous nucleotides of SEQ ID NO: 3, or a sequence complementary thereto. In one embodiment, the fragment comprises all or part of nucleotides 61 to 1019 of SEQ ID NO: 3. For example, the fragment may comprise nucleotides 788 to 1019 of SEQ ID NO: 3, or a nucleic acid molecule comprising at least about 15, 20, 30, 40, 50, 60, 70, 80, 90, 100, 150, or 200 contiguous nucleotides of nucleotides 788 to 1019 of SEQ ID NO: 3. In some embodiments, the probe selectively hybridizes to *EPC1* but not to *ERG1, ERG2, ERG3, ERG4, ERG5, ERG6, ERG7, ERG8, ERG9, EPC2,* a transcript from the prostate cancer-specific promoter, or *TMPRSS2* under defined conditions, including, for example, high stringency hybridization conditions. The length of the probe may vary depending, for example, on the hybridization conditions and the percent identify between the target sequence and the probe, and, therefore can be up to about 6, 10, 20, 30, 40, 50, 100, 150, 200, 300, 400, or 500 nucleotides long.

In some embodiments, therefore, the disclosure provides an isolated nucleic acid comprising at least about 15 contiguous nucleotides of nucleotides 788 to 1019 of SEQ ID NO: 3, wherein the nucleic acid is capable of hybridizing to SEQ ID NO: 3, or the complement thereof, under conditions of high stringency but not to *ERG1, ERG2, ERG3, ERG4, ERG5, ERG6, ERG7*, *ERG8*, *ERG9, EPC2,* a transcript from the prostate cancer-specific promoter, or *TMPRSS2.* In some embodiments, the nucleic acid is up to about 50 nucleotides long. In other embodiments the probe is capable of hybridizing to the desired sequence under conditions of high stringency comprising hybridization for 12 hours at 65°C in 6x SSC followed by a wash in 0.1x SSC at 50°C for 45 minutes.

In a further embodiment, the probe hybridizes to SEQ ID NO: 5 *(EPC2)* or to nucleotides 127 to 807 of SEQ ID NO: 5, or to the complement thereof, under defined hybridization conditions. For example, in one embodiment, the probe is capable of hybridizing to the desired sequence under high stringency hybridization conditions, such as, hybridization for 12 hours at 65°C in 6x SSC followed by a wash in 0.1 x SSC at 50°C for 45 minutes. The probe can include SEQ ID NO: 5 itself, or a fragment of SEQ ID NO: 5 comprising at least about 15, 20, 30, 40, 50, 60, 70, 80, 90, 100, 150, or 200 contiguous nucleotides of SEQ ID NO: 5 or a sequence complementary thereto. In one embodiment, the fragment comprises all or part of nucleotides 127 to 807 of SEQ ID NO: 5. For example, the fragment may comprise nucleotides 127 to 807 of SEQ ID NO: 5, or a nucleic acid molecule comprising at least about 15, 20, 30, 40, 50, 60, 70, 80, 90, 100, 150, or 200 contiguous nucleotides of nucleotides 127 to 807 of SEQ ID NO: 5. In some embodiments, the probe selectively hybridizes to *EPC2* but not to *ERG1, ERG2, ERG3, ERG4, ERG5, ERG6, ERG7, ERG8, ERG9, EPC1,* a transcript from the prostate cancer-specific promoter, or *TMPRSS2* under defined conditions, including, for example, high stringency hybridization conditions. The length of the probe may vary depending, for example, on the hybridization conditions and the percent identify between the target sequence and the probe, and, therefore can be up to about 6, 10, 20, 30, 40, 50, 100, 150, 200, 300, 400, or 500 nucleotides long.

In some embodiments, therefore, the disclosure provides an isolated nucleic acid, comprising at least about 15 contiguous nucleotides of nucleotides 127 to 807 of SEQ ID NO: 5, wherein the nucleic acid is capable of hybridizing to SEQ ID NO: 5, or the complement thereof, under conditions of high stringency but not to *ERG1, ERG2, ERG3, ERG4, ERG5, ERG6, ERG7, ERG8, ERG9, EPC2,* a transcript from the prostate cancer-specific promoter, or *TMPRSS2.* In some embodiments, the nucleic acid is up to about 50 nucleotides long. In other embodiments the probe is capable of hybridizing to the desired sequence under conditions of high stringency comprising hybridization for 12 hours at 65°C in 6x SSC followed by a wash in 0.1x SSC at 50°C for 45 minutes.

In some embodiments, therefore, the disclosure provides an isolated nucleic acid comprising at least about 15 contiguous nucleotides of nucleotides 942-1046, 1285-1374, or 1451-1541 of SEQ ID NO: 46, wherein the nucleic acid is capable of hybridizing to SEQ ID NO: 46, or the complement thereof, under conditions of high stringency but not to *ERG1, ERG2, ERG3, ERG4, ERG5, ERG6, ERG7, ERG9, EPC1, EPC2,* a transcript from the prostate cancer-specific promoter, or *TMPRSS2.* In some embodiments, the nucleic acid is up to about 50 nucleotides long. In other embodiments, the probe is capable of hybridizing to the desired sequence under conditions of high stringency comprising hybridization for 12 hours at 65°C in 6x SSC followed by a wash in 0.1x SSC at 50°C for 45 minutes.

A nucleic acid probe may be optionally fixed to a solid support.

In other embodiments, the nucleic acid is an oligonucleotide primer. The disclosure provides a number of oligonucleotide primers and primer pairs, such as those described in the examples. In some embodiments, an oligonucleotide primer pair comprise a first oligonucleotide primer and a second oligonucleotide primer, where the first oligonucleotide primer contains a sequence that hybridizes to a first sequence in SEQ ID NO: 1, SEQ ID NO: 30, and/or SEQ ID NO: 46 and the second oligonucleotide primer contains a sequence that hybridizes to a second sequence in a nucleic acid strand complementary to SEQ ID NO: 1, SEQ ID NO: 30, and/or SEQ ID NO: 46, wherein the first sequence does not overlap with the second sequence. The first and second oligonucleotide primers are capable of amplifying a target sequence of interest in ERG8. Thus, in some embodiments the primer pairs amplify a target sequence comprising all or part of nucleotides 75 to 1168 of SEQ ID NO: 1, all or part of nucleotides 801 to 1168 of SEQ ID NO: 1, all or part of nucleotides 942-1046 of SEQ ID NO: 46, all or part of nucleotides 1285-1374 of SEQ ID NO: 46, or all or part of nucleotides 1451-1541 of SEQ ID NO: 46. In other embodiments, the target sequence comprises a nucleic acid molecule within nucleotides 75 to 1168 of SEQ ID NO: 1, nucleotides 801 to 1168 of SEQ ID NO: 1, or nucleotides 942-1046 of SEQ ID NO: 46, nucleotides 1285-1374 of SEQ ID NO: 46, or nucleotides 1451-1541 of SEQ ID NO: 46.

In some embodiments, the primer pair amplifies a target sequence that selectively hybridizes to the *ERG8* isoform but does not hybridize to *ERG1, ERG2, ERG3, ERG4, ERG5, ERG6, ERG7, ERG9, EPC1, EPC2,* a transcript from the prostate cancer-specific promoter, or *TMPRSS2* under defined conditions, including, for example, high stringency hybridization conditions, such as, hybridization for 12 hours at 65°C in 6x SSC followed by a wash in 0.1x SSC at 50°C for 45 minutes.

In yet other embodiments, an oligonucleotide primer pair comprise a first oligonucleotide primer and a second oligonucleotide primer, where the first oligonucleotide primer contains a sequence that hybridizes to a first sequence in SEQ ID NO: 3 and the second oligonucleotide primer contains a sequence that hybridizes to a second sequence in a nucleic acid strand complementary to SEQ ID NO: 3, wherein the first sequence does not overlap with the second sequence. The first and second oligonucleotide primers are capable of amplifying a target sequence of interest in *EPC1.* Thus, in some embodiments the primer pairs amplify a target sequence comprising all or part of nucleotides 61 to 1019 of SEQ ID NO: 3 or all or part of nucleotides 788 to 1019 of SEQ ID NO: 3. In other embodiments, the target sequence comprises a nucleic acid molecule within nucleotides 61 to 1019 of SEQ ID NO: 3 or nucleotides 788 to 1019 of SEQ ID NO: 3. In some embodiments, the primer pair amplify a target sequence that selectively hybridizes to the *EPC1* isoform but do not hybridize to *ERG1, ERG2, ERG3, ERG4, ERG5, ERG6, ERG7, ERG8, ERG9, EPC2,* a transcript from the prostate cancer-specific promoter, or *TMPRSS2* under defined conditions, including, for example, high stringency hybridization conditions, such as, hybridization for 12 hours at 65°C in 6x SSC followed by a wash in 0.1 x SSC at 50°C for 45 minutes.

In still other embodiments, an oligonucleotide primer pair comprise a first oligonucleotide primer and a second oligonucleotide primer, where the first oligonucleotide primer contains a sequence that hybridizes to a first sequence in SEQ ID NO: 5 and the second oligonucleotide primer contains a sequence that hybridizes to a second sequence in a nucleic acid strand complementary to SEQ ID NO: 5, wherein the first sequence does not overlap with the second sequence. The first and second oligonucleotide primers are capable of amplifying a target sequence of interest in *EPC2.* Thus, in some embodiments the primer pairs amplify a target sequence comprising all or part of SEQ ID NO: 5 or all or part of nucleotides 127 to 807 of SEQ ID NO: 5. In other embodiments, the target sequence comprises a nucleic acid molecule within SEQ ID NO: 5 or nucleotides 127 to 807 of SEQ ID NO: 5. In some embodiments, the primer pair amplify a target sequence that selectively hybridizes to the *EPC2* isoform but do not hybridize to *ERG1, ERG2, ERG3, ERG4, ERG5, ERG6, ERG7, ERG8, ERG9, EPC1,* a transcript from the prostate cancer-specific promoter, or *TMPRSS2* under defined conditions, including, for example, high stringency hybridization conditions, such as, hybridization for 12 hours at 65°C in 6x SSC followed by a wash in 0.1 x SSC at 50°C for 45 minutes.

The oligonucleotide primers and primer pairs can be provided in kit form. In some embodiments, the kits comprise a pair of oligonucleotide primers that is capable of amplifying a target sequence of interest in *ERG8,* such as those discussed elsewhere in the disclosure, a pair of oligonucleotide primers that is capable of amplifying a target sequence of interest in *EPC1,* such as those discussed elsewhere in the disclosure, and/or a pair of oligonucleotide primers that is capable of amplifying a target sequence of interest in *EPC2*, such as those discussed elsewhere in the disclosure. In this and other embodiments, it is not necessary for the oligonucleotide primers to all have different sequences. For example, it is possible to amplify target sequences that are specific for each of *ERG8, EPC1, EPC2,* or a transcript from the prostate cancer-specific promoter, by selecting an oligonucleotide primer that hybridizes to a nucleotide sequence, or complement thereof, that is unique to *ERG8,* an oligonucleotide primer that hybridizes to a nucleotide sequence, or complement thereof, that is unique to *EPC1,* an oligonucleotide primer that hybridizes to a nucleotide sequence, or complement thereof, that is unique to *EPC2,* an oligonucleotide primer that hybridizes to a nucleotide sequence, or complement thereof, that is unique to a transcript from the prostate cancer-specific promoter, and an oligonucleotide primer that hybridizes to a nucleotide sequence, or complement thereof, that is shared by *ERG8, EPC1,* and *EPC2.* Thus, it is possible to use only four oligonucleotide primers to selectively amplify target sequences in each of *ERG8, EPC1,* and *EPC2.* Other combinations of primers can be selected to amplify, for example, *ERG8* and *EPC1, ERG8* and *EPC2, EPC1* and *EPC2,* or one of more of those isoforms in combination with a transcript from the prostate cancer-specific promoter.

The disclosure additionally describes diagnostic kits comprising an anti-ERG isoform-specific antibody, for example, an anti-ERG8 antibody, an anti-EPC1 antibody, or anti-EPC2 antibody. In one embodiment, the disclosure provides an anti-EPC1 antibody that binds an epitope comprising amino acids 217 to 220 of SEQ ID NO: 4. In another embodiment, the antibody is an anti-EPC2 antibody that binds an epitope within or comprising amino acids 28 to 97 of SEQ ID NO: 6. In either case, the epitope can be a linear epitope or a conformational epitope. In some embodiments, combinations of antibodies can be included in the kit. For example, a kit can comprise an anti-ERG8 and an anti-EPC1 antibody, an anti-ERG8 and an anti-EPC2 antibody, an anti-EPC1 and an anti-EPC2 antibody, or an anti-ERG8, an anti-EPC1, and an anti-EPC2 antibody. The antibodies can be, optionally, detectably labeled. The antibodies can be used in both diagnostic and prognostic applications, as described for the nucleic acid probes and primers.

The nucleic acids, polypeptides, and antibodies for use in diagnosing and prognosing prostate cancer are generally formulated with a pharmaceutically acceptable carrier. When a nucleic acid, polypeptide, or antibody is part of a kit, an agent that reduces or inhibits the growth of microorganisms, such as sodium azide, can optionally be included in the formulation.

### Therapeutic Compositions and Uses

The *ERG* isoform (*e.g., ERG8, EPC1, EPC2,* a transcript from the prostate cancer-specific promoter, ERG1, ERG2, or ERG3) nucleic acids, the polypeptides they encode, and antibodies to those polypeptides can be combined with a suitable pharmaceutical carrier. The resulting pharmaceutical compositions can be used in various applications, such as diagnostic applications already described, and also in therapeutic applications. When the application is therapeutic, the compositions comprise a therapeutically effective amount of the nucleic acid, polypeptide, or antibody and a pharmaceutically acceptable carrier or excipient. Such a carrier includes, but is not limited to, saline, buffered saline, dextrose, water, glycerol, ethanol, and combinations thereof. The formulation should suit the mode of administration.

In therapeutic applications, the *ERG* isoform (*e.g., ERG8, EPC1, EPC2,* a transcript from the prostate cancer-specific promoter, ERG1, ERG2, or ERG3) nucleic acids, polypeptides, compounds used for destabilization, small molecule inhibitors, and antibody compositions will be formulated and dosed in a fashion consistent with good medical practice, taking into account the clinical condition of the individual subject, the site of delivery, the method of administration, the scheduling of administration, and other factors known to practitioners. The effective amount of *ERG* isoform (*e.g., ERG8, EPC1, EPC2,* a transcript from the prostate cancer-specific promoter, ERG1, ERG2, or ERG3) nucleic acids, polypeptides, compounds used for destabilization, small molecule inhibitors, and antibody compositions for purposes herein is thus determined by such considerations.

The disclosure also provides pharmaceutical packs or kits comprising one or more containers filled with one or more of the ingredients of the pharmaceutical compositions described. Associated with such container(s) can be a notice in the form prescribed by a governmental agency regulating the manufacture, use or sale of pharmaceuticals or biological products, which notice reflects approval by the agency of manufacture, use or sale for human administration. In addition, the *ERG* isoform (*e.g., ERG8, EPC1, EPC2,* a transcript from the prostate cancer-specific promoter, ERG1, ERG2, or ERG3) nucleic acids, polypeptides, compounds used for destabilization, small molecule inhibitors, and antibody compositions may be employed in conjunction with other therapeutic compounds.

The pharmaceutical compositions may be administered in a convenient manner such as by the oral, topical, intravenous, intraperitoneal, intramuscular, subcutaneous, intranasal, or intradermal routes. The pharmaceutical compositions are administered in an amount which is effective for treating and/or prophylaxis of the specific indication. In general, they are administered in an amount of at least about 10 micrograms/kg body weight and in most cases they will be administered in an amount not in excess of about 8 milligrams/kg body weight per day.

In pharmaceutical dosage forms, the disclosed compositions can be administered in the form of their pharmaceutically acceptable salts, or they can also be used alone or in appropriate association, as well as in combination, with other pharmaceutically active compounds. The subject compositions are formulated in accordance to the mode of potential administration. Administration of the agents can be achieved in various ways, including oral, buccal, nasal, rectal, parenteral, intraperitoneal, intradermal, transdermal, subcutaneous, intravenous, intra-arterial, intracardiac, intraventricular, intracranial, intratracheal, and intrathecal administration, etc., or otherwise by implantation or inhalation. Thus, the subject compositions can be formulated into preparations in solid, semi-solid, liquid or gaseous forms, such as tablets, capsules, powders, granules, ointments, solutions, suppositories, enemas, injections, inhalants and aerosols. Uses and excipients mentioned elsewhere in the disclosure are merely exemplary and are in no way limiting.

Compositions for oral administration can form solutions, suspensions, tablets, pills, granules, capsules, sustained release formulations, oral rinses, or powders. For oral preparations, the agents, polynucleotides, and polypeptides can be used alone or in combination with appropriate additives, for example, with conventional additives, such as lactose, mannitol, corn starch, or potato starch; with binders, such as crystalline cellulose, cellulose derivatives, acacia, corn starch, or gelatins; with disintegrators, such as corn starch, potato starch, or sodium carboxymethylcellulose; with lubricants, such as talc or magnesium stearate; and if desired, with diluents, buffering agents, moistening agents, preservatives, and flavoring agents.

The *ERG* isoform (*e.g., ERG8, EPC1, EPC2,* a transcript from the prostate cancer-specific promoter, ERG1, ERG2, or ERG3) nucleic acids, polypeptides, compounds used for destabilization, small molecule inhibitors, and antibody compositions can be formulated into preparations for injection by dissolving, suspending, or emulsifying them in an aqueous or nonaqueous solvent, such as vegetable or other similar oils, synthetic aliphatic acid glycerides, esters of higher aliphatic acids or propylene glycol; and if desired, with conventional additives such as solubilizers, isotonic agents, suspending agents, emulsifying agents, stabilizers and preservatives. Other formulations for oral or parenteral delivery can also be used, as conventional in the art.

The *ERG* isoform (*e.g., ERG8, EPC1, EPC2,* a transcript from the prostate cancer-specific promoter, ERG1, ERG2, or ERG3) nucleic acids, polypeptides, compounds used for destabilization, small molecule inhibitors, and antibody compositions can also be introduced into tissues or host cells by other routes, such as viral infection, microinjection, or vesicle fusion. For example, expression vectors can be used to introduce nucleic acid compositions into a cell as described herein. Further, jet injection can be used for intramuscular administration (Furth et al., ANAL BIOCHEM 205:365-368 (1992)). The DNA can be coated onto gold microparticles, and delivered intradermally by a particle bombardment device, or "gene gun" as described in the literature (Tang et al., NATURE 356:152-154 (1992)), where gold microprojectiles are coated with the DNA, then bombarded into skin cells.

In some embodiments, nucleic acids comprising a sequence encoding an ERG isoform (*e.g., ERG8, EPC1, EPC2,* a transcript from the prostate cancer-specific promoter, ERG1, ERG2, or ERG3) protein or functional derivative thereof, are administered to promote ERG function, by way of gene therapy. Alternatively, nucleic acids comprising an siRNA, shRNA, or antisense of *ERG8, EPC1, EPC2,* a transcript from the prostate cancer-specific promoter, ERG1, ERG2, or ERG3 sequence are administered to antagonize *ERG* expression or function. Any of the uses for gene therapy available in the art can be used. For specific protocols, see Morgan, GENE THERAPY PROTOCOLS, 2nd ed., Humana Press (2001). For general reviews of the uses of gene therapy, see Goldspiel et al., CLIN PHARMACY 12:488-505 (1993); Wu et al., BIOTHERAPY 3:87-95 (1991); Tolstoshev, ANN REV PHARMACOL TOXICOL 32:573-596 (1993); Mulligan, SCIENCE 260:926-932 (1993); Morgan et al., ANN REV BIOCHEM 62:191-217 (1993); and May, TIBTECH 11(5):155-215 (1993). Methods commonly known in the art of recombinant DNA technology which can be used are described in Ausebel et al., eds., CURRENT PROTOCOLS IN MOLEC BIOL, John Wiley & Sons, NY (2004); and Kriegler GENE TRANSFER AND EXPRESSION, A LABORATORY MANUAL, Stockton Press, NY (1990).

In some embodiments, the therapeutic comprises an *ERG* isoform, such as *ERG8, EPC1, EPC2,* a transcript from the prostate cancer-specific promoter, *ERG*1, *ERG*2, or *ERG*3, or an antisense of one or more of these *ERG* isoforms. The nucleic acid is part of a vector that has a regulatory sequence, such as a promoter, operably linked to the *ERG* isoform coding region or antisense molecule, said promoter being inducible or constitutive, and, optionally, tissue-specific. In another embodiment, a nucleic acid molecule is used in which an *ERG* isoform (*e.g., ERG8, EPC1, EPC2,* a transcript from the prostate cancer-specific promoter, *ERG*1, *ERG*2, or *ERG*3) coding sequence and any other desired sequences are flanked by regions that promote homologous recombination at a desired site in the genome, thus providing for intrachromosomal expression of the *ERG* isoform (Koller et al., PROC NATL ACAD SCI USA 86:8932-8935 (1989); Zijlstra et al., NATURE 342:435-438 (1989)).

In some embodiments, the nucleic acid to be introduced for purposes of gene therapy comprises an inducible promoter operably linked to the desired nucleic acids, such that expression of the nucleic acid is controllable by the appropriate inducer of transcription.

Delivery of the nucleic acid into a patient may be either direct, in which case the patient is directly exposed to the nucleic acid or nucleic acid-carrying vector, or indirect, in which case, cells are first transformed with the nucleic acid in vitro, then transplanted into the patient. These two approaches are known, respectively, as *in vivo* or *ex vivo* gene therapy.

In a specific embodiment, the nucleic acid is directly administered *in vivo,* where it is expressed to produce the encoded product. This can be accomplished by any of numerous uses known in the art, *e.g*., by constructing it as part of an appropriate nucleic acid expression vector and administering it so that it becomes intracellular, *e.g.,* by infection using a defective or attenuated retroviral or other viral vector (see U.S. Patent No. 4,980,286), or by direct injection of naked DNA, or by use of microparticle bombardment (*e.g.,* a gene gun; Biolistic, DuPont), or coating with lipids or cell-surface receptors or transfecting agents, encapsulation in liposomes, microparticles, or microcapsules, or by administering it in linkage to a peptide which is known to enter the nucleus, by administering it in linkage to a ligand subject to receptor-mediated endocytosis (see, *e.g.,* Wu et al., J BIOL CHEM 262:4429-4432 (1987)). In another embodiment, a nucleic acid-ligand complex can be formed in which the ligand comprises a fusogenic viral peptide to disrupt endosomes, allowing the nucleic acid to avoid lysosomal degradation. In yet another embodiment, the nucleic acid can be targeted *in vivo* for cell-specific uptake and expression, by targeting a specific receptor (see, *e.g.,* PCT Pubs. WO 92/06180; WO 92/22635; WO92/20316; WO93/14188; WO 93/20221). Alternatively, the nucleic acid can be introduced intracellularly and incorporated within host cell DNA for expression, by homologous recombination (Koller et al., PROC NATL ACAD SCI USA 86:8932-8935 (1989); Zijlstra et al., NATURE 342:435-438 (1989)).

In some embodiments, a viral vector that contains an *ERG* isoform (*e.g., ERG8, EPC1, EPC2,* a transcript from the prostate cancer-specific promoter, *ERG*1, *ERG*2, or *ERG*3) nucleic acid or antisense nucleic acid is used. For example, a retroviral vector can be used. (Miller et al., METH ENZYMOL 217:581-599 (1993)). These retroviral vectors have been modified to delete retroviral sequences that are not necessary for packaging of the viral genome and integration into host cell DNA. The *ERG* isoform (*e.g., ERG8, EPC1, EPC2,* a transcript from the prostate cancer-specific promoter, *ERG*1, *ERG*2, or *ERG*3) nucleic acid to be used in gene therapy is cloned into the vector, which facilitates delivery of the gene into a patient. More detail about retroviral vectors can be found in Boesen et al., BIOTHERAPY 6:291-302 (1994), which describes the use of a retroviral vector to deliver the MDRL gene to hematopoietic stem cells in order to make the stem cells more resistant to chemotherapy. Other references illustrating the use of retroviral vectors in gene therapy are: Clowes et al., J CLIN INVEST 93:644-651 (1994); Kiem et al., BLOOD 83:1467-1473 (1994); Salmons et al., HUM GENE THER 4:129-141 (1993); and Grossman et al., CURR OPIN GEN DEVEL 3:110-114 (1993).

Other viral vectors that can be used in gene therapy include adenoviruses, which are capable of infecting non-dividing cells. Kozarsky et al., CURR OPIN GEN DEVEL 3:499-503 (1993) present a review of adenovirus-based gene therapy. Bout et al., HUM GENE THER 5:3-10 (1994) demonstrated the use of adenovirus vectors to transfer genes to the respiratory epithelia of rhesus monkeys. Other instances of the use of adenoviruses in gene therapy can be found in Rosenfeld et al., SCIENCE 252:431-434 (1991); Rosenfeld et al., CELL 68:143-155 (1992); and Mastrangeli et al., J CLIN INVEST 91:225-234 (1993). Adeno-associated virus (AAV) has also been proposed for use in gene therapy (Walsh et al., PROC Soc EXP BIOL MED 204:289-300 (1993)).

Another approach to gene therapy involves transferring a gene to cells in tissue culture by such methods as electroporation, lipofection, calcium phosphate mediated transfection, or viral infection. Usually, the method of transfer includes the transfer of a selectable marker to the cells. The cells are then placed under selection to isolate those cells that have taken up and are expressing the transferred gene. Those cells are then delivered to a patient. In this embodiment, the nucleic acid is introduced into a cell prior to administration *in vivo* of the resulting recombinant cell. Such introduction can be carried out by any method known in the art, including but not limited to transfection, electroporation, microinjection, infection with a viral or bacteriophage vector containing the nucleic acid sequences, cell fusion, chromosome-mediated gene transfer, microcell-mediated gene transfer, spheroplast fusion, etc. Numerous techniques are known in the art for the introduction of foreign genes into cells (see, *e.g*., Loeffler et al., METH ENZYMOL 217:599-618 (1993); Cohen et al., METH ENZYMOL 217:618-644 (1993); Cline, PHARMAC THER 29:69-92 (1985)) and may be used in accordance with the present invention, provided that the necessary developmental and physiological functions of the recipient cells are not disrupted. The technique should provide for the stable transfer of the nucleic acid to the cell, so that the nucleic acid is expressible by the cell and preferably heritable and expressible by its cell progeny. The resulting recombinant cells can be delivered to a patient by various uses known in the art.

The prostate cancer-specific transcripts encode protein products that are thought to either directly or indirectly contribute to the development of the cancerous cell. Accordingly, applications that destabilize these transcripts can be used to reduce or prevent expression of the encoded protein product, thereby preserving the cell in a non-cancerous state, or reverting the cell to a non-cancerous phenotype. In some embodiments, therefore, nucleic acids corresponding to *ERG8, EPC1, EPC2, ERG1, ERG2, ERG3,* or isoforms encoded by transcripts initiated from a prostate cancer-specific promoter *(e.g.,* SEQ ID NO: 7), or a fragment thereof (such as a fragment comprising at least nucleotides 521 to 650 of SEQ ID NO: 7), are used to interfere with the production or translation of their corresponding transcript. In some cases, the nucleic acid is the complement of the transcript sequence. In these cases, the nucleic acids are therapeutic because they modulate the function of nucleic acids encoding an *ERG* isoform, such as *ERG8, EPC1, EPC2, ERG1, ERG2, ERG3,* or isoforms encoded by transcripts initiated from a prostate cancer-specific promoter, and thereby alter expression of the encoded isoform.

One use of modulating the function of one or more *ERG* isoforms is via RNA interference, for example, using siRNA or shRNA against the *ERG* isoform. The siRNA is a short double stranded RNA molecule of about 18-25 nucleotides that comprises a nucleotide sequence complementary to a region of the target. It can be introduced into a target cell or tissue, for example using an expression plasmid, where it interferes with the translation of an *ERG* isoform, such as *ERG8, EPC1, EPC2, ERG1, ERG2, ERG3,* or isoforms encoded by transcripts initiated from a prostate cancer-specific promoter, such as SEQ ID NO: 7 (or a fragment thereof). RNA interference techniques can be carried out using known methods as described, for example, in published U.S. Patent Applications 20060058255, 20040192626, 20040181821, and 20030148519.

Antisense compounds are another class of nucleic acid that is provided by the disclosure for use in modulating the function of nucleic acid molecules encoding one or more *ERG* isoforms, thereby modulating the amount of the *ERG* isoform(s) that is produced. This is accomplished by providing antisense compounds that hybridize with one or more nucleic acids encoding an *ERG* isoform to a cell, for example, by using a gene therapy technique. The nucleic acid can be DNA encoding an *ERG* isoform (*e.g*., *ERG8, EPC1, EPC2, ERG1, ERG2, ERG3,* or isoforms encoded by transcripts initiated from a prostate cancer-specific promoter, such as SEQ ID NO: 7), RNA (including pre-mRNA and mRNA) transcribed from such DNA, and can also be cDNA derived from such RNA. The hybridization of an antisense compound with its target nucleic acid interferes with the normal function of the nucleic acid. The interference can act at the level of replication or transcription of the DNA, translocation of the RNA to the site of protein translation, translation of protein from the RNA, splicing of the RNA to yield one or more mRNA species, or catalytic activity that may be engaged in or facilitated by the RNA. The overall effect of such interference with target nucleic acid function is the modulation of the expression of an *ERG* isoform, such as *ERG8, EPC1, EPC2, ERG1, ERG2, ERG3,* or isoforms encoded by transcripts initiated from a prostate cancer-specific promoter, such as SEQ ID NO: 7 (or a fragment thereof, such as a fragment comprising at least nucleotides 521 to 650 of SEQ ID NO: 7).

Antisense oligonucleotides are one form of antisense compound. These often comprise from about 8 to about 30 nucleobases (*i.e*. from about 8 to about 30 linked nucleosides). In some embodiments, the antisense oligonucleotide comprises from about 12 to about 25, from about 15 to about 22, or from about 18 to about 20 nucleobases. Antisense oligonucleotides can also comprise modified backbones or non-natural internucleoside linkages. Modified oligonucleotides that do not have a phosphorus atom in their internucleoside backbone are also considered oligonucleotides. Examples of modified oligonucleotide backbones include, for example, phosphorothioates, chiral phosphorothioates, phosphorodithioates, phosphotriesters, aminoalkylphosphotriesters, methyl and other alkyl phosphonates including 3'-alkylene phosphonates and chiral phosphonzites, phosphinates, phosphoramidates including 3'-amino phosplioramidate and aminoalkylphosphoramidates, thionophosphoiamidates, thionoalkylphosphonates, thionoalkylphosphotriesters, boranophosphates having normal 3'-5' linkages, 2'-5' linked analogs of these, and those having inverted polarity wherein the adjacent pairs of nucleoside units are linked 3'-5' to 5'-3' or 2'-5' to 5'-2', and backbones formed by morpholino linkages.

Peptide nucleic acid (PNA) compounds are also antisense compounds. In a PNA compound, however, the sugar-backbone of an oligonucleotide is replaced with an aminoethylglycine backbone. The nucleobases are retained and are bound directly or indirectly to aza nitrogen atoms of the amide portion of the backbone.

Antisense compounds, methods for their production, and their use to interfere with nucleic acid function are well known in the art. For example, U.S. Patent No. 6,054,316, describes the production of antisense compounds for nucleic acids encoding Ets-2 and methods of using these antisense compounds. These same methods can be applied to the production of antisense compounds for nucleic acids encoding an *ERG* isoform, such as *ERG8, EPC1, EPC2, ERG1, ERG2, ERG3,* or isoforms encoded by transcripts initiated from a prostate cancer-specific promoter, such as SEQ ID NO: 7 (or a fragment thereof, such as a fragment comprising at least nucleotides 521 to 650 of SEQ ID NO: 7).

In addition to therapeutic applications related to inhibition of expression of *ERG* isoforms *(e.g., ERG8, EPC1, EPC2, ERG1, ERG2, ERG3,* or isoforms encoded by transcripts initiated from a prostate cancer-specific promoter, such as SEQ ID NO: 7), antisense compounds are also useful in diagnostic and prognostic methods because these compounds hybridize to nucleic acids encoding *ERG* isoforms, which can be detected using art-recognized techniques, such as conjugation of an enzyme to the antisense compound, radiolabelling of the antisense compound, or any other suitable detection methods. Kits comprising the antisense compound and a means for detecting it in a sample can also be prepared as described for kits comprising oligonucleotide probes generally.

Antisense modulation of *ERG* isoform expression can be assayed in a variety of ways known in the art. For example, mRNA levels can be quantitated by, *e.g.,* northern blot analysis, competitive polymerase chain reaction (PCR), or real-time PCR (RT-PCR). RNA analysis can be performed on total cellular RNA or poly(A)+ mRNA. Alternatively or in addition, levels of the encoded protein can be quantitated in a variety of ways well known in the art, such as immunoprecipitation, western blot analysis (immunoblotting), ELISA, or fluorescence-activated cell sorting (FACS).

It is also possible to kill or slow the growth of prostate cancer cells by delivering to those cells a cytotoxic or cytostatic gene product expressed under the control of a prostate cancer-specific promoter, such as the promoter sequence set forth in SEQ ID NO: 7. Truncations and variation of the nucleotide sequence set forth in SEQ ID NO: 7 can also be used, so long as they are sufficient to support expression of an operatively linked reporter gene in prostate cancer cells. Examples include promoter sequences comprising at least nucleotides 521 to 650, 404 to 650, or 138 to 650 of SEQ ID NO: 7. Gene therapy can be used to introduce a vector comprising the prostate cancer-specific promoter operably linked to a nucleic acid encoding the cytotoxic or cytostatic protein into a prostate cancer cell. Such gene therapy uses are described herein. When the prostate cancer-specific promoter is used in the gene therapy vector, however, the promoter is only active in the prostate cancer cells so that the cytotoxic or cytostatic protein is only expressed in the prostate cancer cells, irrespective of the cellular range of the gene therapy vector.

There are many different cytotoxic or cytostatic proteins that can be expressed by placing a heterologous gene under the control of a prostate cancer-specific promoter. Examples of such genes include genes encoding bacterial toxins, such as diphtheria toxin, pseudomonas toxin, ricin, cholera toxin, and PE40; tumor suppressor genes, such as APC, CYLD, HIN-1, KRAS2b, p16, p19, p21, p27, p27mt, p53, p57, p73, PTEN, Rb, Uteroglobin, Skp2, BRCA-1, BRCA-2, CHK2, CDKN2A, DCC, DPC4, MADR2/JV18, MEN1, MEN2, MTS1, NF1, NF2, VHL, WRN, WT1, CFTR, C-CAM, CTS-1, zac1, scFV, MMAC1, FCC, MCC, Gene 26 (CACNA2D2), PL6, Beta* (BLU), Luca-1 (HYAL1), Luca-2 (HYAL2), 123F2 (RASSF1), 101F6, and Gene 21 (NPRL2); genes encoding apoptosis-inducing proteins, such as CD95, caspase-3, Bax, Bag-1, CRADD, TSSC3, bax, hid, Bak, MKP-7, PERP, bad, bcl-2, MST1, bbc3, Sax, BIK, BID, and mda7; and genes encoding drug metabolizing enzymes that convert a pro-drug into a cytotoxic product, such as thymidine kinase (from herpes simplex or varicella zoster viruses), cytosine deaminase, nitroreductase, cytochrome p-450 2B1, thymidine phosphorylase, purine nucleoside phosphorylase, alkaline phosphatase, carboxypeptidases A and G2, linamarase, β.-lactamase and xanthine oxidase.

Accordingly, the disclosure provides a use of treating prostate cancer comprising administering to a subject in need thereof an expression vector comprising a polynucleotide encoding a cytotoxic or cytostatic gene product operably linked to a promoter sequence comprising SEQ ID NO: 7 or a fragment of the nucleotide sequence set forth in SEQ ID NO: 7 that is sufficient to support expression of an operatively linked reporter gene in prostate cancer cells, including, for example, a sequence comprising at least nucleotides 521 to 650 of SEQ ID NO: 7. In another embodiment, the disclosure provides a use of reducing the growth of a prostate cancer cell comprising administering to the cell an expression vector comprising a polynucleotide encoding a cytotoxic or cytostatic gene product operably linked to a promoter sequence comprising SEQ ID NO: 7 or a fragment of the nucleotide sequence set forth in SEQ ID NO: 7 that is sufficient to support expression of an operatively linked reporter gene in prostate cancer cells, including, for example, a sequence comprising at least nucleotides 521 to 650 of SEQ ID NO: 7. In either embodiment, the cytotoxic or cytostatic gene product is chosen from bacterial toxins, tumor suppressor gene products, apoptosis-inducing proteins, and drug metabolizing enzymes that convert a pro-drug into a cytotoxic product.

Another way to kill a prostate cancer cell or to inhibit or slow its growth is by modulating the activity of proteins within the cell. For example, an antibody that binds a protein encoded by an *ERG* isoform can be used to inhibit or stimulate the function of that protein. In some embodiments, the antibody binds an epitope that is present in proteins encoded by more than one *ERG* isoforms. Other embodiments involve an antibody that binds the protein encoded by a particular *ERG* isoform, such as *ERG8, EPC1, EPC2, ERG1, ERG2, ERG3,* or an isoform encoded by a transcript initiated from a prostate cancer-specific promoter, such as SEQ ID NO: 7 (or a fragment thereof, such as a fragment comprising at least nucleotides 521 to 650 of SEQ ID NO: 7). Thus, in one embodiment the disclosure provides an antibody that binds an epitope comprising amino acid residues 217 to 220 of SEQ ID NO: 4. In another embodiment, the antibody binds an epitope within or comprising amino acids 28 to 97 of SEQ ID NO: 6. The antibody or combination of antibodies can be expressed intracellularly using gene therapy, as described herein. In another example, the antibody binds an epitope within or comprising amino acid residues 28 to 97 of SEQ ID NO: 6, and it also binds the protein consisting of SEQ ID NO: 6.

These various antibodies can be produced using techniques known in the art. For example, the protein(s) encoded by one or more *ERG* isoform (*e.g., ERG8, EPC1, EPC2, ERG1, ERG2, ERG3,* or an isoform encoded by a transcript initiated from a prostate cancer-specific promoter, such as SEQ ID NO: 7) can be used as an immunogen and then one or more antibodies with the desired specificity and functional properties can be selected. Such antibodies include, but are not limited to, polyclonal antibodies, monoclonal antibodies, chimeric antibodies, single chain antibodies, and antibody fragments. The antibodies may be from mice, rats, rabbits, hamsters, goats, Ilamas, humans, or other species.

Various procedures known in the art can be used for the production of polyclonal antibodies to one or more epitopes of a secreted protein. Rabbits, mice, rats, goats, Ilamas, etc. can be immunized with the native protein, a synthetic version of the protein, or a derivative (*e.g*., fragment) of the protein. Various adjuvants may be used to increase the immunological response, depending on the host species. Examples of adjuvants include, but are not limited to, Freund's (complete and incomplete), mineral gels such as aluminum hydroxide, surface active substances such as lysolecithin, pluronic polyols, polyanions, peptides, oil emulsions, keyhole limpet hemocyanins, dinitrophenol, and potentially useful human adjuvants such as BCG (bacille Calmette-Guerin) and corynebacterium parvum.

For the preparation of monoclonal antibodies, any of a number of art-recognized techniques can be utilized. For example, monoclonal antibodies can be produced using the hybridoma technique (*e.g*., Kohler et al., NATURE 256:495-97 (1975); and as described in Harlow et al., eds., ANTIBODIES: A LABORATORY MANUAL, Cold Spring Harbor Laboratory, (1988); Colligan et al., eds., CURRENT PROTOCOLS IN IMMUNOLOGY, Chpt. 2, John Wiley & Sons, Inc. (2006)). Antibodies can also be produced using recombinant DNA methods (*e.g*., U.S. Patent 4,816,567) or using phage display antibody libraries (*e.g*., Clackson et al., NATURE 352: 624-28 (1991); Marks et al., J MOL BIOL 222: 581-97 (1991)). If desired, chimeric antibodies can be produced using methods known in the art (*e.g*., Morrison et al., PROC NATL ACAD SCI USA 81:6851-55 (1994); Neuberger et al., NATURE 312:604-08 (1984); Takeda et al., NATURE 314:452-54 (1985)). Single chain antibodies can also be produced (*e.g*., U.S. Patent No. 4,946,778). Human antibodies can be prepared using human hybridomas (Cote et al., PROC NATL ACAD SCI USA 80:2026-30 (1983)), by transforming human B cells with EBV virus in vitro (Cole et al., MONOCLONAL ANTIBODIES AND CANCER THERAPY, Alan R. Liss, pp. 77-96 (1985)), or by preparing hybridomas from animals transgenic for one or more human immunoglobulin genes (*e.g*., U.S. Patent No. 5,939,598). A monoclonal antibody can be readily expressed using its encoding DNA sequence(s), and methods for such expression, including gene therapy uses, are well known in the art.

Antibody fragments can also be generated using known techniques. Fragments include but are not limited to F(ab')₂ fragments, which can be produced by pepsin digestion of the antibody molecule; Fab' fragments, which can be generated by reducing the disulfide bridges of the F(ab')₂ fragment; Fab fragments, which can be generated by treating the antibody molecule with papain and a reducing agent; and Fv fragments, including single chain Fv (scFv) fragments.

Following the production of antibodies by, for example, hybridoma technology, screening for the desired antibody can be accomplished by techniques known in the art, *e.g.,* ELISA, and involve no more than routine techniques (*e.g.,* Harlow et al.,eds., ANTIBODIES: A LABORATORY MANUAL, Cold Spring Harbor Laboratory, (1988); Colligan et al., eds., CURRENT PROTOCOLS IN IMMUNOLOGY, Chpt. 2, John Wiley & Sons, Inc., 2006). Thus, an antibody can be selected that binds a linear epitope or a conformational epitope. An antibody also can be selected for the property of binding both to a polypeptide fragment of a larger protein, and to the intact (*e.g.,* full length or wild-type) protein.

When it is necessary to produce an antibody to a protein encoded by an *ERG* isoform (*e.g., ERG8, EPC1, EPC2, ERG1, ERG2, ERG3,* or an isoform encoded by a transcript initiated from a prostate cancer-specific promoter, such as SEQ ID NO: 7), the protein, its fragment, or other derivative, can be produced using standard techniques. Methods of manipulating nucleic acids to express proteins are well known in the art, and include those described in MOLEC CLONING, A LABORATORY MANUAL (2nd Ed., Sambrook, Fritsch and Maniatis, Cold Spring Harbor) and CURRENT PROTOCOLS IN MOLEC BIOL (eds. Ausubel, Brent, Kingston, More, Feidman, Smith and Stuhl, Greene Publ. Assoc., Wiley-Interscience, N.Y., NY, (1992)).

Generally, in order to express the protein encoded by an *ERG* isoform *(e.g., ERG8, EPC1, EPC2, ERG1, ERG2, ERG3,* or an isoform encoded by a transcript initiated from a prostate cancer-specific promoter, such as SEQ ID NO: 7), a suitable cell line is transformed with a DNA sequence encoding that protein under the control of known regulatory sequences. The transformed host cells are cultured and the protein recovered and isolated from the culture medium. The isolated expressed proteins are substantially free from other proteins with which they are co-produced as well as from other contaminants. Suitable cells or cell lines may be mammalian cells, such as Chinese hamster ovary cells (CHO), the monkey kidney COS-1 cell line, or mammalian CV-1 cells. The selection of suitable mammalian host cells and methods for transformation, culturing, amplification, screening, product production and purification are known in the art. (*See*, *e.g.,* Gething and Sambrook, NATURE 293:620-625 (1981); Kaufman et al., MOL CELL BIOL 5(7):1750-1759 (1985); Howley et al., U.S. Patent 4,419,446.))

Bacterial cells may also be used as suitable hosts for expression of the secreted proteins. For example, the various strains of *E*. *coli* (*e.g.,* HB101, MC1061) are well-known as host cells in the field of biotechnology. Various strains of *B. subtilis, Pseudomonas,* other bacilli and the like may also be used. For expression of a protein in bacterial cells, DNA encoding the propeptide is generally not necessary.

Many strains of yeast cells known to those skilled in the art may also be available as host cells for expression of the secreted protein biomarkers. Additionally, where desired, insect cells may be utilized as host cells in the method of the present invention. *See, e.g.,* Miller et al., GENETIC ENGINEERING 8:277-298, Plenum Press (1986).

In some embodiments, the protein encoded by an *ERG* isoform (*e.g., ERG8*, *EPC1, EPC2, ERG1, ERG2, ERG3,* or an isoform encoded by a transcript initiated from a prostate cancer-specific promoter, such as SEQ ID NO: 7) is expressed using a vector that contains a full length DNA sequence encoding the protein and appropriate expression control sequences. Expression control sequences for such vectors are known to those skilled in the art and may be selected depending upon the host cells. Such selection is routine. In other embodiments, the secreted protein biomarker is expressed as a fusion protein comprising the protein sequence of the biomarker and, for example, a tag to stabilize the resulting fusion protein or to simplify purification of the secreted protein biomarker. Such tags are known in the art. Representative examples include sequences which encode a series of histidine residues, the epitope tag FLAG, the Herpes simplex glycoprotein D, beta-galactosidase, maltose binding protein, streptavidin tag or glutathione S-transferase.

In some embodiments, therefore, it is desirable that protein expression of *ERG8, EPC1, EPC2, ERG1, ERG2, ERG3,* or an isoform encoded by a transcript initiated from a prostate cancer-specific promoter, such as SEQ ID NO: 7, is entirely by an *in vitro* method. Of course, as already discussed, in other embodiments it is desirable that the protein expression occurs *in vivo.*

Additional objects and advantages of the invention will be set forth in part in the description which follows, and in part will be obvious from the description, or may be learned by practice of the invention. The objects and advantages of the invention will be realized and attained by means of the elements and combinations particularly pointed out in the appended claims. Moreover, advantages described in the body of the specification, if not included in the claims, are not per se limitations to the claimed invention.

It is to be understood that both the foregoing general description and the following detailed description are exemplary and explanatory only and are not restrictive of the invention, as claimed. Moreover, it must be understood that the invention is not limited to the particular embodiments described, as such may, of course, vary. Further, the terminology used to describe particular embodiments is not intended to be limiting, since the scope of the present invention will be limited only by its claims. The claims do not encompass embodiments in the public domain.

With respect to ranges of values, the invention encompasses each intervening value between the upper and lower limits of the range to at least a tenth of the lower limit's unit, unless the context clearly indicates otherwise. Further, the invention encompasses any other stated intervening values. Moreover, the invention also encompasses ranges excluding either or both of the upper and lower limits of the range, unless specifically excluded from the stated range.

Unless defined otherwise, the meanings of all technical and scientific terms used herein are those commonly understood by one of ordinary skill in the art to which this invention belongs. One of ordinary skill in the art will also appreciate that any methods and materials equivalent to those described herein can also be used to practice or test the invention.

It must be noted that, as used herein and in the appended claims, the singular forms "a," "or," and "the" include plural referents unless the context clearly dictates otherwise. Thus, for example, reference to "a subject polypeptide" includes a plurality of such polypeptides and reference to "the agent" includes reference to one or more agents and equivalents thereof known to those skilled in the art, and so forth.

Further, all numbers expressing quantities of ingredients, reaction conditions, % purity, polypeptide and polynucleotide lengths, and so forth, used in the specification and claims, are modified by the term "about," unless otherwise indicated. Accordingly, the numerical parameters set forth in the specification and claims are approximations that may vary depending upon the desired properties of the present invention. At the very least, and not as an attempt to limit the application of the doctrine of equivalents to the scope of the claims, each numerical parameter should at least be construed in light of the number of reported significant digits, applying ordinary rounding techniques. Nonetheless, the numerical values set forth in the specific examples are reported as precisely as possible. Any numerical value, however, inherently contains certain errors from the standard deviation of its experimental measurement.

The specification is most thoroughly understood in light of the references cited herein..

### ERG Isoform Identification and Expression in Prostate Cancer Tissue and Cell Lines

### Example 1: Identification of ERG8

*ERG1* is the most commonly overexpressed proto-oncogene in malignant prostatic tissue. (Petrovics et al., ONCOGENE 24: 3847-52 (2005).) This overexpression may be due to the fusion of the *TMPRSS2* gene with the *ERG* gene. (Tomlins et al., SCIENCE 310:644-48 (2005).) Alternative splicing generates multiple *ERG* isoforms. (Owczarek et al., GENE 324:65-77 (2004).) Thus, it is possible that other isoforms of *ERG* are also overexpressed, or are selectively expressed, in prostate cancer.

In an initial experiment, we sought to detect the *ERG8* isoform in cDNA derived from laser microdissected (LCM) prostate tumor cells. The cDNA was amplified using a primer pair from the genomic sequence of exon 1 of the *TMPRSS2* gene (primer p2178: 5'-TAGGCGCGAGCTAAGCAGGAG-3' - SEQ ID NO: 8) and from the *ERG* coding sequence (primer p2220: 5'-CCAGGATGCCTTCTTTGCCCATC-3'- SEQ ID NO: 9). The TMPRSS2 gene is often fused to the *ERG* gene in prostate cancer. The p2718 primer corresponds to nucleotides 1 to 21 of SEQ ID NO: 1, while p2220 corresponds to the reverse complement of nucleotides 1042 to 1062 of SEQ ID NO: 1. This primer pair resulted in a PCR product and sequencing confirmed it was *ERG8.*

*ERG*8 cDNA can also be amplified using primer pairs directed to ERG8 specific regions of SEQ ID NO: 30 and SEQ ID NO: 46. For example, the primer pair
Mid-E8N-F: 5'-GGCATTTCCCAGAAGGGTAT-3' (SEQ ID NO: 35)
Mid-E8N-R: 5'-CATCAGCTGTGAGGCTGGTA-3' (SEQ ID NO: 36)
amplifies nucleotides 1744-1848 of SEQ ID NO: 30 and nucleotides 942-1046 of SEQ ID NO: 46. The primer pair
Down-E8N-F: 5'-AGTGACTTCAGGCTGGCAAT-3' (SEQ ID NO: 37)
Down-E8N-R: 5'-GCTAAGCCTTTCCATCATGC-3' (SEQ ID NO: 38)
amplifies nucleotides 2087-2176 of SEQ ID NO: 30 and nucleotides 1285-1374 of SEQ ID NO: 46. The primer pair
PoA-E8N-F: 5'-ACCCAGTGCCTAGAGTCTGC-3' (SEQ ID NO: 39)
PoA-E8N-R: 5'-AAGCTTGAGTCAAACATGAATTTCT-3' (SEQ ID NO: 40)
amplifies nucleotides 2253-2343 of SEQ ID NO: 30 and nucleotides 1451-1541 of SEQ ID NO: 46.

The novel full-length *ERG*8 cDNA was derived from total RNA isolated from the prostate tissue of a prostate cancer patient. *ERG* and TMPRSS2 positive cDNA clones were amplified from the CPDR human prostate cancer Lambda ZAP phage cDNA library. To amplify the clones, 12 µl of 2x Pful enzyme/buffer mixture (Stratagene, La Jolla, CA) was combined with 7 µl of HPLC-grade water, 2 µl (5 pmol) T3 primer, 2 µl (5 pmol) T7 primer (both primers from Lofstrand Labs, Bendigo, Australia), and 1 µl *ERG*-positive Lambda phage clones identified from the library. The reaction conditions were the following: 35 cycles at 94 °C for 1 min, 50 °C for 1 min, and 72 °C for 4 min. The thermal cycles were followed by a 7 min incubation at 72 °C and storage at 4 °C. The PCR product of this reaction was purified using Performa Spin Columns-33617 (Edge Biosystems, Gaithersburg, MD).

Nucleotide sequencing was performed by primer extension using the BigDye Sequencing method (Applied Biosystems, Foster City, CA). Four µl of BigDye Terminator, 2 µl of 5X Sequencing Buffer, 2 µl (5 pmol) of T7 sequencing primer, 2 µl of the PCR product (the template), and 10 µl of water were added into a total reaction volume of 20 µl. The mixture was incubated at 96 °C for 60 sec, followed by 25 cycles of 96 °C for 10 sec, 50 °C for 5 sec and 60 °C for 4 min. The DNA sequencing reaction mixture was then analyzed on an ABI 3700 Sequence Analyzer. The observed DNA sequences were compared to NCBI databases searching for matches to cDNA sequences and expressed sequence tags (EST). The analysis revealed a 100% match in the overlapping region of the identified clone with the NCBI AY204742 *ERG8* cDNA.

The sequence was confirmed with the following internal primers:
5'-GAGCGCCGCCTGGAGCGCGGCAG-3' (SEQ ID NO: 31)
5'-TTCAGAAAGACAGATGGGC-3' (SEQ ID NO: 32)
5'-CACGGATGGTATGATGGTG-3' (SEQ ID NO: 33)
The results of the sequencing demonstrated that the DNA sequences of the identified prostate cancer cDNA clones extend beyond the 3' end of the AY204742 *ERG*8 sequence. The primer
5'-AGCAATACAGGGCGTAGGAG-3' (SEQ ID NO: 34)
was designed to cover the 3' end of the cDNA sequences, reaching the Lambda ZAP polycloning sequences.

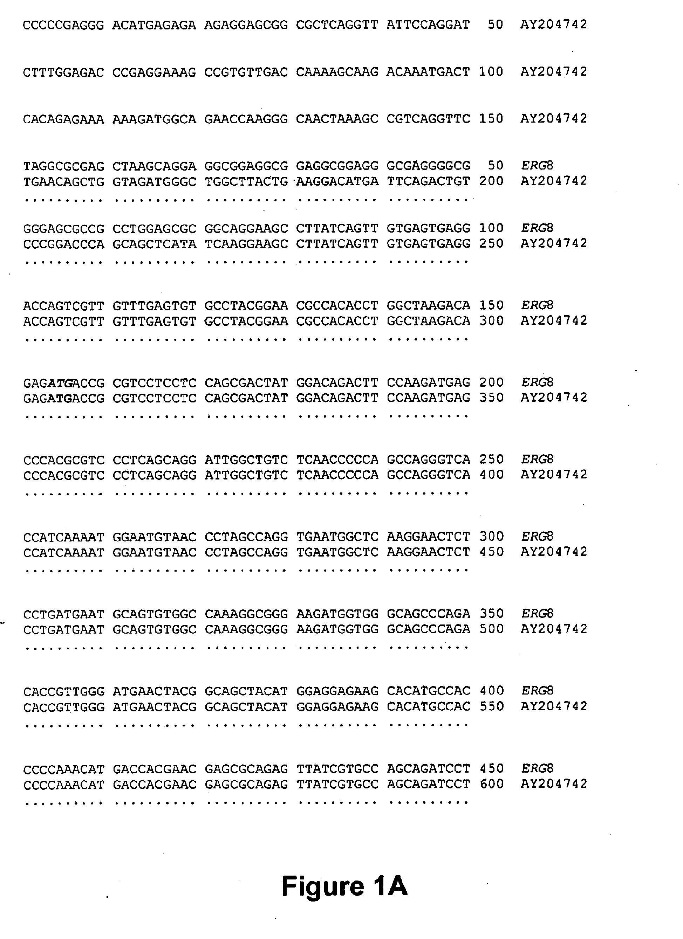

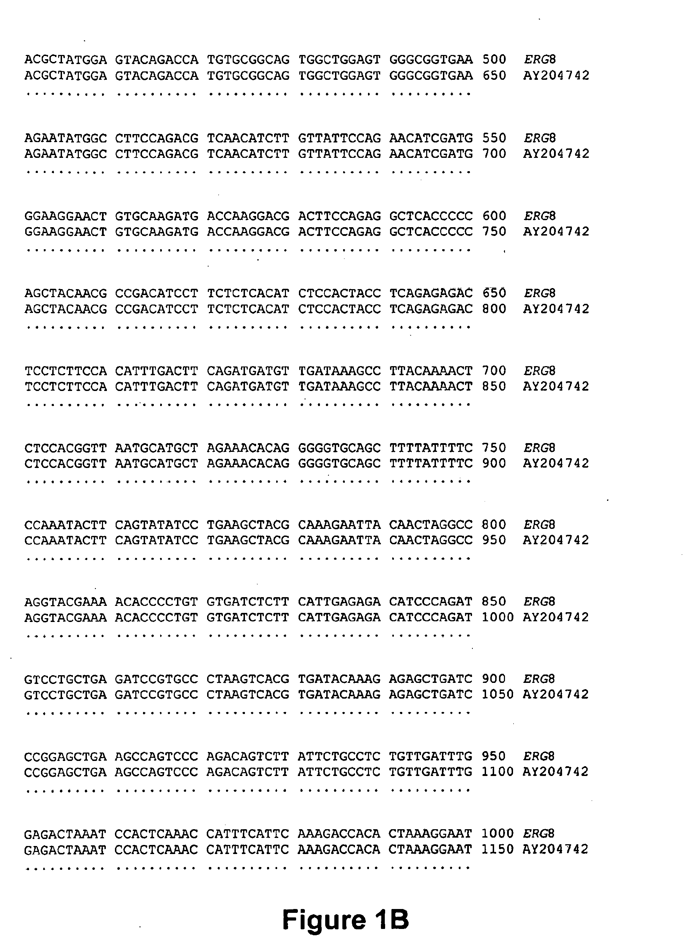

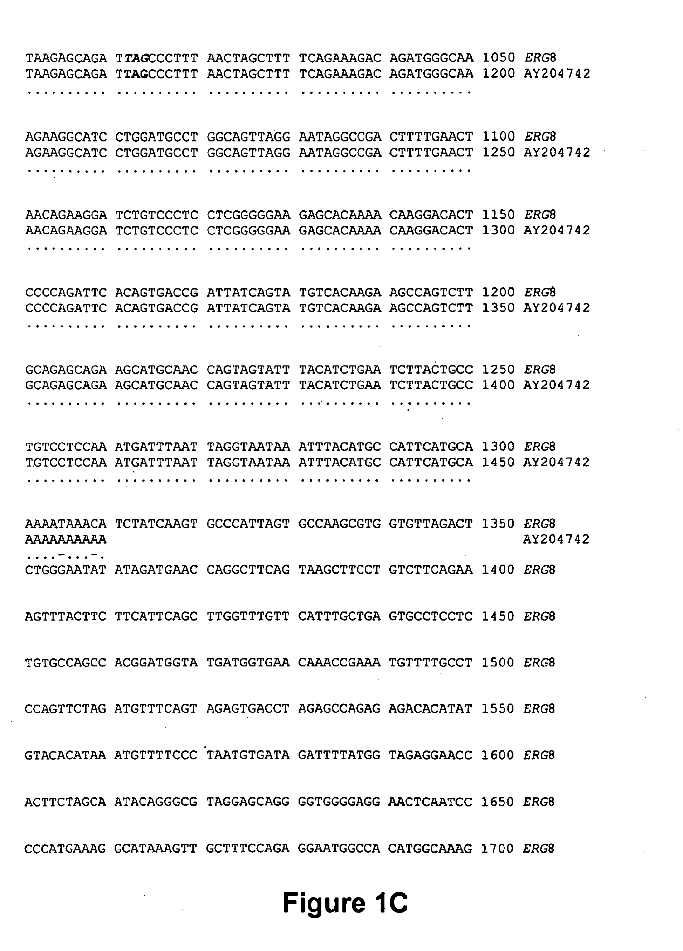

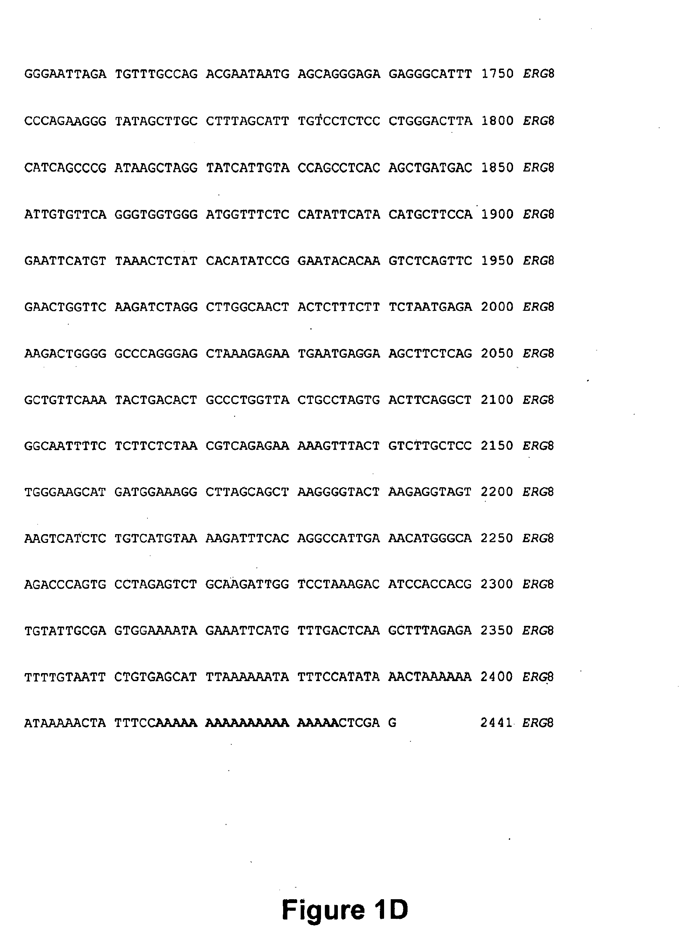

The entire *ERG*8 nucleotide sequence is shown as SEQ ID NO: 30 in Figure 1. It corresponds to nucleotides 150-1460 of NCBI Accession No. AY204742, with the exception that the adenosine residues identified at nucleotide positions 1455 and 1459 in AY204742, are identified herein as thymidine at position 1455 and cytosine at position 1459. The *ERG*8 sequence of SEQ ID NO: 30 comprises a 20-mer polyA tail at nucleotides 2416-2435 (Figure 1).

### Example 2: ERG8 is selectively expressed in prostate cancer tissue

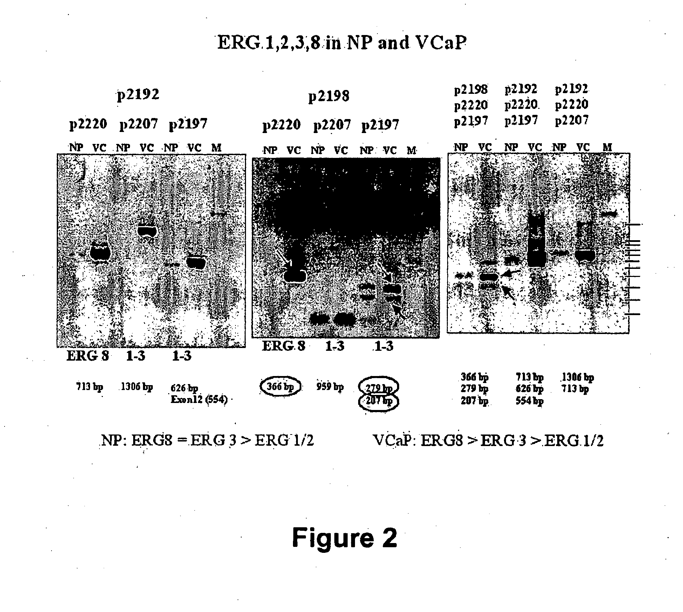

We then undertook a more thorough examination of the expression ratios of the *ERG1, ERG2, ERG3,* and *ERG8* isoforms in normal prostate cells and in the prostate cancer-derived cell line VCaP (American Type Culture Collection ("ATCC"), Rockville, MD). We isolated mRNA from normal prostate of 11 individuals and from prostate cancer-derived VCaP cells, respectively. After converting the mRNA to cDNA, we assessed *ERG* isoform ratios by comparing the intensities of isoform-specific PCR products in a semi-quantitative multiplex PCR approach. Figure 2 presents the results of the multiplex PCR analysis. The *ERG* primers used for the PCR were as follows: p2192 (exon 9): 5' - ACCGTTGGGATGAACTACGGCA - 3' (SEQ ID NO: 10, which corresponds to nucleotides 352 to 373 of SEQ ID NO: 1); p2220: (*ERG8* specific): 5'-CCAGGATGCCTTCTTTGCCCATC-3' (SEQ ID NO: 11, which corresponds to the reverse complement of nucleotides 1042 to 1064 of SEQ ID NO: 1); p2207: (exon 16): 5' -CCCTCCCAAGAGTCTTTGGATCTC - 3 (SEQ ID NO: 12); p2197: (exon 15): 5' - CCTGGATTTGCAAGGCGGCTACT - 3' (SEQ ID NO: 13); and p2198: (exon 11): 5'-CTCTCCACGGTTAATGCATGCTAG-3' (SEQ ID NO: 14, which corresponds to nucleotides 699 to 722 of SEQ ID NO: 1).

Primer pair p2192-p2220 results in a 713 bp PCR product when *ERG8* is present. The combination of primers p2192 and p2207 amplifies -1300 bp products representing *ERG* isoforms 1,2 and 3. When p2192 (Exon 9) is paired with primer p2197 (Exon 15), that primer combination amplifies one or more of *ERG* isoforms 1,2 and 3. Primer pair p2198-p2220 is also specific to the *ERG8* isoform, and this primer pair amplifies a 366 bp PCR product when *ERG8* is present. The combination of p2198 (Exon 11) and p2207 (Exon 16) results in a 959 bp product detecting *ERG* isoforms 1,2 and 3, while the p2198 - p2197 combination yields products of 279 bp (isoform 3) and 207 bp (isoforms 1 and 2).

In Figure 2, the normal prostate samples are labeled "NP", while samples using the VCaP cells are labeled "VC". *ERG8* is the predominant isoform detected in VCaP cells (Figure 2, right photograph, upper arrow). It is also present at higher levels than *ERG1* and *ERG2* in normal prostate, but its level in normal prostate is comparable to that of *ERG*3*.*

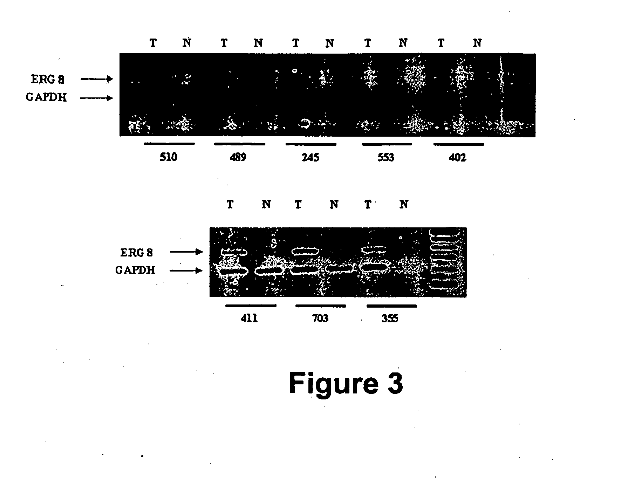

We have also assayed the expression of *ERG8* transcripts in RNA specimens extracted from laser microdissected (LCM) tumor and benign epithelial cells of 14 individual prostate cancer patients. Primers specifically recognizing the *ERG8* isoform (p2198-p2220) were used together with *GAPDH* primers as internal controls in the same RT-PCR reaction tubes. Figure 3 shows a photograph of a PCR gel with data for eight of the patients. Tumor cell samples are labeled "T", while the benign epithelial cell samples from each patient are labeled "N". *ERG8* expression was detected in the tumor cell samples of 11 of the 14 prostate cancer patients tested. We did not detect *ERG8* expression in the benign cells of any patient in this cohort. Thus, *ERG8* isoform detection indicates the presence of cells with a cancerous phenotype. In Figure 3, the level of *ERG8* is below the detection limit in the normal samples, which include only epithelial cells. The difference between *ERG8* detection in Figure 2 and Figure 3, therefore, can be explained by the presence of the other cell types (*e.g*., stroma and endothelial cells) included in the pooled prostate tissue used for the analysis in Figure 2.

Interestingly, the *ERG8* transcript (SEQ ID NO: 1, SEQ ID NO: 30, and SEQ ID NO: 46) is a fusion between *TMPRSS2* and *ERG8.* The open reading frame, however, is entirely encoded by the *ERG8* sequence (nucleotides 75 to 1168 of SEQ ID NO: 1 and SEQ ID NO: 30). The encoded protein (SEQ ID NO: 2), therefore, does not contain any amino acid sequences from *TMPRSS2.*

Cancer cells gain growth advantage by activating cell growth promoting genes and by silencing inhibitory genes of cell proliferation. Certain genes in these cell growth or proliferation pathways may produce alternative transcript that counteract the function of natural transcriptional products. In the case of *ERG8,* the encoded protein product lacks the DNA binding domain of, for example, *ERG*1 and *ERG2,* but it retains the entire protein-protein interaction domain. Overexpression of *ERG8* in the context of prostate cancer, therefore, likely results in the functional nullification of protein interaction partners of ERG1 and ERG2, resulting in a dominant negative effect. *ERG8* could also represent an oncogenic "gain of function" isoform.

The finding that *ERG8* is selectively expressed in prostate cancer cells provides a powerful therapeutic option, as this oncogenic *ERG8* product can be inhibited by selective targeting through its distinct 3' sequences. This selective targeting for cancer therapy can be accomplished using siRNA, shRNA, and other nucleic acid-based products capable of targeting the *ERG8-*specific sequence. At the protein level, antibodies and therapeutic agents such as small inhibitory peptides can be used to inhibit the activity of the protein produced by *ERG8* or to target that protein for degradation. Moreover, *ERG8* can differentiate tumor cells from normal epithelial cells in prostate specimens. Accordingly, detection of *ERG*8 using, for example, amplification primers or hybridization-based methods, can also be used to diagnose and prognose prostate cancer.

### Example 3: EPC1 and EPC2 are newly identified transcripts that are selectively expressed in prostate cancer tissue

To Whom It May Concern: identify tumor specific *ERG* transcripts, we pooled prostate tumor tissue samples from six patients and extracted total RNA. Polyadenylated RNA (mRNA) was then isolated, converted into cDNA, and packaged into the Lambda Zap express system (Stratagene) to obtain a bacteriophage library. We screened phage plaques by hybridization of radioactively labeled *ERG*2 probes. The *ERG*2 sequence includes exons used by all other *ERG* isoforms; accordingly, it can be used as a general *ERG* probe. Hybridization was performed with 1 x 10⁶ cpm ³²P-radiolabelled human ERG2 cDNA /ml hybridization solution at 65°C for overnight. Following hybridization, the membranes were washed sequentially with 2xSSC supplemented with 0.1 % SDS, then 0.2x SSC supplemented with 0.1% SDS, at 65°C. Before we isolated DNA for sequencing, we subjected hybridization positive clones to two more rounds of plaque screening to obtain single plaques.

Two clones yielded novel *ERG* isoform transcripts. Each clone has a unique 3' sequence. Because these *ERG* transcripts have only been observed in prostate cancer tissue, we called the clones "EPC1" and "EPC2" for *ERG* **P**rostate **C**ancer-**S**pecific Isoform **1** and **2**.

The nucleic acid sequence of the EPC1 clone is set forth in SEQ ID NO: 3. This transcript is also a fusion between exons of *TMPRSS2* and *ERG.* The *TMPRSS2* derived sequence occurs at the 5' end upstream of the initiation methionine (ATG at position 140 to 142 of SEQ ID NO: 3). The last four carboxy-terminal amino acids of the EPC1 amino acid sequence (SEQ ID NO: 4) are not found in any *ERG* exon, and they appear to be derived from an *ERG* intronic sequence. The unique 3' end of *EPC1* corresponds to nucleotides 788 to 1019 of SEQ ID NO: 3 and can be used in both nucleic acid (*e.g*., amplification and hybridization-based) and protein (*e.g*., antibody-based) detection methods for the detection of cancer cells or precancerous cells in specimens and biofluids.

The nucleic acid sequence of the *EPC2* clone is set forth in SEQ ID NO: 5. The amino acid sequence of EPC2 is set forth in SEQ ID NO: 6. The unique 3' sequence corresponds to nucleotides 127 to 807 of SEQ ID NO: 5. The 5' end corresponds to sequences within *ERG* exon 10, and the sequence appears to continue into the adjacent 3' exon without splicing, resulting in a unique transcript sequence.

We next investigated the expression of *EPC1* transcripts in RNA specimens extracted from laser microdissected (LCM) tumor and benign epithelial cells of 14 prostate cancer patients using RT-PCR. We selected primers specifically recognizing the *EPC1* isoform (p2301-p2302) and used them together with *GAPDH* primers (p2135-p2144) as internal controls in the same reaction tubes. The *EPC1* primer sequences were: p2302: 5' - CAGAAAGCAGCCTTCCCTTA - 3' (SEQ ID NO: 15, corresponding to nucleotides 820 to 839 of SEQ ID NO: 3); and p2301: 5' - TTGATAATAGAGCATCAGACTTCCA - 3 (SEQ ID NO: 16, corresponding to the reverse complement of nucleotides 953 to 977 of SEQ ID NO: 3).

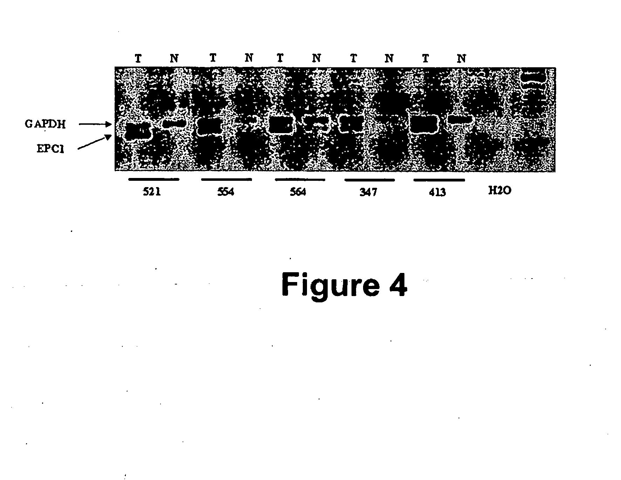

Figure 4 shows a photograph of a PCR gel with data for five of the 14 patients. Tumor cell samples are labeled "T," while the benign epithelial cell samples from each patient are labeled "N." *EPC1* expression was measured, along with the control gene GAPDH. EPC1 expression detected in the tumor cells of 11 of the 14 prostate cancer patients tested. In seven patients, *EPC1* expression could be detected only in their prostate tumor cells, while in four patients, *EPC1* expression could be detected in both their tumor and benign epithelial cells. In those instance where *EPC1* was detected in both tumor and benign epithelial cells, *EPC1* expression was increased in tumor cells relative to benign epithelial cells.

*EPC1* and *EPC2* are *ERG* isoforms that are uniquely expressed in cancerous prostate. At the transcript level, the 3' end of each transcript is unique and distinct from all known *ERG* isoforms. It may be therapeutically beneficial to degrade *EPC1* and/or *EPC2* mRNA (*e.g*., using siRNA or shRNA) or to inhibit the EPC1 and/or EPC2 protein by using antibodies raised against each distinct C-terminal region.

### Example 4: The ERG8 and EPC1 isoforms are abundantly expressed

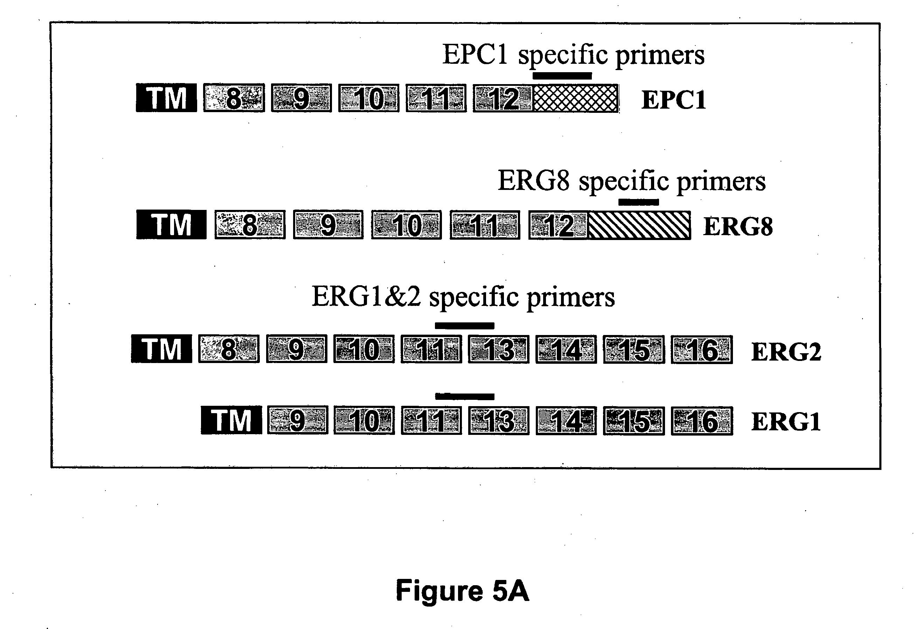

In order to compare the relative abundance of *ERG8* and *EPC1* isoforms to that of *ERG1,* we prepared samples from prostate cancer-derived VCaP cells as well as from microdissected tumor cells from prostate cancer patients. We then used quantitative PCR to determine the copy numbers using primer pairs specific for *EPC*1, *ERG*8, and for a sequence in common between *ERG*1 and *ERG*2. The positions of the various primers and the domain structure of the *ERG* isoforms are shown in Figure 5A. In the schematic diagram, "TM" denotes T*MPRSS2* and the boxes numbered 8-16 correspond to exons, numbered according to Owczarek et al., GENE 324:65-77 (2004)). The ERG8 specific primers and probe were as follows:
ERG8 forward primer: TTCAGAAAGACAGATGGGCAAA (SEQ ID NO: 17);
ERG8 reverse primer: GTTCAAAAGTCGGCCTATTCCTAA (SEQ ID NO: 18);
ERG8 probe: AAGGCATCCTGGATGCCTGGCA (SEQ ID NO: 19);
EPC1 forward primer: GCACTTCTGCCAAGCATATGAGT (SEQ ID NO: 20);
EPC1 reverse primer: CGCTGATCATTTCAACACCCT (SEQ ID NO: 21);
EPC1 probe: TGCCTTGAAGATCAAAGTCAAAGAGAAATGGA (SEQ ID NO: 22);
ERG1/2 Ex 11-13 forward primer:
   TTCAGATGATGTTGATAAAGCCTTACA (SEQ ID NO: 23);
ERG1/2 Ex11-13 reverse primer: TCCAGGCTGATCTCCTGGG (SEQ ID NO:24);
ERG1/2 Ex 11-13 probe: ATGCATGCTAGAAACACAGATTTACCAT (SEQ ID NO: 25).

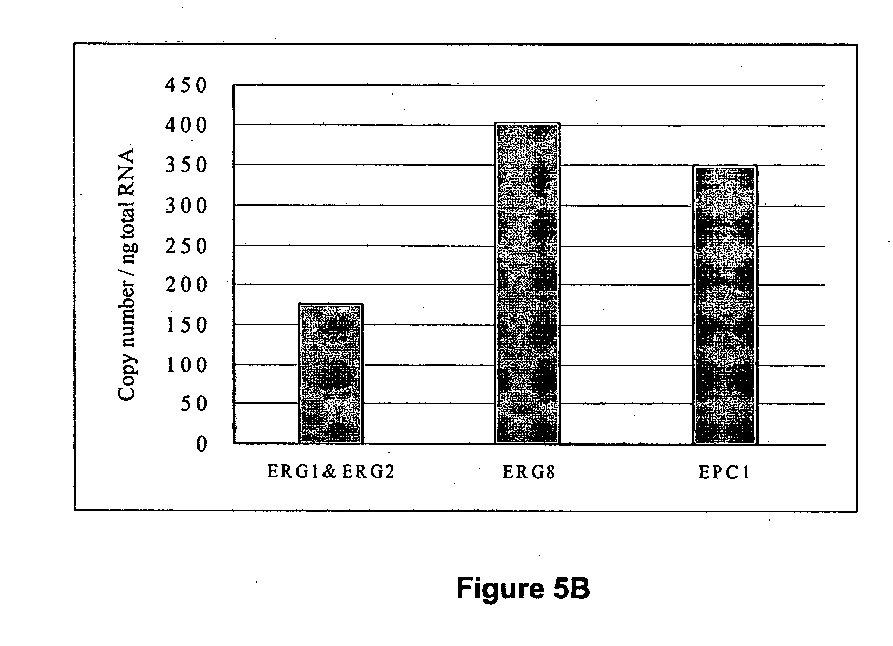

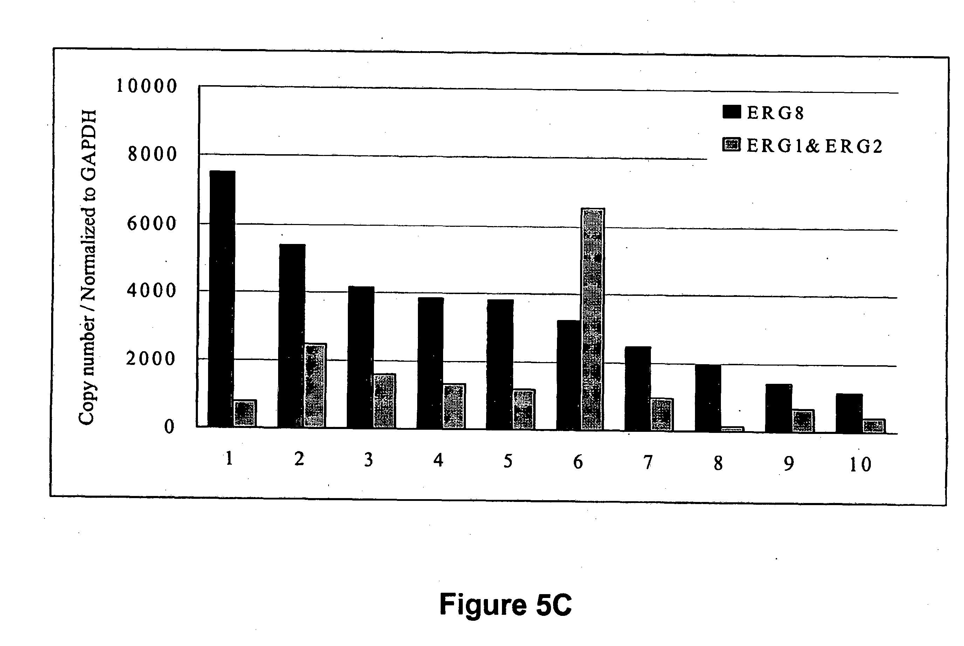

The number of copies of different *ERG* isoforms was determined in VCaP cells by TaqMan QRT-PCR using the specific primers and probes shown in Figure 5A and the results are shown in Figure 5B. Plasmid constructs comprising a target gene (different *ERG* isoforms) insert were used to generate standard dilution series in which the copy number of plasmids in the dilution series is known. A formula was derived from the standard curve to correlate the Ct value with the target gene copy number. Using this formula and the standard curve, we calculated the copy numbers of the target genes in the samples. As shown in Figure 5B, the copy number of both *ERG8* and *EPC1* is two-fold or more greater than the copy number of the combination of *ERG*1 and *ERG*2 in VCaP cells. In addition, microdissected tumor cells of nine of ten prostate cancer patients exhibited a higher copy number for *ERG*8 than for the *ERG*1 and *ERG*2 combination (Figure 5C). These data indicate that the *ERG*8 and *EPC*1 isoforms are abundantly expressed and accordingly provide potential targets in diagnostic and prognostic applications.

### Example 5: Combined detection of ERG8 and EPC1 is inclusive of all TMPRSS2-ERG fusions examined and results in a robust detection system for prostate cancer

Our finding that *ERG8* is overexpressed in prostate cancer and that *EPC1* is selectively expressed in prostate cancer tissue can be used to develop a particularly robust diagnostic and prognostic assays because these two genes possess unique 3' ends. The 3' end of an mRNA transcript is relatively resistant to degradation compared to its 5' end, making it possible to detect sequences near the 3' end in clinical samples that might be difficult to detect if they were located toward the 5' end of the sequence. Thus, although one mechanism of over- or selective expression of *ERG*8, *EPC*1, and *EPC*2 may involve a 5' fusion to *TMPRSS2,* the 3' portion of the *ERG*8, *EPC*1, and *EPC*2 sequences should be more stable and readily detectable in clinical samples than the 5' *TMPRSS2* sequence. As a result, detecting the 3' end of *ERG8, EPC1,* and *EPC2* transcripts can reduce false negatives compared to detecting 5' sequences such as the 5' *TMPRSS2* sequence in *TMPRSS2-ERG* fusion transcripts. In addition, biofluids, such as urine, serum, plasma, saliva, and prostatic fluid that are easier and cheaper to obtain but more prone to mRNA degradation, can be used to detect 3' sequences of *ERG8, EPC1,* and *EPC2.*

Accordingly, we developed a simple PCR assay that detects aberrant expression due to any type of ERG fusion event. We tested an assay that utilizes three pairs of PCR primers. Namely, we used p2302 (SEQ ID NO: 15) and p2301 (SEQ ID NO: 16) to detect *EPC1;* p2220 (SEQ ID NO: 11) and p2198 (SEQ ID NO: 14) to detect *ERG8;* and p2236 and p2237, described in Petrovics et al., ONCOGENE 24:3847-3852 (2005), to detect the 3' UTR of *ERG1*/*2.* These primer combinations detect sequences in the 3' end that are retained following any 5' fusion, such as with *TMPRSS2,* and that are relatively resistant to degradation.

We used these three primer pairs to test the presence or absence of ERG isoforms in LCM selected prostate cancer cells. We divided the samples into two groups, based upon whether we could detect a *TMPRSS2-ERG* fusion transcript. Table 1 presents the results.

**TABLE 1**

| **FP** | ***ERG*fusionA** | ***ERG*fusionA** | ***EPC*1** | ***ERG*8** | ***ERG*1** | **Combined Signal** |
|---|---|---|---|---|---|---|
| FP347 | 0.865 | Yes | T | T | | YES |
| FP411 | 8.07 | Yes | T | T | | YES |
| FP413 | 2.52 | Yes | T | T | | YES |
| FP473 | 5.105 | Yes | T | T | T | YES |
| FP480 | 12.005 | Yes | T | no | | YES |
| FP519 | 1.44 | Yes | T | T | | YES |
| FP521 | 1.07 | Yes | T | T | T | YES |
| FP554 | 3.9 | Yes | T | no | | YES |
| FP564 | 2.24 | Yes | T and N | T | | YES |
| FP703 | 2.66 | Yes | T and N | T | | YES |
| FP245 | -3.305 | Yes | T and N | T | | YES |
| FP349 | 1.315 | Yes | T | T | | YES |
| FP355 | 2.12 | Yes | T | T | T | YES |
| FP391 | 2.16 | Yes | T | T | | YES |
| FP402 | 3.595 | Yes | T | T | T (and N) | YES |
| FP430 | 2.77 | Yes | T | T | | YES |
| FP441 | 6.2 | Yes | T and N | no | | YES |
| FP489 | 3.6 | Yes | T | T | | YES |
| FP504 | 5.435 | Yes | | T | | YES |
| FP510 | 4.47 | Yes | T | no | | YES |
| FP553 | 2.94 | Yes | T | no | | YES |
| FP320 | | No | no | no | | |
| FP326 | | No | no | no | | |
| FP346 | | No | no | no | | |
| FP393 | | No | no | no | | |
| FP513 | | No | no | no | | |
| FP535 | | No | no | no | | |
| FP573 | | No | no | no | | |
| FP590 | | No | no | no | | |
| FP598 | | No | no | no | | |
| FP620 | | No | no | no | | |
| FP257 | | No | T and N | no | | YES |
| FP260 | | No | no | no | | |
| FP318 | | No | | no | | |
| FP394 | | No | no | no | | |
| FP446 | | No | no | no | | |
| FP488 | | No, has fusionC | T and N | T | | YES |
| FP491 | | No | no | no | | |
| FP493 | | No | no | | | |
| FP495 | | No | no | no | | |
| FP508 | | No | no | | | |
| FP523 | | No | no | no | | |
| FP575 | | No, has fusionC | T | T | | YES |

In Table 1, the "FP" numbers in the left column are the coded specimen numbers. The first 21 samples presented are those in which we could detect the "A type" *TMPRSS2-ERG* fusion transcript. *ERG* fusion A is the most frequent fusion (95% of all fusion transcripts) and involves fusion of the first exon of *TMRPSS2* to *ERG* exon 8. The numeric values in the first column labeled "*ERG*fusionA" indicate the threshold cycle numbers, normalized to GAPDH, in a quantitative RT-PCR analysis. In 22 samples, we were unable to detect *ERG* fusion A, but in two samples, FP488 and FP575, we detected *ERG* fusion C. Fusion "C" is a rare fusion between *TMPRSS2* exon 1 and *ERG* exon 9. In the EPC1, *ERG*8, and *ERG*1 columns, "T" indicates detection in tumor cells, "N" indicates detection in normal epithelial cells, and "no" indicates that no signal was detected. The "combined signal" column summarizes the cumulative detection of *ERG* products ("YES" = expression of any of isoforms *EPC1, ERG8,* or *ERG1*).

By using this combined signal approach, we could detect an amplification product in all samples bearing an *ERG* fusion. In addition, in those samples in which *EPC1* was detected but *ERG8* was not (*e.g.,* FP480), we still obtained a combined signal. Although we examined *ERG1* expression in several samples to validate the assay, the results show that it is not necessary to include *ERG1* in the analysis. Instead, the combined signal from *EPC1* and *ERG8* was all that was needed to detect all fusion events. Accordingly, the combined signal approach can help to minimize false negatives that could arise by looking only at a particular *ERG* transcript. In addition, we expect that the combined approach can be readily used in clinical samples, such as biofluids, that would be inappropriate for use with primers for the more 5' *TMPRSS2-ERG* fusion event.

### Example 6: A novel ERG promoter is activated in prostate cancer

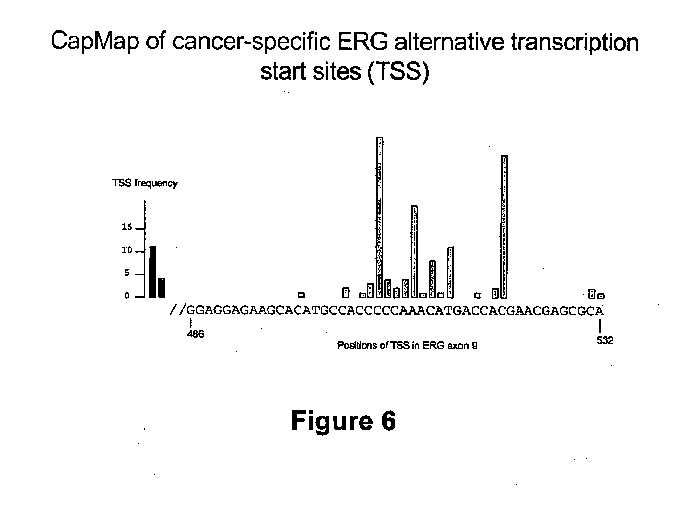

To determine whether there are additional alterations that occur in the *ERG* locus in prostate cancer, we systematically evaluated transcription initiation sites within the *ERG* locus using the 5' oligocapping method. This information was used to map cancer-specific *ERG* alternative transcription start sites. We isolated total RNA from prostate cancer tissues of six patients with verified *ERG* gene rearrangements, pooled the RNA, then treated it with dephosphatases to degrade non-capped RNA molecules, thereby enriching 5' cap protected mRNA molecules. An RNA oligonucleotide adapter was ligated to substitute the 5' capping structure and cDNA was generated by reverse transcription. We then used the oligocap adaptor and internal primers from *ERG* exon 10 to amplify 5' *ERG* sequences. In the first amplification we used *ERG* primer p2181: 5'-GGCGTTGTAGCTGGGGGTGAG-3' (SEQ ID NO: 26). In the second amplification, we used *ERG* primer p2268: 5'-CAATGAATTCGTCTGTACTCCATAGCGTAGGA-3' (SEQ ID NO: 27). The resulting PCR products were cloned into the pUC19 vector, then sequenced. DNA sequences indicating transcription initiation sites from ERG sequences in tumor tissue were matched to the *ERG* locus and analyzed by generating a score that represented the frequency of individual transcription start sites within the locus. The 5' capping frequency map (CapMap) of *ERG* gene transcripts is shown in Figure 6. Of the 152 clones sequenced, 137 of the 5' capping clones had novel, prostate cancer-specific transcription initiation sites within a 23 bp *ERG* exon 9 region.

In a separate oligocapping experiment, 5' cap sites were assessed in RNA from normal prostates pooled from 11 individuals (AMBION) with a negative prostate cancer diagnosis. In this experiment, we terminated our analysis after 30 clones because the products were homogenous. The results indicated that transcription initiation in normal prostate uniformly occurs in *ERG* exon 5, in sharp contrast to the multiple exon 9 initiation sites we observed in the tumor specimens. Transcription initiation in *ERG* exon 5 indicates that *ERG* isoform 3 is expressed in normal prostate. Also, our results suggest that *ERG* isoforms 1, 2, 5, 6, 7, 8 and 9 are either not expressed in normal prostate, or they are present only at low levels.

The transcription initiation sites detected in the prostate cancer samples indicated that the central segment of *ERG* exon 9 is a cancer-specific promoter site. The promoter region is defined as the area between -520 bp and + 130 bp relative to the most 3' transcription initiation site detected in the mapping experiment. The promoter sequence is set forth below: 
TCTGTCGCCA GTCTGGAGTG CAGTGGCATG ATCTCAGCTC ACTGCAACCT 50
CCACCTCCCG GATTCAAGCA ATTTTCCTGC CTCAGCCTCC TGAGTAGCTG 100
GGACTACAGG CATGCCCAGC TAATTTTTGT ATTTTTAGTA GAGACGGGGT 150
TTCACCATGT TGGCCAGGAT GGTCTGGATC TCTTGACCTC ATGATCCGCC 200
CACCTCGGCA TCCCAAAGTG TTGGGACTAC AGGCATGAGC CACGGCACCC 250
CGCCTGTATT TGGCTTTTCA CACTTGTCCT TTCTCCCCCA GTCTCTTCCG 300
CCTTGCCCTT CTTTGGTTCT CTCTGTGTAT TGTGAGAAGT CGATGGAGAC 350
ATGCTCTTTG ATTGCTGTTA TAATGGAAGA ATATTTCTTC TCCTCCAGGA 400
ACTCTCCTGA TGAATGCAGT GTGGCCAAAG GCGGGAAGAT GGTGGGCAGC 450
CCAGACACCG TTGGGATGAA CTACGGCAGC TACATGGAGG AGAAGCACAT 500
GCCACCCCCA AACATGACCA CGAACGAGCG CAGAGTTATC GTGCCAGCAG 550
GTCAGGTGCC CACAGCTTCA CTGCCCTCGG CAGATCGCAA CTTCCCCAAG 600
GCTAGGCTGA GCCTCAGGGA GCTCTTCTCC CCCACCTGTG GCATTGATCA 650
 (SEQ ID NO: 7). In the sequence, the most 3' transcription start site that is frequently used is bolded and boxed.

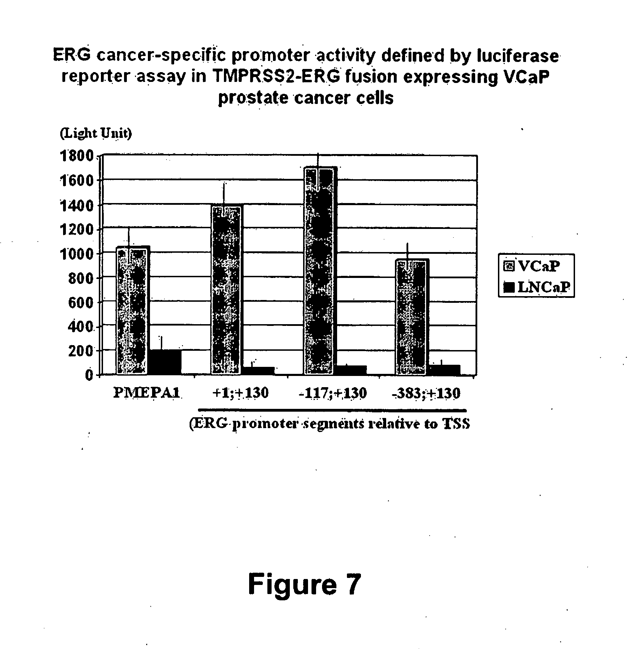

The putative TATA-less promoter is predicted at -20; -40 bp from the transcription initiation site. Interestingly, there is also a MED (Multiple Elements of Initiation Downstream) in the +130 region, which may explain the multiple start sites we observed. We have verified that this promoter is functional by operably linking it to the luciferase reporter gene. Figure 7 demonstrates that the prostate cancer-specific promoter is able to direct expression of luciferase protein in prostate cancer-derived VCaP cells (light grey bars), which contain a TMPRSS2-*ERG* fusion, but not in LNCaP cells (dark grey bars). A promoter fragment from -117 to +130 (nucleotides 404 to 650 of SEQ ID NO: 7) yielded the greatest expression levels in the luciferase assay, followed by the +1 to +130 fragment (nucleotides 521 to 650 of SEQ ID NO: 7), then the - 383 to +130 fragment (nucleotides 138 to 650 of SEQ ID NO: 7).

Activation of a dormant promoter within the *ERG* gene locus in a cancer specific manner produces transcripts coding for N-terminal deletion mutants. The encoded protein products lack the protein-protein interaction domain of wild type *ERG*. Therefore, expression products of this dormant promoter may act as dominant negative or gain-of-function molecules. Nucleic acid or protein-based products that manipulate the activity of this promoter can therefore be used for prostate cancer therapy. In addition, the prostate-specific expression of this promoter means that expression vectors in which the promoter is operably linked to a gene encoding a toxin or other inhibitor of cell growth can be used to selectively express the encoded protein in prostate cancer cell.

### Example 7: A regulatory loop exists between ERG and the androgen receptor

Gene rearrangements involving the fusion of the androgen receptor-regulated *TMPRSS2* gene promoter and *ERG* occur at a high frequency (-60%) in prostate cancer and are likely to be a direct cause of prostate cell transformation, but the mechanism by which the genomic alteration leads to prostate cancer is thus far unexplained. It is likely that this genomic alteration is responsible for, or at least contributes to, the overexpression of *ERG1* in prostate cancer. It is also known that androgen receptor function is central to the growth and differentiation of the normal prostate gland. Further, androgen receptor dysfunction favors the growth and survival of prostate cancer cells and appears to play a role in prostate cancer progression. It is unclear, however, how these alterations interact to result in prostate cancer.

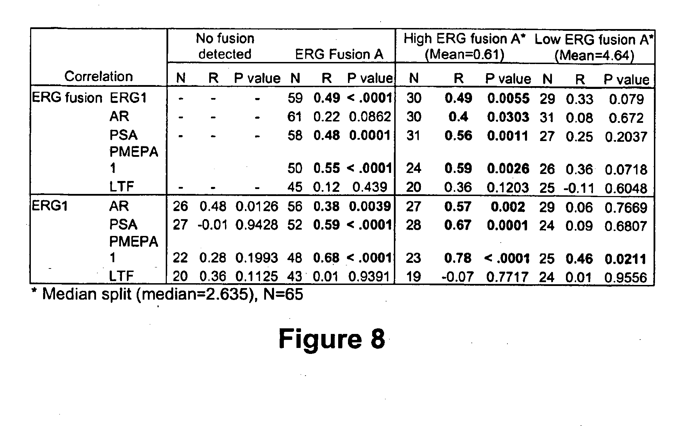

To investigate whether *ERG* protein contributes to prostate cancer by interfering with androgen receptor signaling, we correlated the expression of *TMPRSS2-ERG* fusion transcripts with *ERG1*, androgen receptor (*AR*), *PSA,* and the androgen-regulated gene *PMEPA1*. *LTF* was also analyzed as a negative control. The results of this analysis are shown in Figure 8, which compares the quantitative RT-PCR data for the *TMPRSS2-ERG* fusion to quantitative RT-PCR data obtained by amplifying the 3' untranslated regions of *ERG ("ERG1").*

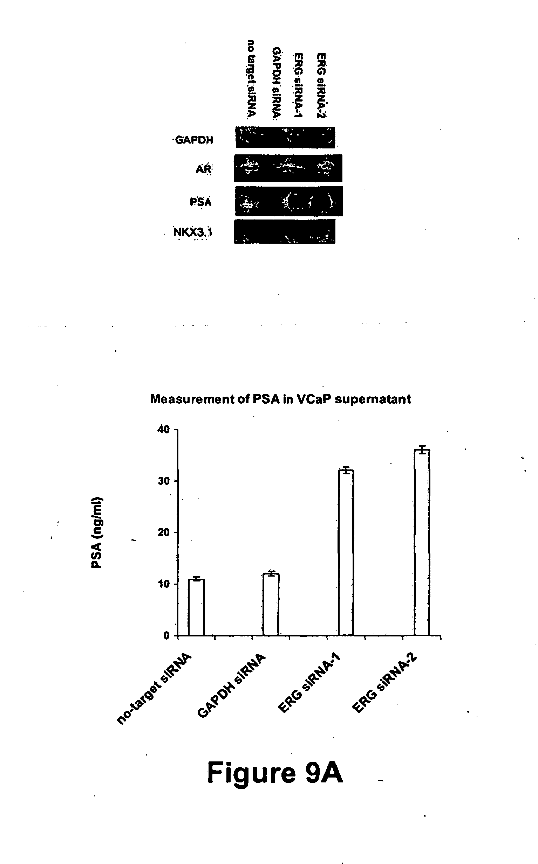

We next investigated the effect of *ERG* expression on transcriptional targets of the androgen receptor. We introduced two different *ERG* siRNAs into the VCaP prostate cancer cell line. The sequence of the siRNAs is: siRNA-1 (p2094): TGATGTTGATAAAGCCTTA (SEQ ID NO: 28). which targets exon 11, and siRNA-2 (p2095): CGACATCCTTCTCTCACAT (SEQ ID NO: 29), which targets exon 10. VCaP cells possess a *TMPRSS2-ERG* fusion and overexpress *ERG.* The results of these experiments are shown in Figure 9A. Introduction of either siRNA led to the up regulation of *NKX3.1* and *PSA*/*KLK3* (Figure 9A, top panel). The upregulation of *PSA*/*KLK3* could also be detected as increased PSA levels in the VCaP culture supernatant (Figure 9A, bottom panel).

*ERG* knockdown also inhibited prostate tumor cell proliferation *in vitro* and *in vivo.* Figure 9B shows the morphology of VCaP cells transfected with 50 nM *ERG* siRNA, compared to controls ("NT"). Experiments were performed in triplicate and the cell morphology monitored on days 1, 4, 6, and 8 post transfection (Figure 9B, top left panel).

*ERG* siRNA inhibited VCaP cell proliferation, decreasing the number of cells present in the cultures compared to cells treated with control RNA (Figure 9B, top right panel). Cells transfected with *ERG* siRNA were counted in triplicate at days 1, 2, 4, 6, 8, and 10 post transfection, and observed to grow at a slower rate than control cells (p=0.001).

Fluorescent activated cell sorting ("FACS") analysis of the cell cycle demonstrated a inhibitory effect of *ERG* siRNA on the number of cells in S phase, compared to control RNA. The population of cells in S phase and the distribution of cells in G1, S and G2+M phases was assessed by analyzing the cells at the indicated time points from three independent experiments. Phospho-Rb to total Rb ratios were measured at day four by Western blot assay. *ERG* siRNA induces a redistribution of the number of cells in G1, S and G2+M phases, significantly increasing the number of cells in S phase.

Inhibiting *ERG* expression with ERG siRNA drastically reduced the growth of VCaP cells injected into severe combined immunodeficient ("SCID") mice. Male SCID mice (Harlan Sprague-Dawley, National Cancer Institute, Frederick, MD) 4-6 weeks old and weighing 18 to 20 g, housed in sterile filter-capped cages, fed and given water *ad libitum,* were injected subcutaneously with VCaP cells. All animal studies were carried out according to NIH-approved protocols, in compliance with the Guide for the Care and Use of Laboratory Animals.

The injected VCaP cells were treated with *ERG* siRNA or NT control RNA, trypsinized, washed, and three million cells in a volume of 0.2 ml were injected into the flanks of the SCID mice. Tumor formation was assessed bi-weekly for up to seven weeks. Tumor growth analysis was performed by determining tumor volume (LW2/2), as described by Polverino et al., CANCER RES 66:8715-21, whereby L and W represent the length and the width of the tumor.

Figure 9B, lower right panel, shows the tumor growth at days 35 (p= 0.0072) and 42 (p= 0.0072). Standard deviation (SD) and average tumor volumes (AVG) at day 42 are shown in the table. All of the animals treated with *ERG* siRNA had a reduced tumor load compared to the control animals and some had no tumor load (Figure 9B, lower right panel).

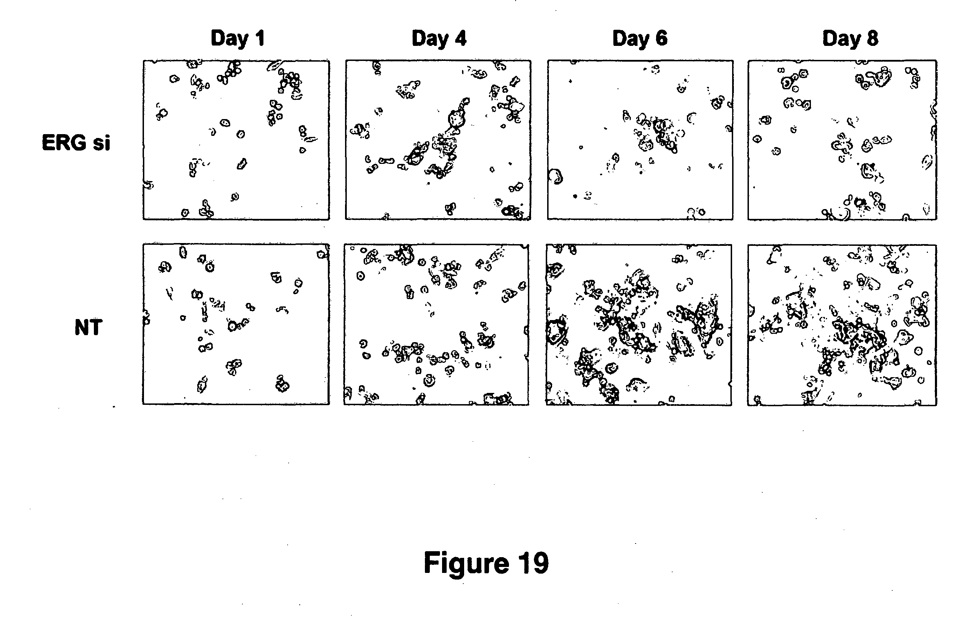

ERG knockdown inhibited VCaP cell growth in complete medium. VCaP cells seeded into 10 cm tissue culture dishes in medium comprising 10% cFBS were cultured for three days, then *ERG* siRNA or control RNA. Cells from triplicate experiments were examined on day 1, 4, 6, and 8 by microscopy, as shown in Figure 19.

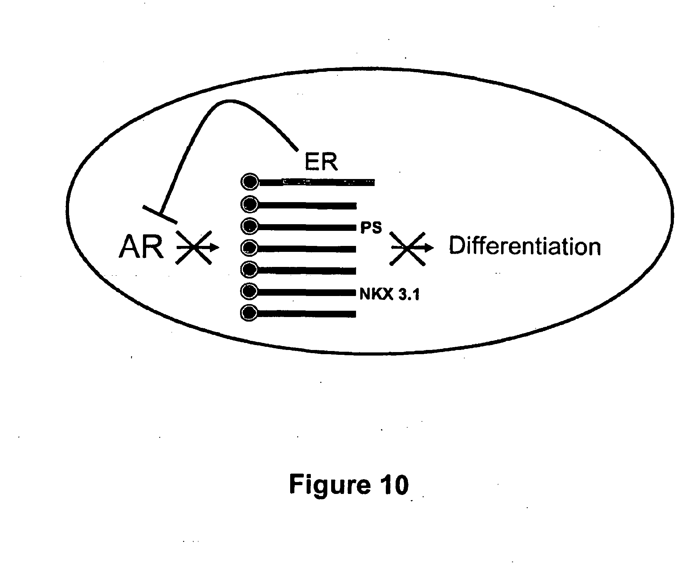

These data demonstrate a relationship between the *in vivo* expression of *TMPRSS2-ERG* or *ERG* and the expression of other androgen-regulated genes, such as *PSA*/*KLK3* and *NKX3*.*1*. Because downregulation of *ERG* expression correlates with increased expression of *NKX3.1* and *PSA*/*KLK3,* this indicates that these androgen-regulated genes are downregulated by ectopic *ERG* expression. *NKX3*.*1* is a tumor suppressor gene and also a transcriptional target of the androgen preceptor. Suppression of *NKX3.1* by *ERG* overexpression in prostate cancer cells may therefore interfere with androgen receptor-mediated cell differentiation and negative regulation of cell growth. A schematic of this model is shown in Figure 10.

As an example of an application for the use of inhibitory molecules in targeting transcripts of the *ERG* locus for degradation, we used siRNA-1 to inhibit *ERG* expression in VCaP prostate cancer cells. As noted, siRNA-1 targets exon 11, which is found in *ERG1, ERG2, ERG3, ERG8, EPC1,* and *EPC2* transcripts and in the predicted products of the alternative internal promoter. VCaP cells respond to androgen hormone treatment, therefore, the effect of *ERG* inhibition on cell growth can be tested by stimulating the cells with androgen hormone.

To perform the siRNA inhibition, cells were first plated to 30% confluence in 100 mm cell culture dishes. Growth was synchronized by incubating the cells in hormone depleted serum (cFBS) containing media for three days. Then the cells were transfected with siRNA-1 and non-targeting (NT) control siRNA using the lipofectamine 2000 reagent (Invitrogen, Carlsbad, CA). After transfection 0.1 nM of R1881 synthetic androgen (New England Nuclear, Boston, MA) was added to the media. The cells were incubated for nine days, with a media change every three days. Cell cultures were then photographed and 100X magnification of representative view fields were captured.

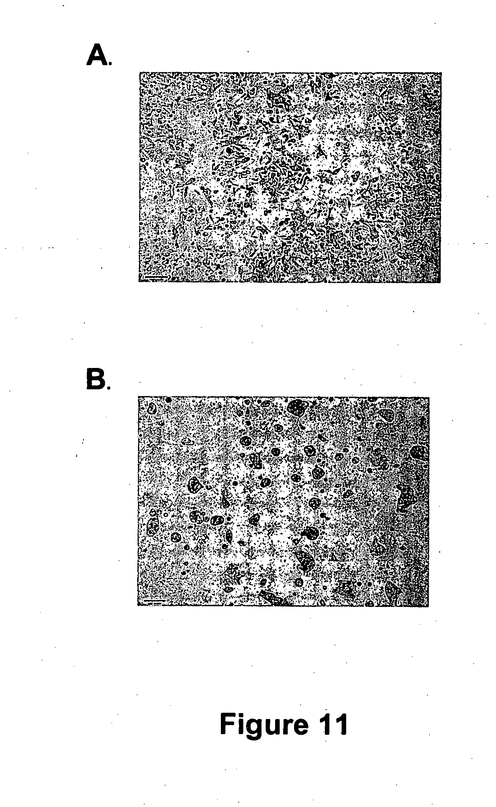

A microscopic view of VCaP cells is shown in Figure 11. Cells treated with the control NT siRNA are shown in Figure 11A, while Figure 11 B shows cells treated with siRNA-1. VCaP cells treated with siRNA-1 exhibited a robust reduction in cell numbers. In addition, striking changes in cell morphology were also apparent. Thus, we were able to show that siRNA-1 treatment inhibited androgen-stimulated growth of VCaP cells.

Taken together, these data suggest that there is a regulatory loop between *ERG* and the androgen receptor and that negative regulation of the androgen receptor by *ERG* may contribute to prostate tumorigenesis. Accordingly, there are several therapeutic interventions that can be applied in an early stage prostate cancer (such as well to moderately differentiated tumors) harboring a *TMPRSS2-ERG* fusion or *ERG* overexpression. For example, *ERG-*siRNA, shRNA, or other small molecules can be used to reduce *ERG* expression in early stage prostate cancer, which is the most common stage of prostate cancer identified in post-PSA screening era. Alternatively or in addition, the androgen receptor can be selectively inhibited with beneficial effects.

It should be again noted that in the context of therapeutic interventions, any mention of *"ERG"* includes not only *ERG8, EPC1, ECP2,* and transcript products from the prostate cancer-specific promoter described herein, but also *ERG1, ERG2,* and *ERG3,* as well as their combinations, unless specifically indicated to the contrary by context or by an explicit exclusion of one or more of those isoforms. Thus, although we have exemplified inhibition of androgen-stimulated growth with an siRNA specific for exon 11, which is shared by *ERG1, ERG2, ERG3, ERG8, EPC1* and *EPC2* transcripts, siRNA, shRNA, or other small molecule inhibitors targeted to only one, or any combination of more than one, of those isoforms may also be employed. Such siRNA, shRNA, or other inhibitors that are specific for only one of *ERG1, ERG2, ERG3, ERG8*, *EPC1, EPC2,* or a transcript product from the prostate cancer-specific promoter, or that inhibit combinations of those isoforms, can be designed using the sequence data provided elsewhere in the Examples and may include the various primer and probe sequences mentioned.

For example, *ERG8* gene expression can be blocked by targeting ERG8 specific nucleotide sequences with inhibitory nucleic acids. Examples of sense-strand specific sites suitable for targeting are:
5'-GGAACCACTTCTAGCAATA-3' (SEQ ID NO: 41)
5' -CGAATAATGAGCAGGGAGA-3' (SEQ ID NO: 42)
5' -CCAGGGAGCTAAAGAGAAT-3' (SEQ ID NO: 43)
5' -CTGGGAAGCATGATGGAAA-3' (SEQ ID NO: 44)
5' -GACTCAAGCTTTAGAGATT-3' (SEQ ID NO: 45)
SEQ ID NO: 41 corresponds to nucleotides 1595-1613 of SEQ ID NO: 30; SEQ ID NO: 42 corresponds to nucleotides 1722-1740 of SEQ ID NO: 30; SEQ ID NO: 43 corresponds to nucleotides 2013-2031 of SEQ ID NO: 30; SEQ ID NO: 44 corresponds to nucleotides 2150-2168 of SEQ ID NO: 30; and SEQ ID NO: 45 corresponds to nucleotides 2334-2352 of SEQ ID NO: 30.

Progressive tumors that do not express ERG, or express ERG only at low levels, reflect an escape from an intact androgen receptor signaling network. These tumors may be treated by selective upregulation of androgen-regulated genes (*e.g.,* tumor suppressors or cell differentiation and growth inhibitors, such as NKX3.1 and PMEPA1), so as to restore the protective component of the feedback regulation between *ERG* and the androgen receptor.

### Example 8: The androgen receptor function index

The readout of androgen receptor ("AR") regulated genes ultimately reflects the status of *in vivo* AR function (ARF) in primary prostate cancer tissue, and consequently carries important information regarding prognosis and rational therapeutic decision making. Assessing the status of AR function in prostate cancer samples can provide early warning signs of androgen independence (van Gils et al., EUR UROL 48(6):1031-41 (2005)). Well characterized, annotated, and preserved human tissues (with long term follow-up data) from the CPDR Biospecimen Bank were used in high throughput screens to identify and validate prostate cancer biomarker genes.

In recent years, we have analyzed cell type specific gene expression from microdissected matched tumor and benign prostate epithelial cells. We found a general decrease in androgen regulated gene expression with prostate cancer progression. (Petrovics et al., ONCOGENE 24:3847-52 (2005).) Others have also recently noted a signature of attenuated AR function in late stage, especially in metastatic prostate cancer in human specimens (Tomlins et al., NAT GENET 39(1):41-51 (2007)), as well as in a xenograft model system (Hendriksen et al., CANCER RES 66(10):5012-20 (2006)). As part of a 12-gene panel, PSA was found to be underexpressed in aggressive prostate cancer. (Bismar et al., NEOPLASIA 8(1):59-68 (2006).) It should be noted, however, that several laboratories reported high AR expression, amplification, or activity in late stage metastatic prostate cancer. (Heinlein et al., ENDOCRINE REV 25:276-308 (2004); Chen et al., NAT MED 10:26-7 (2004); Dehm et al., J CELL BIOCHEM 99:333-344 (2006); Linja et al., CANCER RES 61:3550-55 (2001); Li et al., Ann J SURG PATHOL 28:928-34 (2004).) These different findings underline the heterogeneous nature of late stage, especially androgen independent, metastatic prostate cancer. (Shah et al., CANCER RES. 64(24):9209-16 (2004).)

To develop an *in vivo* readout of AR functional status in prostate cancer cells, we have been pursuing parallel quantitative measurements of various AR regulated genes in carefully isolated benign and tumor cells of over 200 specimens as shown in Example 8. Quantitative expression analyses of androgen regulated genes at the mRNA level, such as *PSA*/*KLK3, PMEPA1, PCA3,* as well as androgen independent genes *(AMACR, LTF),* representing over 2000 data points, suggest that *PSA*/*KLK3* and other androgen regulated genes reflect *in vivo* functional status of the AR and that their expression levels can be used to measure positive or negative correlation with aggressiveness of prostate cancer, as defined, for example, by Gleason grade, pathological stage, and/or biochemical recurrence. Initially we chose to focus on *PSA*/*KLK3* mRNA as it is one of the most robust direct transcriptional targets of AR and is easily detectable in prostate cancer cells. (Kim et al., J CELL BIOCHEM 93(2):233-41 (2004).)

Our most recent data show that quantitative gene expression patterns of a panel of AR regulated genes in primary prostate cancer provide prognostic fingerprints. Using high-throughput assays as well as rational candidate gene strategies, we defined a set of six androgen inducible/co-regulated genes *(PSA*/*KLK3, PMEPA1, NKX3.1, ODC1, AMD1,* and *ERG).* Different combinations of two or more of these six genes, or their isoforms, can be used to provide a quantitative measure of *in vivo* AR function in prostate cancer specimens, *i.e.,* the androgen receptor function index, or ARF index (ARFI). Although real time, quantitative PCR (QRT-PCR) was used to measure the expression levels of these genes, other techniques known in the art, such as immunohistochemistry, can be used to detect RNA or protein levels.

The ARFI readout can be converted into a single number index representing the overall *in vivo* AR activity, which in turn can be incorporated into nomograms, such as the one created by Kattan *et al.* that demonstrated the importance of PSA, Gleason sum, extra-capsular extension, surgical margins, seminal vesicle invasion, lymph node involvement, treatment year, and adjuvant radiotherapy in predicting 10-year probability of prostate cancer recurrence after radical prostatectomy. The nomograms can be used to model time-to-event data, including prediction of prostate cancer progression, combined with established clinical and pathological characteristics that predict this endpoint. The concordance index, C, can be used to assess the improvement in model fit upon inclusion of ARFI. (Harrell et al., JAMA 247(18):2543-6 (1982).) Current nomogram calculators incorporate measurable patient factors in an attempt to use such factors to predict an outcome, such as PSA recurrence following surgery, to aid in treatment decision making in advance of invasive procedures.

The ARFI genes are either direct targets of AR or are tightly regulated by AR, and cover major biological functions regulated by AR in prostate cancer. The gene set includes five androgen regulated genes and *ERG.* Our original observations of frequent overexpression of certain isoforms of *ERG* in prostate cancer (Foley et al., ENDOCR RELAT CANCER 11(3):477-88 (2004)), and subsequent independent study showing prevalent chromosomal rearrangements leading to the activation of *ERG* expression through AR-regulated *TMPRSS2* gene promoter (Tomlins et al., SCIENCE 310(5748):644-8 (2005)), have highlighted *ERG* as an aberrant AR activated gene specific to prostate cancer. Therefore, the quantitative evaluation of *ERG* expression has been integrated in ARFI. The *ERG* read-out can be applied to *TMPRSS2-ERG* positive tumors, which account for greater than 60% of prostate cancer patients. (*Id*.)

It should be noted that although the following Examples use *ERG1* as a model *ERG* isoform, *ERG2, ERG3, ERG8, EPC1, ECP2,* and combinations of those *ERG* isoforms can also be used, as may transcript products from the prostate cancer-specific promoter described in Example 5. Accordingly, any mention of *"ERG"* in the context of an ARFI readout includes not only *ERG1,* but also *ERG2, ERG3, ERG8, EPC1, ECP2,* and transcript products from the prostate cancer-specific promoter described herein, as well as their combinations, unless specifically indicated to the contrary by context or by an explicit exclusion of one or more of those isoforms. For example, ARFI readouts may employ an *ERG* gene that is not *ERG1* or *ERG2*. Similarly, in some embodiments it may be desirable to include *ERG8, EPC1,* or *EPC2* in the readout, but not *ERG1* or *ERG2.*

### Example 9: Co-regulation of ARFI genes reflects robust in vivo functional linkage to AR signaling

We have recently completed a comprehensive gene expression analyses of microdissected prostate cancer cells and matched benign epithelial cells from radical prostatectomy specimens of 40 patients (80 GeneChips) (Petrovics et al., ONCOGENE 24:3847-52 (2005)). The GeneChip dataset was evaluated for androgen regulated gene expression. *PSA*/*KLK3, PMEPA1, NKX3.1, ODC1,* and *AMD1,* along with *ERG* (which can become androgen regulated in prostate cancer cells through fusion with a *TMPRSS2* promoter in the majority of patients), were selected by their wide dynamic ranges of expression, as well as by their reported response to androgenic stimuli. (Heinlein et al., ENDOCRINE REV 25:276-308 (2004); Linja et al., J STEROID BIOCHEM MOL BIOL 92:255-64 (2004); Shaffer et al., LANCET ONCOL 4:407-14 (2003); Chen et al., NAT MED 10:26-7 (2004); Dehm et al., J CELL BIOCHEM 99:333-344 (2006); Segawa et al., ONCOGENE 21(57):8749-58 (2002); Xu et al., INT J CANCER 92(3):322-8 (2001).) Moreover, some of these genes (*NKX3.1, ERG, PMEPA1)* may be causally linked to prostate cancer development.

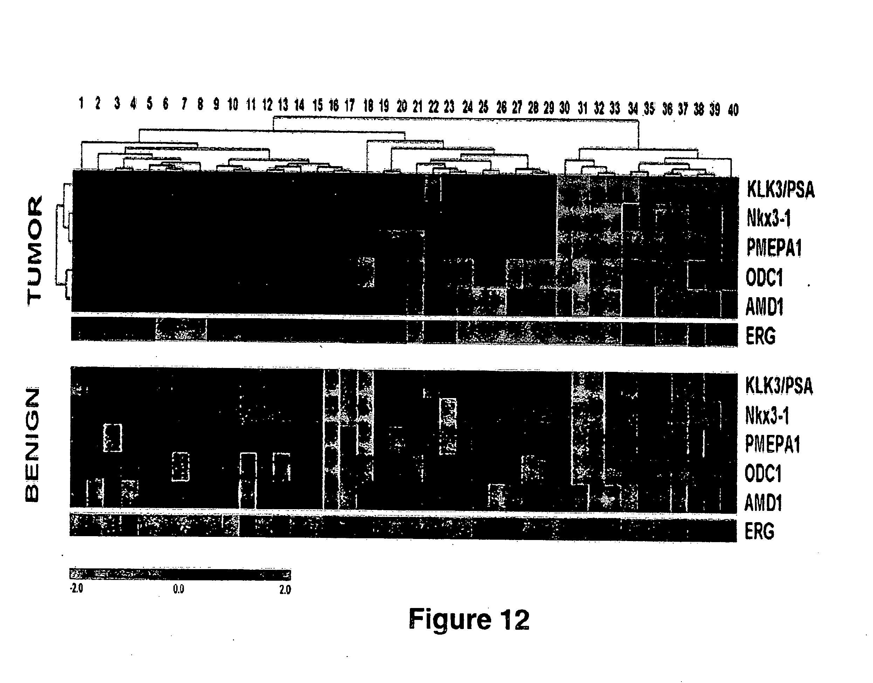

The concerted expression of this gene panel (ARFI) is reflective of the functional status of *in vivo* AR activity. Normalized expression intensity values are depicted in a heat map format (Figure 12). A non-supervised hierarchical cluster analysis (software from TIGR, Gaithersburg, MD) was performed both by patients and also by genes and revealed robust *in vivo* co-regulation of ARFI genes in the tumor cells of prostate cancer patients, reflecting either active or dysfunctional AR (Figure 12). Two tight gene sub-clusters emerged: *PSA*/*KLK3*, *NKX3.1, PMEPA1,* and *ODC1, AMD1* (polyamine pathway), differing in expression only in the middle 12-patient cluster, which underlines the importance of using a panel of ARFI genes representing different downstream AR pathways. The other two large patient clusters show tight co-regulation of all ARFI genes reflecting either active AR (left 17-patient cluster), or dysfunctional AR (right 11-patient cluster) (Figure 12). *ERG* also co-regulates closely with other ARFI genes in tumor cells of the majority of prostate cancer patients, where *ERG* is likely fused to the androgen regulated *TMPRSS2* promoter, providing a highly specific tumor cell marker.

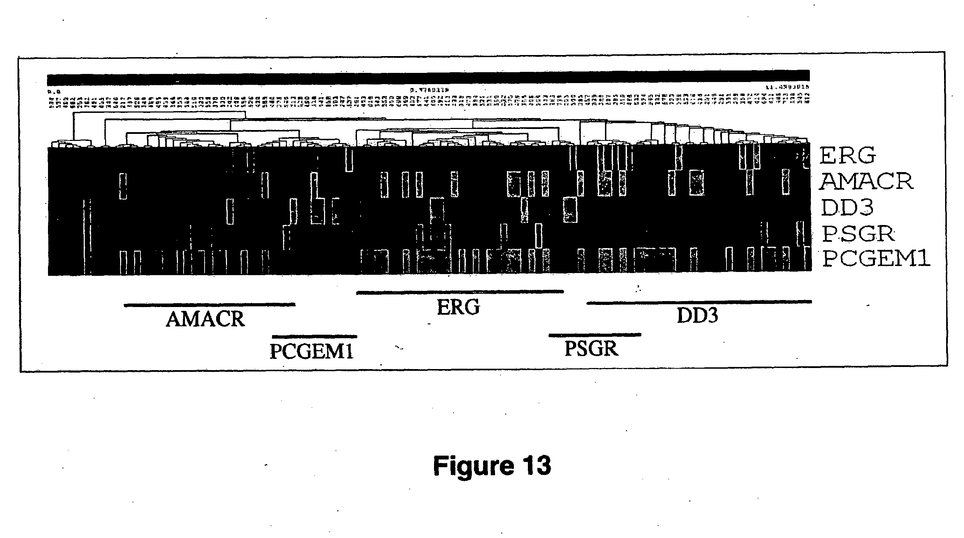

We have also shown that *ERG* can be used as part of a multigene panel with other prostate cancer-associated genes that are not androgen regulated. Figure13 shows a heat map for a mutligene panel that includes *ERG, AMACR, DD3, PSGR,* and *PCGEM1.* The heat map is a non-supervised hierarchical clustering of tumor over normal gene expression ratios derived from TaqMan QRT-PCR analysis of microdissected cell samples from prostate tissue sections. When non-AR genes were used in the multigene panel, we found strong overexpression of the various marker genes in distinct, but overlapping subsets of patients.

### Example 10: Validation of in vivo co-regulation of ARFI genes by QRT-PCR

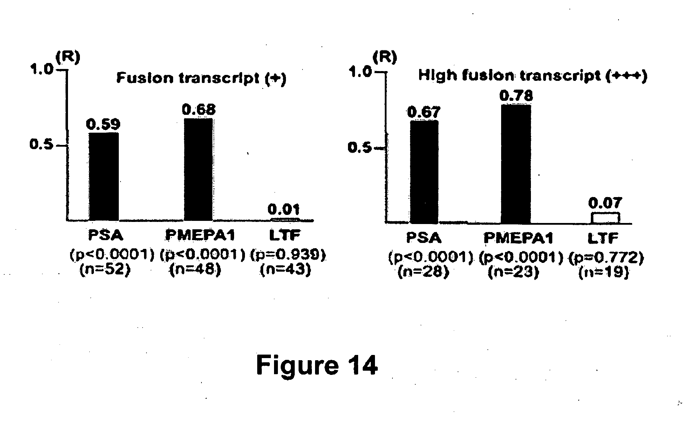

Using QRT-PCR, we evaluated the expression of *ERG* transcripts for a relationship with the expression of the androgen-regulated genes, *PSA*/*KLK3* and *PMEPA1* (Dehm et al., J CELL BIOCHEM 99:333-344 (2006); Xu et al., CANCER RES 63(15):4299-304 (2003)), in prostate cancer cells of patients with *TMPRSS2-ERG* fusion. *LTF* (Ward et al., CELL MOL LIFE SCI 62(22):2540-8 (2005)), a non-androgen regulated control gene, was assayed in the same tumor cells (Figure 14). In the figure, significant correlations (R>0.5) are marked by solid bars. *LTF,* a non-androgen regulated gene, was used as a negative control. The Pearson correlation coefficient (R) is shown above the bars. P values and the number of patients (n) assessed in the experiments are indicated under the bars.

Sixty five patients with detectable *TMPRSS2-ERG* fusion transcript in prostate cancer cells were selected for this study. Striking co-regulation was observed between the expression levels of *ERG,* tissue *PSA*/*KLK3* (p<0.0001) and *PMEPA1* (p<0.0001) in patients with detectable *TMPRSS2-ERG* transcripts. The co-regulation is even stronger in the subset of these patients where the expression level of the *TMPRSS2-ERG* fusion transcript is above the median ("High fusion transcript," Figure 14 right panel). These data indicate that the level of co-regulation within the ARFI genes (including *TMPRSS2-ERG)* reflects the overall functional status of AR in prostate cancer cells and that decreased expression of ARFI genes correlates with compromised or diminished androgen receptor signaling in prostate tumor cells. Furthermore, the data indicate that the expression levels of ARFI genes are reduced in advanced prostate cancer, such as pT3 stage prostate cancer.

### Example 11: PSA/KLK3 and TMPRSS2-ERG indicate a decrease in in vivo AR activity during prostate cancer progression

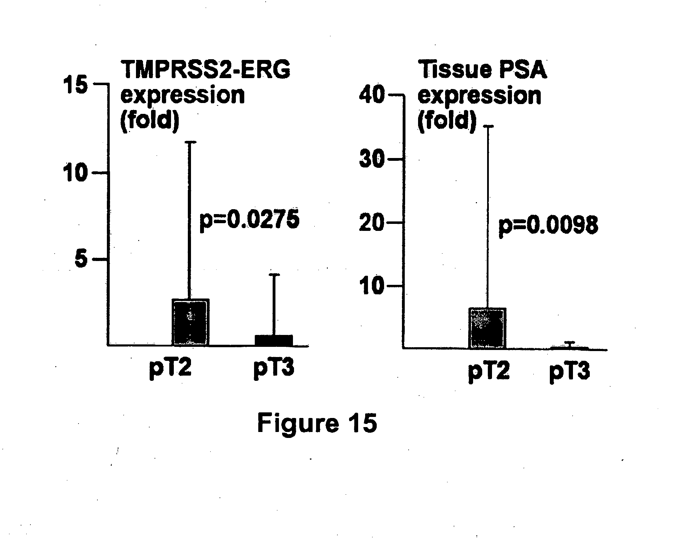

*PSA*/*KLK3* and *ERG* mRNA expression were further analyzed for their relationship to prostate cancer progression in a larger patient cohort. As shown in Figure 15, patients with pT3 prostate cancer (locally invasive tumor growing outside the capsule) had significantly (p=0.0098) lower expression of *PSA*/*KLK3* transcript levels as compared to patients with pT2 stage disease (organ confined). Moreover, decreased *TMPRSS2-ERG* fusion transcript levels were also apparent in the prostate cancer cells of pT3 patients (p=0.0275).

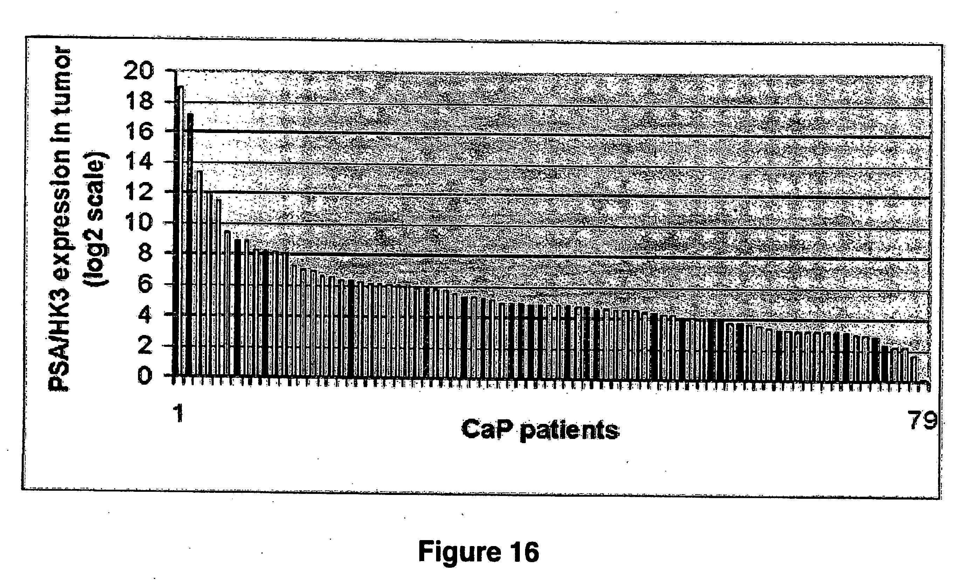

To study patients with intermediate serum PSA levels, further analysis was limited to patients with serum PSA from 2 to 10 ng/mL (n = 79). Based on serum PSA levels, these patients have an uncertain prognosis. Figure 16 shows the distribution of *PSA*/*KLK3* mRNA expression levels in tumor cells of prostate cancer patients with biochemical recurrence.

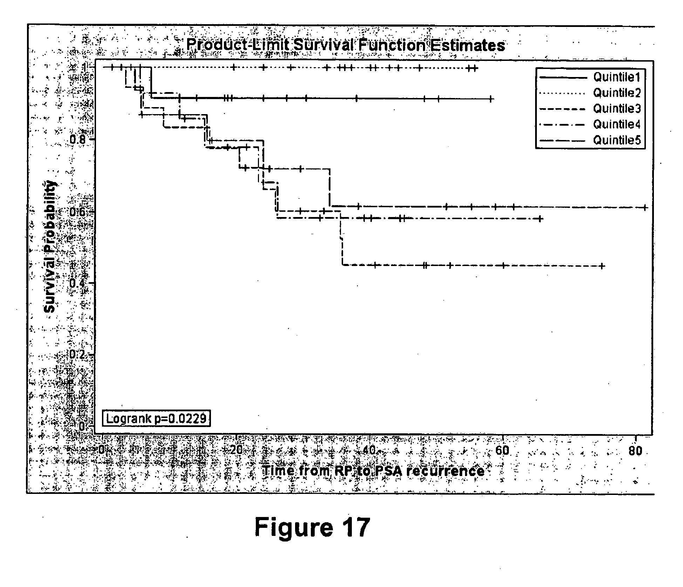

Statistical analysis of the data presented in Figure 16 demonstrates that the expression of tissue *PSA*/*KLK3* mRNA in tumor cells of biochemical recurrence free patients was significantly higher than in patients with biochemical recurrence (p=0.0062, Student t-test). *PSA*/*KLK3* mRNA expression in benign epithelial cells did not show such correlation. This prostate cancer patient cohort was divided into quintiles based on tissue *PSA*/*KLK3* mRNA expression levels in tumor cells, and was compared with respect to time to biochemical relapse. As seen in Figure 17, an unadjusted Kaplan-Meier analysis demonstrates improved biochemical survival for patients with the highest tissue *PSA*/*KLK3* mRNA expression (Quintiles 1 and 2) (p = 0.0229). Thus, *PSA*/*KLK3* mRNA expression in tumor cells of prostate cancer patients inversely correlates with disease recurrence. High expression levels of tumor *PSA*/*KLK3* mRNA correlates with biochemical recurrence free survival, whereas with low expression levels of *PSA*/*KLK3* mRNA reflect an alteration of AR signaling in the tumor cell microenvironment, leading to an increased likelihood of tumor recurrence after prostatectomy.

### Example 12: ERG activates C-MYC, a central target of ERG in prostate cancer

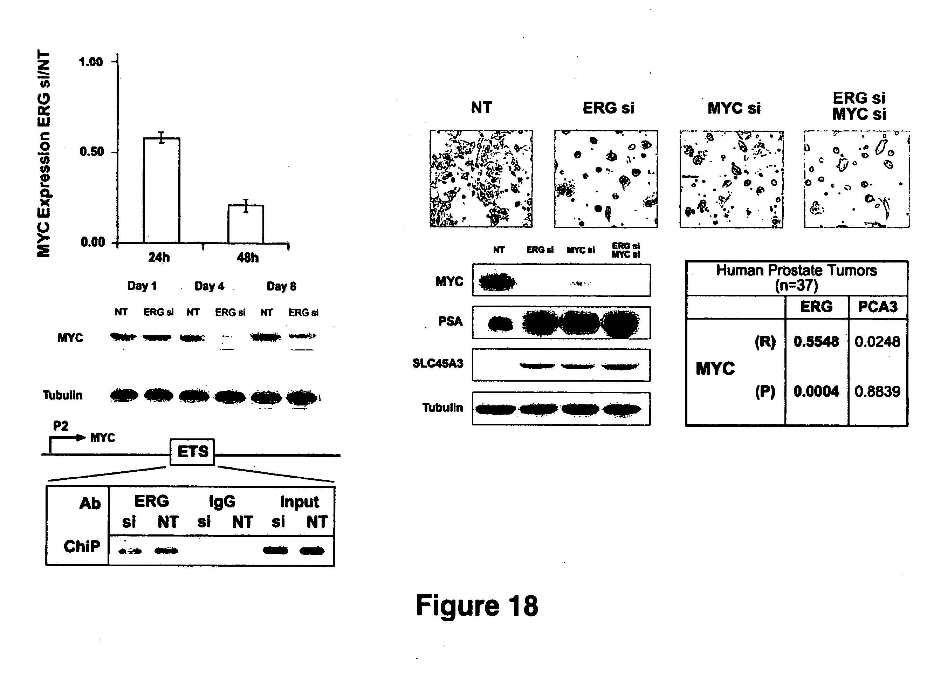

Inhibiting *ERG* decreased *C-MYC* expression and upregulated the prostate differentiation marker genes PSA and protein. Furthermore, inhibiting *C-MYC* recapitulated the *ERG* siRNA phenotype. A double knockdown of *ERG* and *C-MYC* using 50-50% doses of inhibitory *ERG* and *C-MYC* siRNA molecules effectively controlled cell growth and rescued the differentiation program in prostate cancer cells (Figure 18). *C-MYC* expression significantly correlated with *ERG* expression in human prostate tumor cells (Figure 18).

Small interfering RNA ("siRNA") oligo duplexes designed to interfere with ERG function were based on the human ERG having the NCBI locus ID GXL_163565 and Accession No. NM_004440. The ERG siRNA 5'-CGACATCCTTCTCTCACAT-3' (SEQ ID NO: 29) was purchased from Dharmacon, Lafayette, CO). The siRNA pool against human C-MYC (L-003282-00) (Locus ID: GXL_67312, Accession: NM_002467) and non-target ("NT") siRNA duplexes (D-001206-13-20) were both from Dharmacon, Lafayette, CO.

VCaP cells were seeded into 10 cm tissue culture dishes in medium containing 10% charcoal-treated fetal bovine serum ("cFBS") (Gemini Bio-Products, Calabasas, CA) for three days. The cells were transfected with 50 nM NT, 50 nM *ERG* siRNA, 50 nM *MYC* siRNA, or the combination of 25 nM *ERG* siRNA and 25 nM *MYC* siRNA. Cotransfection of siRNAs and plasmids was carried out with Lipofectamine 2000 (Invitrogen, Carlsbad, CA), as described by the manufacturer for HEK 293 cells.

The coding region of *ERG* (NM_004440) was sub-cloned into an adenoviral transfer vector containing an internal ribosome entry site ("IRES"), wherein green fluorescent protein ("GFP") is expressed from the IRES translation initiation sequence ("IRES-GFP"). The generation of recombinant adenovirus plasmid and production of recombinant adenovirus were performed as described by Sun et al., ONCOGENE 25, 3905-13 (2006). Adenovirus titer was determined by GFP assay and plaque forming assay. VCaP and LNCaP cells were infected with the Ad-ERG or Ad-Control vectors and the proteins were detected by Western blot. A wild type ERG3 (NCBI Accession No. NM_182918) expression vector (pIRES-EGFP-ERG3) was generated by amplifying the coding sequences with the primers 5'-GGCTTTGATGAAAGCTCTAAACAAC-3' (SEQ ID NO: 50) and TCAAAAGTGCCTCAAGAGGA-3' (SEQ ID NO: 51) from a human normal prostate cDNA library (Catalog No. AM 3337, Ambion, Austin, TX) and was verified by DNA sequencing. HEK293 cells were transfected with wild type ERG3 or TM-ERG3- expressing vectors and the proteins were detected by Western blot.

Twelve hours after transfection with siRNA, the cells were treated with 100 pM R1881 and processed for the subsequent analyses. To knockdown ectopic ERG protein, TMPRSS2-ERG2 and TMPRSS2-ERG3 plasmids (pIRES-EGFP, Clontech, Palo Alto, CA) were generated by PCR amplification of human TMPRESS2 and ERG cDNA with the following primer 5'-TAGGCGCGAGCTAAGCAGGAG-3' (SEQ ID NO: 8) and 5'-CCCTCCCAAGAGTCTTTGGATCTC-3' (SEQ ID NO: 12) or (SID ID NO: and the sequences were verified by DNA sequencing. Cotransfection of siRNAs and plasmids was carried out with Lipofectamine 2000 (Invitrogen, Carlsbad, CA), as described by the manufacturer for HEK 293 cells.

*C-MYC* expression in response to inhibition by *ERG* siRNA was measured by QRT-PCR and Western blot. In the QRT-PCR analysis, VCaP cells were transfected with siRNAs and harvested two or four days after transfection. Total RNA preparation and RT-PCR were performed as described by Gao et al., CLIN CAN RES 9:2545-50 (2003). Each RNA sample was evaluated for *ERG* knockdown by ERG siRNA in triplicate RT-PCR reactions and one control reaction performed in the absence of reverse transcriptase. The *ERG* PCR forward primer 5'-ACCGTTGGGATGAACTACGGCA -3' (SEQ ID NO: 10) and reverse primer 5'- TGGAGATGTGAGAGAAGGATGTCG -3' (SEQ ID NO: 53) were used in the reaction. *GAPDH* gene expression was detected using forward primer 5'- GAGCCACATCGCCTCAGACACC -3' (SEQ ID NO: 54) and reverse primer 5'- GTTCTCAGCTTGACGGTGCC-3' (SEQ ID NO: 55). RT-PCR derived *ERG* or *GAPDH* fragments were separated by electrophoresis on Tris-borate EDTA-1% agarose gels and visualized by ethidium bromide staining. Band densities were quantified with Quantity One (Bio-Rad Laboratories, Hercules, CA) and *ERG* expression was normalized to *GAPDH* levels.

In the Western blot analysis, cells were lysed in M-PER mammalian protein extraction reagent (Pierce, Rockford, IL) supplemented with protease and phosphatase inhibitor cocktails (Sigma, St. Louis, MO). Immunoblot assays were performed according to standard procedures, for example, probing NuPAGE Bis-Tris gels (Invitrogen, Carlsbad, CA) with antibodies. Antibodies used in Western blot experiments include an immunoaffinity purified anti-ERG peptide polyclonal antibody prepared in our laboratory to the peptide having the amino acid sequence DFHGIAQALQPHPPESSLYKYPSDLPYMGSYHAHPQKMNFVAPHPPAL (SEQ ID NO: 52), anti-PSA (Dako, Carpinteria, CA), anti-MYC (Upstate Biotechnology, Lake Placid, NY), anti-SLC45A3 (Dako, Carpinteria, CA) or anti-GAPDH antibodies (SantaCruz, Santa Cruz, CA). To detect the endogenous ERG protein in VCaP cells, 80 µg cell lysate were loaded into each lane of the gel.

Reduced recruitment of *ERG* to the MYC P2 promoter downstream ETS element was assessed at 48 hours post-transfection by chromatin immunoprecipitation ("ChIP") assay, using an anti-ERG antibody (Figure 18, bottom left panel). IgG and control genomic DNA amplicons ("Input") were used as controls.

ChIP assays were performed according to Masuda et al. J MOL BIOL 353:763-71 (2005). To detect specific ChIP products, 38 amplification cycles were performed. ETS binding sites within the target regions were identified by matrix match analysis using the GEMS Launcher software (Genomatix GmbH, Munich, Germany). ERG protein was detected with the polyclonal anti-ERG antibody sc-353 (SantaCruz Biotechnology, Inc., Santa Cruz, CA). Promoters directed to NCBI Accession No. GLX_67312/NM_002467 amplified the human *C-MYC* gene. The primer pair directed to the ETS binding site V$ETSF/PDEF0.1 P2 downstream promoter 5'- GCCCCTTGCATCCTGAGCTCC-3' (SEQ ID NO: 56) directed the 5'- GGTCGGACATTCCTGCTTTA-3' (SEQ ID NO: 57) and 5'-ACCCAACACCACGTCCTAAC-3' (SEQ ID NO: 58) was used as described in Meulia et al. MOL CELL BIOL 12:4590-600 were used. The androgen receptor ("AR") was immunoprecipitated with anti-AR antibodies and the PSA(*KLK3*) AREIII enhancer target region was amplified as described. (Masuda et al. J MOL BIOL 353:763-71 (2005)). The *SLC45A3* gene (NCBI Accession No. GXL_151340/XM_001490454) promoter upstream ARE (V$GREF_ARE0.2) and ETS (V$ETSF_PDEF0.1) binding site containing region (5'-AGAGCACAGAAAGGCTGCCCTGG AAGTGGCTG GGCATCCTGTCAGCT-3') (SEQ ID NO: 59) was amplified by the 5'-TGTGGGACTTCTCTGCTGAA-3' (SEQ ID NO: 60) and 5'-CAACGTTCAAGGGGAAGAAA-3' (SEQ ID NO: 61) primers.

Cell morphology was also examined. Photomicrographs of VCaP cells at day 8 are shown in the top right panel of Figure 18. PSA mRNA and protein expression were also measured by QRT-PCR and Western blot assays, respectively, and are shown in the bottom panel of Figure 18. Cell lysates were prepared and assayed by Western blot with anti-C-MYC, anti-PSA, or anti-SLC45A3 (protein) antibodies. Tubulin was used as a control.

ERG-MYC correlation analysis was performed by assessing quantitative gene expression data of *C-MYC* and *ERG* or *C-MYC* and *PCA3* from 37 laser capture microdissected human tumors. The results were expressed as R and P-values, as shown in the table in the bottom right panel of Figure 18. The correlation of *ERG* with *C-MYC* is highly significant; R=0.5548, p=0.0004.

### Example 13: GeneChip^{®} data analysis

VCaP cells were plated in medium containing 10% cFBS (Gemini Bio-Products, Calabasas, CA) for three days. Cells were transfected with 50 nM of siERG or 50 nM of control NT and grown in FBS containing media for 24 or 48 hours. Total RNA was isolated, five micrograms of RNA from each of the VCaP transfectants were biotin labeled, and GeneChip^{®} HG U133 Plus 2.0 chips were hybridized with the labeled probes. Expression data were normalized by Robust Multi-array Averages (RMA) and fold changes in ERGsi/NT (24h) and ERGsi/NT (48h) treatment groups were calculated. A two fold cut-off criterion was applied for subsequent pathway analysis by the Bibliosphere Software using the functional co-citation-based analysis function (Genomatix GmbH, Munich, Germany) (Scherf et al., BRIEF BIOINFORM 6:287-97 (2005). Total RNA from human prostate specimens was isolated from laser capture microdissected tumor and benign epithelial cells, as described by Shaheduzzamen *et al.,* CAN BIOL THER 6:(2007, Epub ahead of print). The RNA was quantified, amplified, biotinylated, hybridized to the high-density oligonucleotide human genome array HG U133A (Affymetrix, Santa Clara, CA), and normalized as described by Petrovics et al., ONCOGENE 24:3847-52 (2005). Tumor/normal ERG expression ratios were evaluated in the data set from well-differentiated prostate tumors. Gene expression changes were averaged in seven data sets where ERG expression was elevated 19-38 fold. A two-fold cut-off criterion was applied for further pathway analysis using Bibliosphere Software (Genomatix GmbH, Munich, Germany). Normalized human ERG overexpressing tumor data was compared to the 48 h ERG siRNA treatment gene expression data. Common genes were selected for the subsequent network analysis by the Bibliosphere Software.

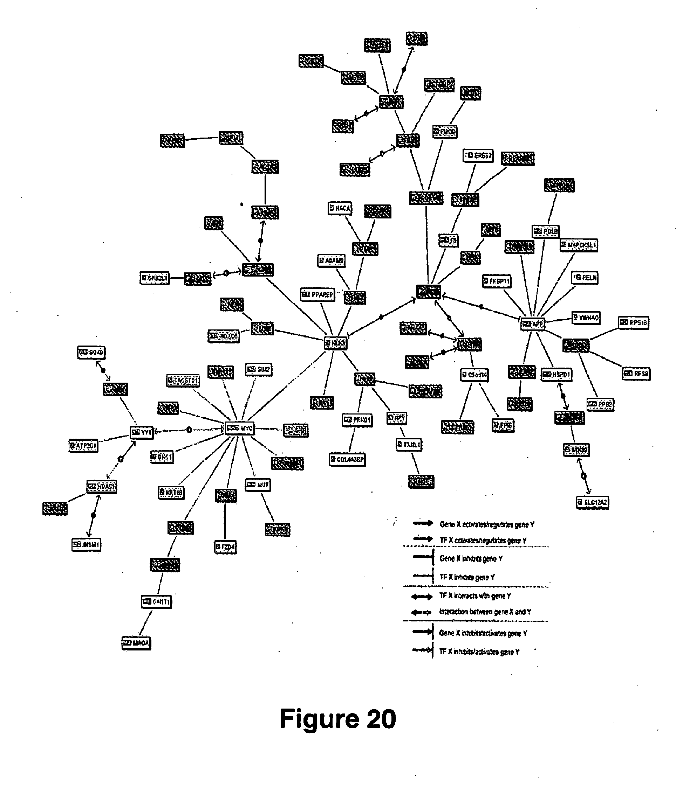

Figure 20 shows a gene network in ERG expressing human prostate tumors. Normalized (tumor/normal) gene expression data of seven well-differentiated prostate tumors with 19-38 folds of ERG overexpression were analyzed by the Bibliosphere software (Shaheduzzamen et al., CAN BIOL THER 6:(2007, Epub ahead of print)). Red (medium grey) and yellow (light grey) boxes indicate upregulation, shades of blue (dark grey) indicate downregulation. The functional connections are disclosed in the insert to Figure 20.

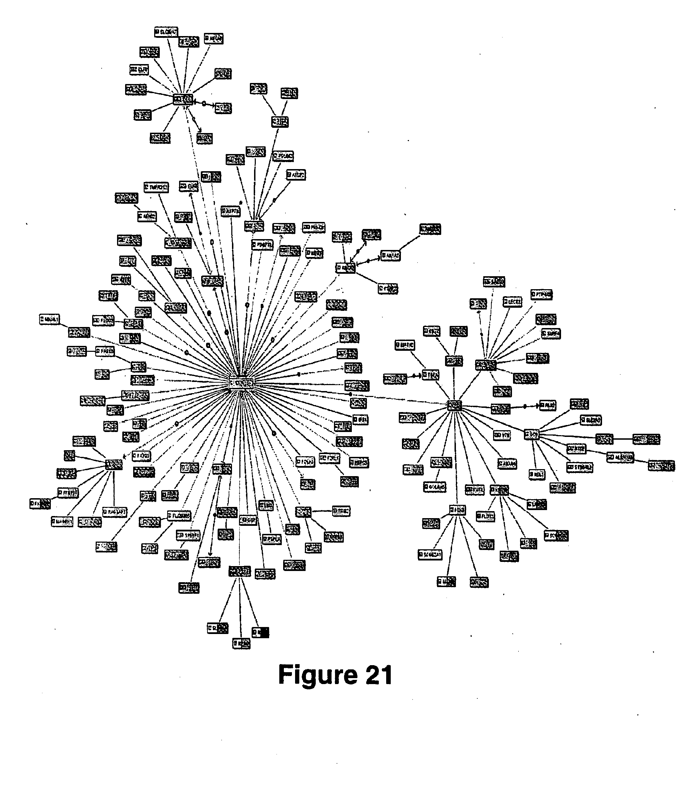

The network of genes affected in response to ERG knockdown in VCaP cells is shown in Figure 21. Cells were transfected with either 50 nM of *ERG* siRNA or with 50 nM of NT and were incubated for 24 hours (left side codes) or 48 hour (right side codes). For probe labeling, total RNA was isolated from the cells and was labeled for hybridization. GeneChip^{®} HG U133 Plus 2.0 chips were hybridized with the labeled probes. ERG si/NT expression ratios were calculated and a two-fold cut-off criterion was applied towards the subsequent pathway analysis by Bibliosphere Software. Gene symbols within the boxes indicate changes in gene expression (Figure 21).

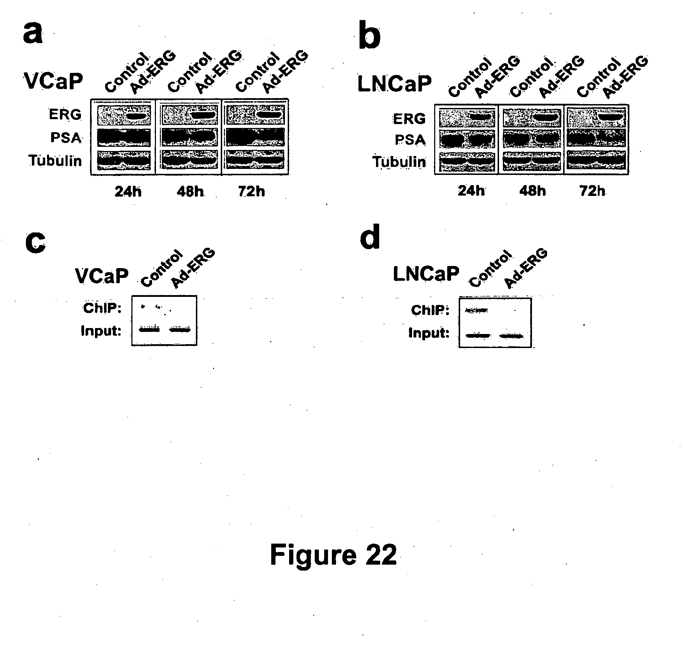

Transient *ERG* expression diminished PSA protein levels and decreased recruitment of AR to the PSA AREIII enhancer. VCaP and LNCaP cells were infected with adenoviral ERG (Ad-ERG) or Ad-Control (Control) vectors. Cell lysates prepared at 24, 48, and 72 hours post-infection were analyzed in immunoblot assays with anti-ERG, anti-PSA, and anti-tubulin antibodies. ChIP assessment of AR recruitment to the *KLK3*/*PSA* gene AREIII enhancer in VCaP and LNCaP cells in response to the transient expression of ERG by adenoviral Ad-ERG or Control vectors (Figure 22).

### Example 14: Prostate differentiation genes are repressed by ERG

*ERG* siRNA treatment resulted in the recruitment of androgen receptor to the PSA AREIII enhancer (Figure 23). PSA mRNA and protein expression were measured by QRT-PCR and Western blot assays, respectively. Increased AR binding to the PSA enhancer (ARE) and decreased ERG recruitment to the overlapping ETS cognate element was measured by ChIP assay 48 hours after transfection. After 9 days of *ERG* siRNA treatment, VCaP cells were stained for cytokeratin CK8/18, PSA, and DNA (Figure 23).

*ERG* siRNA treatment also resulted in the recruitment of androgen receptor to the prostein (SLC45A3) gene upstream enhancer. Expression of TMPRSS2-ERG negatively correlated with prostein (SLC45A3) expression in human specimens (Figure 24). SLC45A3 (Prostein) expression in *ERG* siRNA transfected VCaP cells was measured by Western blot. Recruitment of AR and *ERG* to the SLC45A3 promoter upstream ARE and ETS elements was assessed by ChIP assay 48 hours post-transfection. A matrix representation of TMPRSS2-ERG expression and SLC45A3 immunostaining of prostate tumors of 26 patients demonstrated a correlation between *ERG* siRNA treatment and prostein expression. Sections of whole mounted radical prostatectomy specimens were assessed by immunohistochemistry with anti- SLC45A3 (SLC) antibody.

Radical prostatectomy specimens from 26 patients were fixed in 10% buffered formalin and embedded as whole mounts in paraffin. Each prostate was sectioned at 0.22 cm intervals in a transverse plane perpendicular to the long axis of the posterior surface of the prostate and completely embedded as whole mounts. The volume of each tumor was calculated in three dimensions (apex to base, right to left, and anterior to posterior) using the largest dimension in each direction to determine the index tumor. Index tumor was analyzed for the presence or absence of TMPRSS2-ERG fusion transcripts as described by Furusato et al., MOD PATHOL 21(2):67-75 (2008), and for Prostein immunohistochemical staining on adjacent four-micron sections of the whole-mounted blocks. Slides were incubated with anti-SLC45A3 antibody (Dako North America, Carpinteria, CA), diluted 1:160. Vector VIP (purple) was used as the chromogen substrate (Vector Laboratories, Burlingame, CA) and the slides were counterstained with hematoxylin. SLC45A3 expression was assessed based on both the amount and intensity of immunopositive cells. Intensities were scored as "0" if not stained, "1" if stained weakly, and "2" if stained strongly. The percentage of positively stained area was also estimated and scored as "1" if less than 25% of the area stained positive, "2" if 25-50% stained positive, "3" if 51-75% stained positive, and "4" if more than 75% stained positive. The final score was determined by multiplying the intensity score and the percentage of positively stained area.

VCaP cells were fixed with 4% paraformaldehyde and centrifuged onto silanized slides (Sigma, St. Louis, MO) with a cytospin centrifuge. Cells were immunostained with anti-cytokeratin 8/18 and anti-PSA (both from Dako, Carpinteria, CA) followed by goat anti-mouse Alexa-488 and goat anti-rabbit Alexa-594 secondary antibodies (invitrogen, Carlsbad, CA). Images were captured using a 40X/0.65 N-Plan objective lens on a Leica DMLB upright microscope with a Qlmaging Retiga-EX CCD camera (Burnaby, BC, Canada) controlled by OpenLab software (Improvision, Lexington, MA). Images were converted into color and merged by using Adobe Photoshop.

The specification is most thoroughly understood in light of the teachings of the references cited within the specification. The embodiments within the specification provide an illustration of embodiments of the invention and should not be construed to limit the scope of the invention.

## Claims

1. A method of detecting prostate cancer in a biological sample comprising:
(a) combining the biological sample with at least a first and a second oligonucleotide primer under hybridizing conditions, wherein the first oligonucleotide primer contains a sequence that hybridizes to a first sequence in a target sequence from *ERG8* and the second oligonucleotide primer contains a sequence that hybridizes to a second sequence in a nucleic acid strand complementary to the target sequence, wherein the first sequence does not overlap with the second sequence and wherein the target sequence comprises SEQ ID NO:46, or nucleotides 942 to 1046, nucleotides 1285 to 1374, or nucleotides 1451 to 1541 of SEQ ID NO:46;
(b) amplifying a plurality of amplification products when the target sequence is present in the biological sample by adding at least one polymerase activity to the biological sample containing the first and second oligonucleotide primers;
(c) immobilizing the plurality of amplification products;
(d) combining an oligonucleotide probe with the immobilized plurality of amplification products to thereby permit the probe to hybridize to at least one immobilized amplification product; and
(e) detecting whether a signal results from hybridization between the oligonucleotide probe and at least one amplification product, wherein detection of the signal indicates the expression of *ERG8* and the presence of prostate cancer in the biological sample.

2. The method of claim 1, wherein the target sequence comprises nucleotides 942 to 1046 of SEQ ID NO:46.

3. The method of claim 1, wherein the target sequence comprises nucleotides 1285 to 1374 of SEQ ID NO:46.

4. The method of claim 1, wherein the target sequence comprises nucleotides 1451 to 1541 of SEQ ID NO:46.

5. An isolated ERG8 nucleic acid molecule comprising SEQ ID NO:46 or the complement thereof.

6. Use of a nucleic acid molecule for detecting prostate cancer in a biological sample, wherein the nucleic acid molecule comprises at least about 15 contiguous nucleotides of nucleotides 942 to 1046 or 1285 to 1374 of SEQ ID NO:46, wherein the nucleic acid is capable of hybridizing to SEQ ID NO:46, or the complement thereof, under conditions of high stringency but not to *ERG1, ERG2, ERG3, ERG4, ERGS, ERG6, ERG7, ERG9, EPC1, EPC2,* or *TMPRSS2.*

7. The use of claim 6, wherein the nucleic acid is up to about 50 nucleotides long.

8. The use of claim 6, wherein the conditions of high stringency comprise hybridization for 12 hours at 65°C in 6x SSC followed by a wash in 0.1 X SSC at 50°C for 45 minutes.

## Patentansprüche

1. Verfahren zum Nachweis eines Prostatakarzinoms in einer biologischen Probe, umfassend:
(a) Kombinieren der biologischen Probe mit mindestens einem ersten und einem zweiten Oligonukleotidprimer unter Hybridisierungsbedingungen, wobei der erste Oligonukleotidprimer eine Sequenz enthält, die an eine erste Sequenz in einer Zielsequenz von *ERG8* hybridisiert und der zweite Oligonukleotidprimer eine Sequenz enthält, die an eine zweite Sequenz in einem Nukleinsäurestrang, der komplementär zu der Zielsequenz ist, hybridisiert, wobei die erste Sequenz die zweite Sequenz nicht überlappt und wobei die Zielsequenz SEQ ID NO: 46 umfasst oder die Nukleotide 942 bis 1046, die Nukleotide 1285 bis 1374 oder die Nukleotide 1451 bis 1541 von SEQ ID NO: 46 umfasst;
(b) Amplifizieren mehrerer Amplifikationsprodukte, wenn die Zielsequenz in der biologischen Probe vorliegt, durch Hinzufügung mindestens einer Polymeraseaktivität zu der biologischen Probe, die den ersten und zweiten Oligonukleotidprimer enthält;
(c) Immobilisieren der mehreren Amplifikationsprodukte;
(d) Kombinieren einer Oligonukleotidsonde mit den immobilisierten mehreren Amplifikationsprodukten, um dadurch zu gestatten, dass die Sonde an mindestens ein immobilisiertes Amplifikationsprodukt hybridisiert; und
(e) Nachweisen, ob ein Signal aus der Hybridisierung zwischen der Oligonukleotidsonde und mindestens einem Amplifikationsprodukt resultiert, wobei ein Nachweis des Signals die Expression von ERG8 und die Gegenwart des Prostatakarzinoms in der biologischen Probe anzeigt.

2. Verfahren nach Anspruch 1, wobei die Zielsequenz die Nukleotide 942 bis 1046 von SEQ ID NO: 46 umfasst.

3. Verfahren nach Anspruch 1, wobei die Zielsequenz die Nukleotide 1285 bis 1374 von SEQ ID NO: 46 umfasst.

4. Verfahren nach Anspruch 1, wobei die Zielsequenz die Nukleotide 1451 bis 1541 von SEQ ID NO: 46 umfasst.

5. Isoliertes *ERG8*-Nukleinsäuremolekül, welches SEQ ID NO: 46 oder dessen komplementären Strang umfasst.

6. Verwendung eines Nukleinsäuremoleküls zum Nachweis eines Prostatakarzinoms in einer biologischen Probe, wobei das Nukleinsäuremolekül mindestens etwa 15 zusammenhängende Nukleotide der Nukleotide 942 bis 1046 oder 1285 bis 1374 von SEQ ID NO: 46 umfasst, wobei die Nukleinsäure mit SEQ ID NO: 46 oder dessen komplementärem Strang, jedoch nicht mit *ERG1, ERG2, ERG3, ERG4, ERG5, ERG6, ERG7, ERG9, EPC1, EPC2* oder *TMPRSS2* unter hochstringenten Bedingungen hybridisieren kann.

7. Verwendung nach Anspruch 6, wobei die Nukleinsäure bis zu etwa 50 Nukleotide lang ist.

8. Verwendung nach Anspruch 6, wobei die hochstringenten Bedingungen Hybridisierung für 12 Stunden bei 65°C in 6x SSC gefolgt von Waschen in 0,1x SSC bei 50°C für 45 Minuten umfassen.

## Revendications

1. Procédé de détection du cancer de la prostate dans un échantillon biologique comprenant les étapes suivantes :
(a) combiner l'échantillon biologique avec au moins une première et une deuxième amorces oligonucléotidiques dans des conditions d'hybridation, dans lequel la première amorce oligonucléotidique contient une séquence qui s'hybride à une première séquence dans une séquence cible d'*ERG8* et la deuxième amorce oligonucléotidique contient une séquence qui s'hybride à une deuxième séquence dans un brin d'acide nucléique complémentaire à la séquence cible, dans lequel la première séquence ne se chevauche pas avec la deuxième séquence et dans lequel la séquence cible comprend SEQ ID NO: 46, ou les nucléotides 942 à 1046, les nucléotides 1285 à 1374, ou les nucléotides 1451 à 1541 de SEQ ID NO: 46 ;
(b) amplifier une pluralité de produits de l'amplification quand la séquence cible est présente dans l'échantillon biologique en ajoutant au moins une activité de la polymérase à l'échantillon biologique contenant la première et la deuxième amorces oligonucléotidiques;
(c) immobiliser la pluralité de produits de l'amplification;
(d) combiner une sonde oligonucléotidique avec la pluralité de produits de l'amplification immobilisée pour ainsi permettre à la sonde de s'hybrider sur au moins un produit de l'amplification immobilisé; et
(e) détecter si un signal résulte de l'hybridation entre la sonde oligonucléotidique et au moins un produit de l'amplification, dans lequel la détection du signal indique l'expression d'*ERG8* et la présence du cancer de la prostate dans l'échantillon biologique.

2. Procédé selon la revendication 1, dans lequel la séquence cible comprend les nucléotides 942 à 1046 de SEQ ID NO: 46.

3. Procédé selon la revendication 1, dans lequel la séquence cible comprend les nucléotides 1285 à 1374 de SEQ ID NO: 46.

4. Procédé selon la revendication 1, dans lequel la séquence cible comprend les nucléotides 1451 à 1541 de SEQ ID NO: 46.

5. Molécule d'acides nucléiques d'*ERG8* isolée comprenant SEQ ID NO: 46 ou son brin complémentaire.

6. Utilisation d'une molécule d'acides nucléiques pour détecter le cancer de la prostate dans un échantillon biologique, dans laquelle la molécule d'acides nucléiques comprend au moins environ 15 nucléotides contigus parmi les nucléotides 942 à 1046 ou 1285 à 1374 de SEQ ID NO: 46, dans laquelle l'acide nucléique est capable de s'hybrider dans des conditions très rigoureuses à SEQ ID NO: 46, ou à son brin complémentaire, mais pas à *ERG1, ERG2, ERG3, ERG4, ERG5, ERG6, ERG7, ERG9, EPC1, EPC2,* ou *TMPRSS2.*

7. Utilisation selon la revendication 6, dans laquelle l'acide nucléique fait jusqu'à environ 50 nucléotides de long.

8. Utilisation selon la revendication 6, dans laquelle les conditions très rigoureuses comprennent une hybridation pendant 12 heures à 65°C dans 6 x SSC suivi par un lavage dans 0,1 X SSC à 50°C pendant 45 minutes.
